# EUROPEAN PATENT APPLICATION

(11) **EP 1 491 216 A1**
(43) Date of publication of application: **29.12.2004**
(21) Application number: 03745405.5
(22) Date of filing: 05.03.2003
(51) Int. Cl.: A61K 48/00, A61K 45/00, A61K 9/51, A61K 9/08, A61K 38/18, A61K 38/21, A61P 1/16, A61P 35/00

(54) **DRUGS COMPRISING PROTEIN FORMING HOLLOW NANOPARTICLES AND THERAPEUTIC SUBSTANCE TO BE TRANSFERRED INTO CELLS FUSED THEREWITH**

(30) Priority: 29.03.2002 JP 2002097280
(71) Applicant: Japan Science and Technology Agency, Kawaguchi-shi, Saitama 332-0012 (JP)
(72) Inventor: KURODA, Shunichi, Suita-shi, Osaka 565-0872 (JP); TANIZAWA, Katsuyuki, Toyono-gun, Osaka 563-0214 (JP); KONDO, Akihiko, Kobe-shi, Hyogo 657-0015 (JP); UEDA, Masakazu, Shinjuku-ku, Tokyo 162-0837 (JP); SENO, Masaharu, Okayama-shi, Okayama 703-8273 (JP); TADA, Hiroko, Okayama-shi, Okayama 700-0016 (JP)
(74) Representative: Bentz, Jean-Paul
(86) International application number: PCT/JP2003/002602
(87) International publication number: WO 2003/082345

(57) **Abstract**

The subject invention provides a disease-treating drug that uses hollow protein nanoparticles to specifically act on a target cell or tissue. The present invention allows a protein drug to be effectively capsulated in the particles. The invention also provides a therapeutic method using such a drug. The drug according to the present invention is capable of recognizing a specific cell, such as hepatocytes, and manufactured by fusing a disease-treating substance for a target cell (for example, interferon, hepatocyte growth factor etc.) with hollow nanoparticles of a particle-forming protein (for example, hepatitis B virus surface-antigen protein).

## Description

### TECHNICAL FIELD

The present invention relates to a drug containing hollow nanoparticles of particle-forming protein, fused with a disease-treating-target-cell substance. The invention particularly relates to a drug containing a disease-treating target-cell-substance, that is encapsulated in the particles to be specifically transferred to a specific cell or tissue.

### BACKGROUND ART

In the field of medicine, there has been active research on drugs that directly and effectively act on the affected area without causing serious side effects. One area of active research is a method known as a drug delivery system (DDS), in which active ingredients of drugs or other substances are specifically delivered to a target cell or tissue, where they can exhibit their effects.

One known example of conventional method of sending genes to cells is so-called a gene transfer method. In this method, genes encoding the protein are incorporated into an expression vector, and this expression vector is transferred to the target cell by an electroporation method or the like. The transferred vector is expressed in the cell to be the protein functioning to the drug.

However, none of the conventional gene transfer methods is sufficient to specifically transfer genes to a target cell/ tissue and express the protein therein to produce a drug. Further, to this date, there has been no effective method of directly delivering a protein as a drug into a target cell/tissue.

Under these circumstances, the inventors of the present invention have previously proposed a method of specifically and safely delivering and transferring various substances (including genes, proteins, compounds) into a target cell or tissue, using hollow nanoparticles of a protein that has the ability to form particles and has incorporated a bio-recognizing molecules, as disclosed in International Publication No. WO01/64930 (published on September 7, 2001) (hereinafter referred to as "International Publication WO01/64930"). However, there has been a need to develop this method to produce new protein drugs transferable to specific cells or tissues, particularly in view of the following problems.

Owning to the difficulty in specifically and safely delivering and transferring a protein (drug) into a target cell or tissue, a great burden has been put on the patients receiving treatment using such a protein drug.

For example, for the treatment of viral hepatitis (hepatitis C in particular), an interferon, which is one form of a protein drug, is administered systemically through intravenous injection over an extended time period. Though the effectiveness of the treatment is well recognized, it has many side effects due to the non-specific action of the interferon, including high fever, loss of hair, tiredness, and immune response, which occur every time the drug is administered.

The hepatocyte growth factor is known to be effective for the treatment of liver cirrhosis. However, since systemic administration of the drug through intravenous injection may cause unexpected side effects, the hepatocyte growth factor is directly administered with a catheter. The use of catheter requires surgery, which puts a burden on the patient if he or she must receive prolonged treatment.

The present invention was made in view of the foregoing problems, and an object of the invention is to provide a disease-treating drug, that specifically acts on a target cell or tissue with its hollow protein nanoparticles that allow a protein drug to be efficiently encapsulated in the particles. The present invention further relates to a thermonkeyutic method using such a drug.

### DISCLOSURE OF INVENTION

As a result of intensive study, the inventors of the present invention accomplished the present invention by successfully preparing a vector for expressing a protein in which the particle-forming protein is fused with a disease-treating protein drug (target-cell-substance), and by producing particles of the drug with the vector. This method achieves effective encapsulation of a protein drug in the particles.

Specifically, a drug according to the present invention comprises hollow nanoparticles of a particle-forming protein, that is capable of recognizing a specific cell or tissue, and is fused with a disease-treating target-cell-substance.

The suitable examples of particle-forming protein include a hepatitis B virus surface-antigen protein. Particles of such a particle-forming protein may be obtained through the protein expression in the eukaryotic cell. Specifically, in eukaryotic cells, the particle-forming protein is expressed on the endoplasmic reticulum as a membrane protein and accumulates thereon before it is released as particles. The drug of the present invention is obtained in the form of protein particles fused with a target-cell-substance (i.e., protein drug) by transforming an eukaryotic cell (yeasts, insects, or animals including mammals) with a vector that contains a first gene encoding the particle-forming protein and a second gene, downstream of the first gene, encoding the target-cell-substance, and by expressing the first and second genes in the eukaryotic cell.

Since the target-cell-substance is fused with the protein that forms particles, it may be encapsulated in the particles upon preparation of the particles; therefore, extra step for transferring the target-cell-substance into the particles after the formation of the particles is not necessary, thus offering easy manufacturing. With this method, encapsulation of substances into particles may be efficiently performed even with giant molecules etc.

The particles made of a hepatitis B virus surface-antigen protein identify hepatocytes, thus specifically transferring the substance encapsulated in the particles to the hepatocytes. With this property, the hepatitis B virus surface-antigen protein therein encapsulating a hepatic-disease-treating substance (protein drug) functions as an effective drug that can specifically and securely act on hepatocytes. The encapsulated substance may be, for example, a protein drug, such as interferons (IFN), a hepatocytes growth factor (HGF) etc. IFN is generally used for treatment of viral hepatitis, and HGF reproduces a hepar infected with hepatic cirrhosis. These substances may be specifically transferred to hepatocytes by being encapsulated in the particles, thus allowing effective treatment of viral hepatitis or hepatic cirrhosis.

Further, by modifying the hepatitis B virus surface-antigen protein to lack the original infectivity to hepatocytes and to display a growth factor or an antigen before formed as particles, the resulting particles will be able to specifically transfer the substance encapsulated therein to other target cells or tissues than hepatocytes. For example, by modifying the protein to display a cancer specific antibody, the protein will identify the cancer cell, thus specifically delivering substances encapsulated in the particles to target cells or tissues.

The present invention discloses a drug that can be used by a convenient method of intravenous injection to effectively treat specific diseased cells or tissues. The drug is a great leap forward from conventional disease treatment methods in that it does not require large dose or any surgical operation in disease treatment including gene therapy, and that the risk of side effect is greatly reduced. The drug is therefore usable in clinical applications in its present form.

The present invention discloses a treatment method for treating diseases through administration of the drug disclosed in the present invention.

### BRIEF DESCRIPTION OF DRAWINGS

Figure 1 is a schematic diagram of particles for making a drug of the present invention, in which HBsAg L protein is fused with a protein drug.
Figure 2 is a schematic diagram showing protein regions of HBsAg L protein genes described in Examples of the present invention, where the numbers 1 through 8 indicate respective functions of different sites on a surface antigen.
Figure 3 is an explanatory drawing schematically showing one example of expression and purification procedures for HBsAg L protein particles using recombinant yeasts, as described in Examples of the present invention, wherein (a) illustrates preparation of recombinant yeasts, (b) illustrates incubation in High-Pi medium, (c) illustrates incubation in 8S5N-P400 medium, (d) illustrates disruption, (e) illustrates density gradient centrifugation, and (f) illustrates HBsAg L particles.
Figure 4 is an explanatory view showing a preparation method of a plasmid for expressing a fusion protein of HBsAg L protein and EGFP, according to Examples of the present invention.
Figure 5 is a picture from Examples of the present invention, which picture is observed by a confocal laser fluorometry microscope and showing a human hepatic cancer cell HepG2 supplied with EGFP, that has been transferred by a fusion protein of HBsAg L protein and EGFP.
Figure 6 is a picture from Examples of the present invention, which picture is observed by a confocal laser fluorometry microscope and showing human squamous-carcinoma-derived cells A431 supplied with EGFP, that has been transferred by a fusion protein of HBsAg L protein and EGFP.
Figure 7 is a picture from Examples of the present invention, which picture is observed by a confocal laser fluorometry microscope and showing a tumor area of a mouse, that had been implanted with a human hepatic-cancer-derived cells HuH-7, that has had a intravenous injection of a fusion protein of HBsAg L protein and EGFP.
Figure 8 is a picture from Examples of the present invention, which picture is observed by a confocal laser fluorometry microscope and showing a tumor area of a mouse, that had been implanted with a human colon-cancer-derived cells WiDr, that has had a intravenous injection of a fusion protein of HBsAg L protein and EGFP.
Figure 9 is the first part of a table of examples of target-cell substances encapsulated in a substance carrier.
Figure 10 is the second part of the table of the examples of target-cell substances encapsulated in a substance carrier.
Figure 11 is the rest of the table of the examples of target-cell substances encapsulated in a substance carrier.

### BEST MODE FOR CARRYING OUT THE INVENTION

The present invention discloses a drug including hollow nanoparticles, in which a protein able to form particles is fused with a target-cell-substance. By incorporating a bio-recognizing molecule (molecule that recognizes a specific cell) to the protein with the particle-forming ability, the drug becomes capable of specifically delivering a substance to a target cell or tissue. The protein with the particle-forming ability may be sub viral particles obtained from various viruses. Specific examples of such a protein include hepatitis B virus (HBV) surface-antigen protein.

Particles of such a particle-forming protein may be obtained through the protein expression in the eukaryotic cell. Specifically, in eukaryotic cells, the particle-forming protein is expressed on the endoplasmic reticulum as a membrane protein and accumulates thereon before it is released as particles. The eukaryotic cell may be obtained from yeasts, insects, or animals including mammals.

As will be described later in Examples, the inventors of the present invention have reported that the expression of HBV surface-antigen L protein in recombinant yeast cells produces ellipsoidal hollow particles with a minor axis of 20 nm and a major axis of 150 nm, with a large number of L proteins embedded in the yeast-derived lipid bilayer membrane (J. Biol. Chem., Vol. 267, No. 3, 1953-1961, 1992). The particles contain no HBV genome and lack the viral function. Therefore, the particles are very safe to the human body. Further, since the particles have on its surface a specific receptor for hepatocytes with high infectivity for HBV hepatocytes, the particles reliably function as a carrier for specifically transferring a substance to hepatocytes.

Therefore, forming the protein particles using recombinant yeasts offers a preferable method of efficiently producing particles from soluble proteins in the yeasts.

The insect cell, being a eukaryote closer to some of the higher animals than the recombinant yeast, is able to form a higher order structure such as a sugar chain unachievable by yeasts. In this connection, the insect cell provides a preferable method of producing heteroproteins in large amounts. The conventional insect cell line used the baculovirus and involved viral expression. This has caused a cell death or lysis in the protein expression. A problem of this method, then, in that the protein expression proceeds continuously, or the proteins are decomposed by the free protease separated from the dead cells. Further, in the secretion and expression of proteins, inclusion of a large amount of fetal bovine serum contained in the culture medium has made it difficult to purify proteins even when proteins are secreted in the medium. In recent years, Invitrogen Corporation has developed and marketed an insect cell line that can be cultured without a serum and without being meditated by the baculovirus. Such an insect line can be used to obtain protein particles that are easy to purify and form into higher order structures.

Hollow protein nanoparticles of the present invention are prepared by modifying a receptor in the surface of particles, that are obtained by the foregoing methods, to a bio-recognizing molecule. With such modification, the hollow protein nanoparticles can very specifically deliver and transfer a substance to a cell or tissue other than hepatocytes.

The particle-forming protein is not limited to the hepatitis B virus surface-antigen protein but may be any protein able to form particles. For example, animal cells, plant cells, viruses, natural proteins derived from fungi, and various types of synthetic proteins may be used. Further, when there is a possibility that, for example, virus-derived antigen proteins may trigger antibody reaction in a target organism, a particle-forming protein with suppressed antigenic action may be used. For example, such a particle-forming protein may be the hepatitis B virus surface-antigen protein modified to suppress its antigenic action, or other types of modified proteins (hepatitis B virus surface-antigen protein modified by genetic engineering), as disclosed in International Publication WO01/64930. Further, another example may be one obtained by adding a growth factor, antibody, or other proteins to a hepatitis B virus surface-antigen protein or a modified hepatitis B virus surface-antigen protein.

Preferable example for the bio-recognizing molecule incorporated in the particle-forming protein (it may be a bio-recognizing molecule contained in the particle-forming protein or a bio-recognizing molecule fused (or bonded directly/indirectly) with the particle-forming protein) include a cell-function-adjusting molecule, such as a growth factor or cytokine, antigens displayed on the cell surface, antigens for specific tissues, molecules for recognizing the cell or tissue, such as a receptor, molecules derived from a virus or a bacteria, an antibody, sugar chain, and lipid. Other example may be an antigen for an EGF receptor and an IL-2 receptor specifically displayed on a cancer cell, or a receptor displayed by EGF or HBV. Among these, a most suitable one is selected according to the type of target cell or tissue. Note that, the "bio-recognizing molecule" here refers to a molecule that recognizes a specific cell (in other words, a molecule giving the cell-specifying ability to the drug of the present invention).

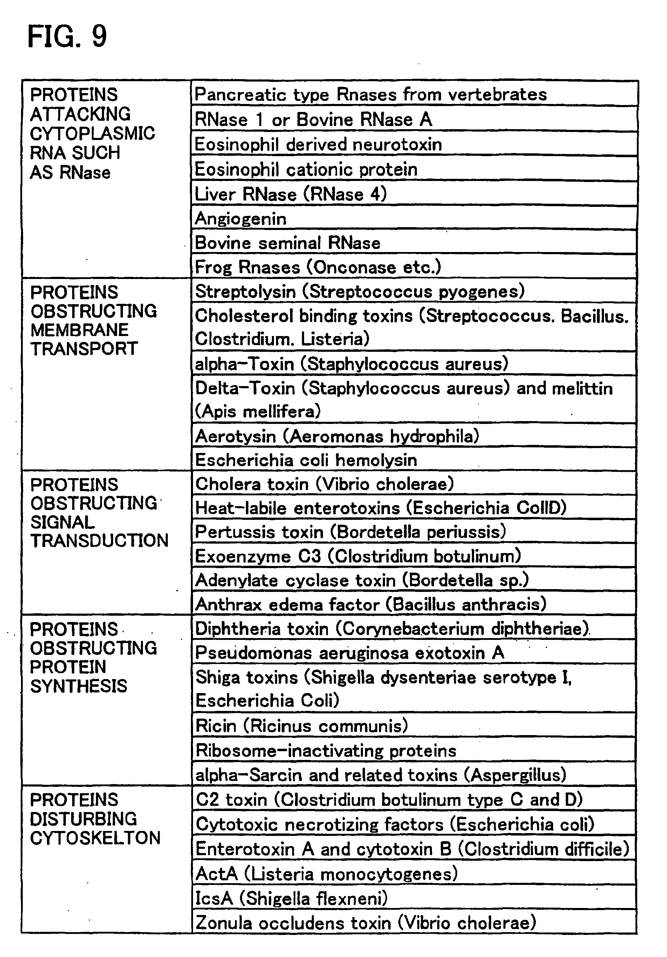

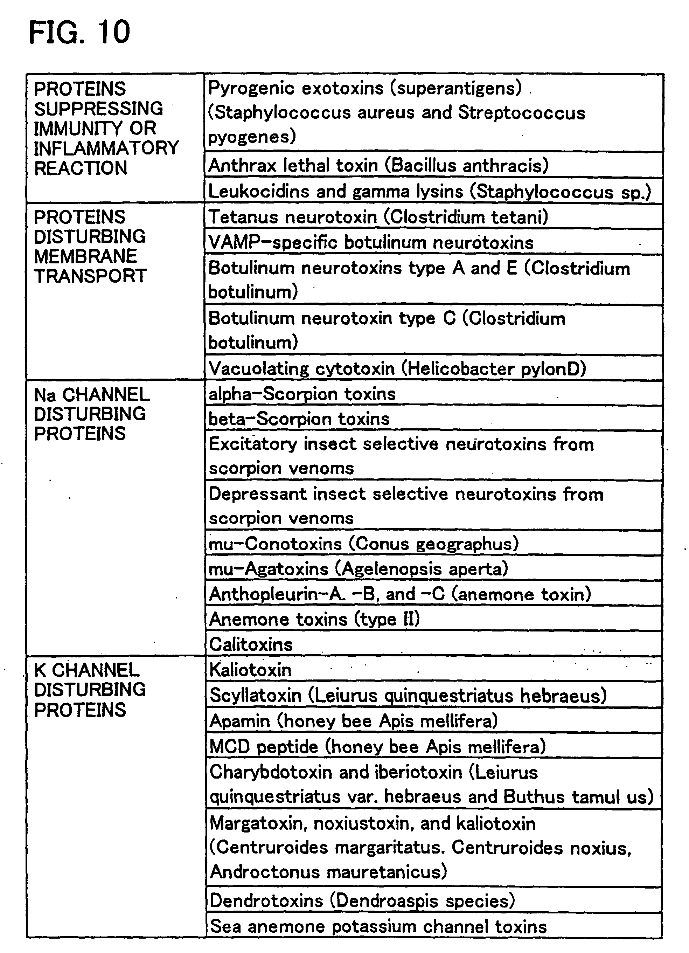

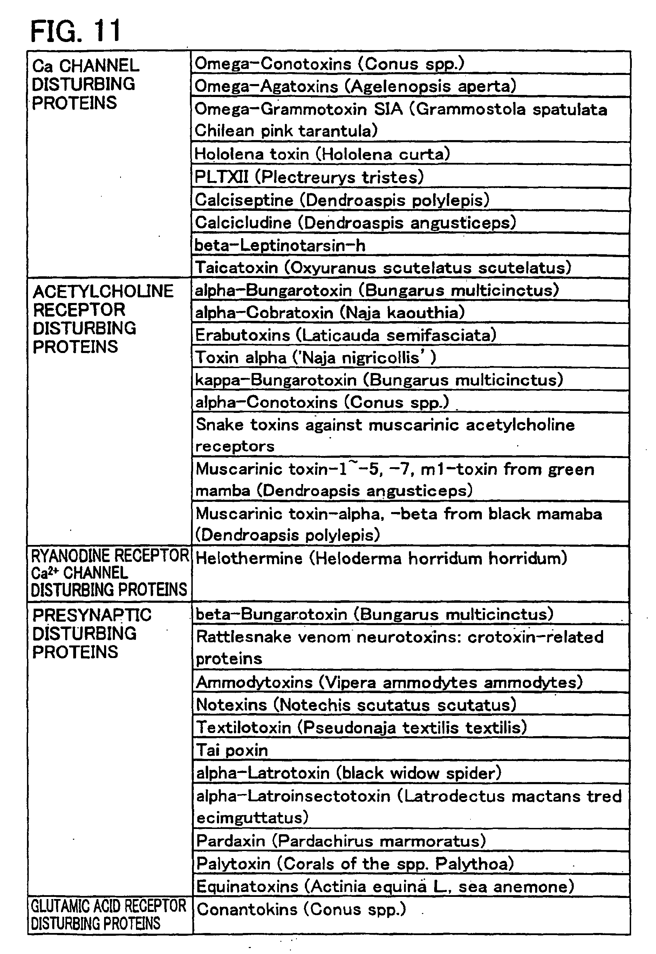

The present invention produces hollow protein nanoparticles by fusing a particle-forming protein with a substance (target-cell-substance) to be transferred into a target cell or tissue, and thereby provides a substance carrier having cell specificity. As mentioned above, the substance carrier may contain, for example, a protein drug (including a peptide), such as interferons (IFNα, IFNβ, IFNβ1 etc.), a hepatocytes growth factor (HGF) etc. Figures 9 through 11 show some other substance examples.

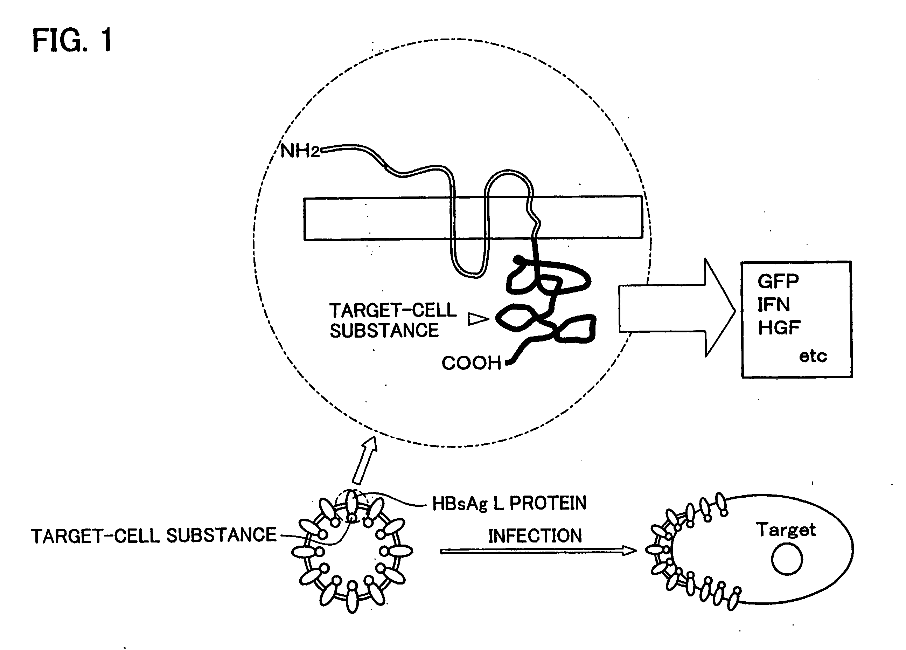

The step of fusing a particle-forming protein with a substance (target-cell-substance) to be transferred into a target cell or tissue is performed with a plasmid. The plasmid contains a gene encoding the hepatitis B virus surface-antigen protein, and also contains a gene encoding the protein drug on the downstream of the gene encoding the hepatitis B virus surface-antigen protein. Using such a plasmid, particles are formed in eukaryotic cell, thereby producing the drug of the present invention in which the hepatitis B virus surface-antigen protein, that forms the particles, is fused with a protein drug (see Figure 1).

The drug thus created can effectively deliver a drug specifically to a target cell. For example, by administrating the drug of the present invention, that is created by fusing particles of the hepatitis B virus surface-antigen protein with IFN, into a living body through intravenous injection, the particles circulate around the body and are lead to the hepatocytes by the hepatocyte-specifying receptor displayed on the particle surface, and finally infect the cell. Consequently, the IFN is transferred to the hepatocytes, that is, the IFN is specifically transferred inside the hepar tissue. Note that, the administration of the drug may also be performed through other method than intravenous injection, for example, oral administration, intramuscular administration intraabdominal administration, or subcutaneous administration.

A protein drug, such as interferon or interleukin, conventionally has strong side-effects, thereby putting a burden on the patient when the drug is administered systemically. For this reason, it has been required that the protein drug is transferred specifically to a target cell or tissue. As explained above, the present invention provides a drug selectively transferable to a specific cell or tissue, thus enabling the treatment without a burden on the patient even when using a drug having strong side effects.

As explained, the drug of the present invention allows a substance to be specifically transported into cells or tissues *in vivo* or *in vitro.* Specific transport of the substance into a specific cell or specific tissue may be used as a treatment method of various diseases, or one of the steps in the procedure of the treatment method.

In the following, the present invention will be described in more detail by way of Examples with reference to the attached drawings. It should be appreciated that the present invention is not limited in any ways by the following Examples, and various modifications to details of the invention are possible.

### [Examples]

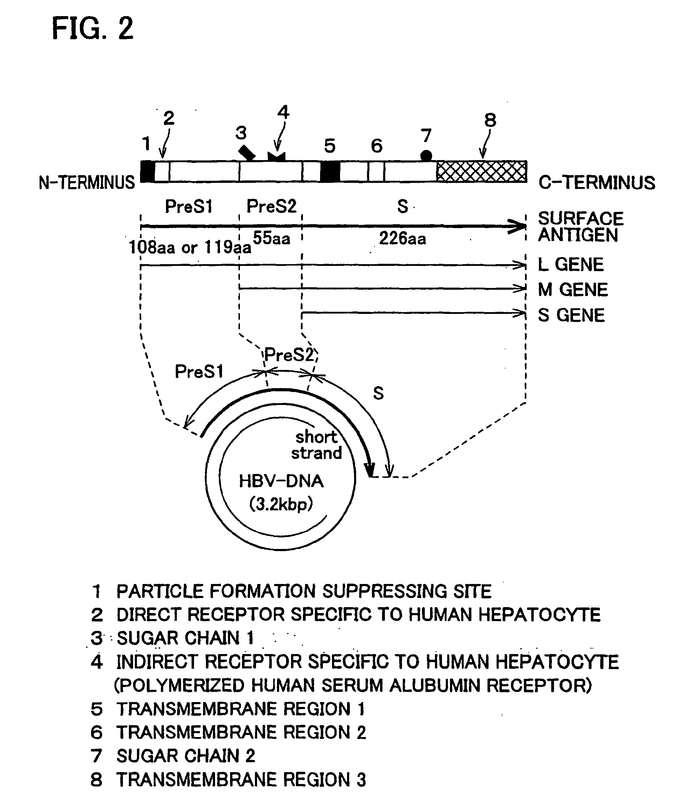

In the following, HBsAg refers to hepatitis B virus surface antigen. HBsAg is an envelope protein of HBV, and includes three kinds of proteins S, M, and L, as schematically illustrated in Figure 2. S protein is an important envelope protein common to all three kinds of proteins. M protein includes the entire sequence of the S protein with additional 55 amino acids (pre-S2 peptide) at the N-terminus. L protein contains the entire sequence of the M protein with additional 108 amino acids or 119 amino acids (pre-S1 peptide) at the N-terminus.

The pre-S regions (pre-S1, pre-S2) of HBV have important roles in the binding of HBV to the hepatocytes. The Pre-S1 region has a direct binding site for the hepatocytes, and the pre-S2 region has a polymeric albumin receptor that binds to the hepatocytes via polymeric albumin in the blood.

Expression of HBsAg in the eukaryotic cell causes the protein to accumulate as membrane protein on the membrane surface of the endoplasmic reticulum. The L protein molecules of HBsAg agglomerate and are released as particles into the ER lumen, carrying the ER membrane with them as they develop.

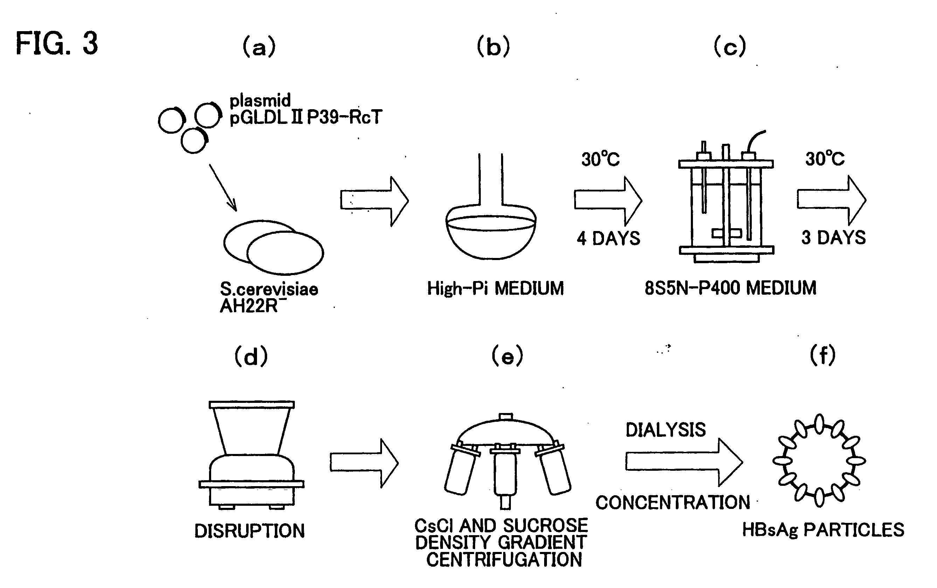

The Examples below used L proteins of HBsAg. Figure 3 briefly illustrates procedures of expression and purification of HBsAg particles described in the following Examples.

### (Example A) Expression of HBsAg particles in recombinant yeasts

Recombinant yeasts (*Saccharomyces cerevisiae* AH22R-strain) carrying (pGLDLIIP39-RcT) were cultured in synthetic media High-Pi and 8S5N-P400, and HBsAg L protein particles were expressed (Figures 3(a) through 3(c)). The whole procedure was performed according to the method described in J. Biol. Chem., Vol. 267, No. 3, 1953-1961, 1992 reported by the inventors of the present invention.

From the recombinant yeast in stationary growth phase (about 72 hours), the whole cell extract was obtained with the yeast protein extraction reagent (product of Pierce Chemical Co., Ltd.). The sample was then separated by sodium dodecyl sulfate-polyacrylamide gel electrophoresis (SDS-PAGE), and the HBsAg in the sample was identified by silver staining.

The result showed that HBsAg was a protein with a molecular weight of about 52 kDa.

### (Example B) Purification of HBsAg particles from the recombinant yeasts

(1) The recombinant yeast (wet weight of 26 g) cultured in synthetic medium 8S5N-P400 was suspended in 100 ml of buffer A (7.5 M urea, 0.1 M sodium phosphate, pH 7.2, 15 mM EDTA, 2 mM PMSF, and 0.1% Tween 80), and disrupted with glass beads by using a BEAD-BEATER. The supernatant was collected by centrifugation (Figures 3(c) and 3(d)).
(2) The supernatant was mixed with a 0.75 volume of PEG 6000 solution (33%, w/w), and cooled on ice for 30 min. The pellets were collected by centrifugation at 7000 rpm for 30 min, and resuspended in buffer A without Tween 80.
(3) The solution was layered onto a 10-40% CsCl gradient, and ultracentrifuged at 28000 rpm for 16 hours. The centrifuged sample was divided into 12 fractions, and each fraction was tested for the presence of HBsAg by Western blotting (the primary antibody was the anti-HBsAg monoclonal antibody). The HBsAg fractions were dialyzed against buffer A without Tween 80.
(4) 12 ml of the dialyzed solution obtained in (3) was layered onto a 5-50% sucrose gradient, and ultracentrifuged at 28000 rpm for 16 hours. As in (3), the centrifuged sample was divided into fractions, and each fraction was tested for the presence of HBsAg. The HBsAg fractions were dialyzed against buffer A containing 0.85% NaCl, without urea or Tween 80 ((2) through (4): Figure 3e).
(5) By repeating the procedure (4), the dizlyzed sample was concentrated with the ultrafilter Q2000 (Advantec), and stored at 4°C for later use (Figure 3(f)).

The result of Western blotting after CsCl equilibrium centrifugation in (3) revealed that HBsAg was a protein with S antigenicity with a molecular weight of 52 kDa. At the end of the procedure, about 24 mg of pure HBsAg particles were obtained from the yeast (26 g wet weight) derived from 2.5 L medium.

Each fraction obtained in the purification process was analyzed by silver staining SDS-PAGE. Further, in order to confirm whether the purification had successfully removed the yeast-derived protease, the HBsAg particles obtained in (5) were incubated at 37°C for 12 hours, separated by SDS-PAGE, and identified by silver staining.

The result of confirmation showed that the yeast-derived protease had been completely removed by the purification process.

### (Example C) Preparation of HBsAg particles fused with EGFP

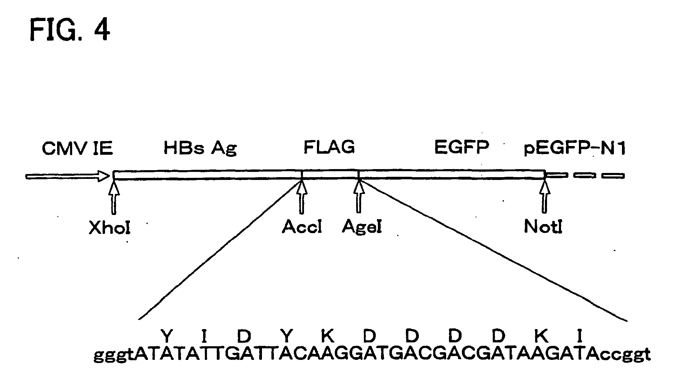

### (1) Preparation of a plasmid expressing a fusion protein of EGFP and HBsAg (see Figure 4)

By cutting the HBsAg-expressing plasmid pGLDLIIP39-RcT by XhoI and AccI, a gene fragment (hereinafter referred to as HBsAg gene), that encodes a HBVsAg L protein fused with chicken lysozym secretory signal, is obtained. Here, upstream side of HBsAg gene is cut by XhoI, and downstream side by AccI.

The plasmid pEGFP-N1 (pEGFP-F (product of Clontech)) has a gene fragment encoding a green fluorescent protein EGFP. This plasmid pEGFP-N1 is cut by XhoI and AgeI to be cleaved. Here, the plasmid is cleaved between the EGFP gene and the promoter (CMVIE), and upstream side of EGFP gene is cut by AgeI, and downstream side of the promoter is cut by XhoI.

Further, the fusion protein of HBsAg and EGFP can be detected using an anti-FLAG antibody, by inserting a FLAG tag (NH2-YIDYKDDDDKI-COOH), that is a well-known protein, between HBsAg and EGFP. To express a FLAG tag, an oligonucleotide with a sequence number 2, and an oligonucleotide with a sequence number 1 were prepared to be used respectively for sense-strand and antisense-strand. This synthetic DNA encoding the FLAG tag is designed to contain a restriction enzyme AccI site in the upstream side and contain a restriction enzyme Agel site in the downstream side.

The sites cut by the same restriction enzyme of the respective plasmids for expressing HBVsAg, FLAG tag, EGFP are bonded together by T4DNA ligase. With this process, the HBVsAg and FLAG tag are inserted between the promoter of EGFP-expressing plasmid and the EGFP gene, thereby constructing a plasmid pBOP001 containing genes of EGFP and HBsAg. In this connection, the genes inserted in the downstream side of the CMV promoter of the plasmid respectively encode proteins that are fused with, from the amino-terminus, chicken-lysozyme-derived secretory signal, HBVsAg L protein, FLAG tag, and EGFP protein.

### (2) Transfer of plasmid to monkey-kidney-derived cells COS-7, and the expression of the plasmid

After checking the base sequence of the genes, the plasmid pBOP001 was transferred to COS-7 cells derived from an African grivet kidney, using the gene transfer device gene pulser (Bio-Rad Laboratories, Inc.). After the transfer, the sample was inoculated in 16-holes well plates in an amount of 1×10⁴ cell for each plate, and was cultivated overnight in a Dulbecco-modified medium D-MEM containing 10% fetal bovine serum at 37°C under 5%CO₂. Next, day, the medium was replaced with a serum-free medium CHO-SFMII (Gibco-BRL), and further cultivated for four days. Then the medium containing COS-7 was collected.

First, the HBsAg-streptag particles in the medium were measured for the presence or absence of expression, and the expression was confirmed. Further, with the IMx kit (Dainabot Co. Ltd.), antigenicity of the medium was confirmed, and particles were detected in the medium.

Further, with the primary antibody fixed into the agarose beads of IMx, the particles in the medium were immunoprecipitated. The precipitated protein was then subjected to sodium dodecyl sulfate-polyacrylamide gel electrophoresis (SDS-PAGE), followed by Western blotting, detecting the protein by an anti FLAG antibody. As a result, a band with molecular amount of 80kDa was detected, and expression of the fusion protein in the intended form was confirmed.

Further, the fluorescence spectrum of EGFP was detected by excitation light 480nm with a flurophotometer. This confirmed that the original structures of HBsAg L protein and EGFP were kept in the particles of the expressed fusion protein.

### (3) Transfer of plasmid to yeast cells, and the expression of the plasmid

Further, to express the fusion protein in the yeast cells, a plasmid pBOP001 was cut at that site recognizing the restriction enzyme NotI, which exists on the side of the translation stop codon 3' of the EGFP gene, and the adhesion end was smoothed by a E. coli DNA polymerase large fragment. Then, by inserting Xhol linker 5'-CCTCCGAGG-3', the smoothed end was bonded and closed, thus constructing a plasmid. Then the site encoding the fusion protein was cut out from the plasmid by a restriction enzyme XhoI. Then, the HBsAg L protein contained in the plasmid pGLDLIIP39-RcT was replaced with the cut out site to form a plasmid pBOP002. This plasmid was used to transform yeast (Saccharomyces Cerevisiae AH22R-strain), and the resulting recombinant yeast was cultivated in synthetic mediums High-Pi and 8S5N-P400, thereby expressing a HBsAg L protein fused with a EGFP protein.

From the recombinant yeast in stationary growth phase (about 72 hours), the whole cell extract was obtained with the Yeast Protein Extraction Reagent (product of Pierce Chemical Co., Ltd.). Then, the HBsAg was tested for S-antigenicity with an IMx kit.

Further, with the primary antibody fixed into the agarose beads of IMx, the particles in the medium were immunoprecipitated. The precipitated protein was then subjected to SDS-PAGE, followed by Western blotting, detecting the protein by an anti FLAG antibody. As a result, a band with molecular amount of 80kDa was detected.

Further, the fluorescence spectrum of EGFP was detected by excitation light 480nm with a flurophotometer. This confirmed that the original structures of HBsAg L protein and EGFP were kept in the particles of the expressed fusion protein.

### (4) Transfer of plasmid to insect cells, and the expression of the plasmid.

Next, the XhoI fragment of the gene encoding the fusion protein was cut out from the plasmid pBOP002, and the adhesion end was smoothed by a E. coli DNA polymerase large fragment. Then, the smoothed end was inserted in EcoRV site of vector pIZT/V5-His (used for stable expression in insect cells) (Invitrogen Corporation) to close the ring. After confirming the base sequence, the plasmid was named pBOP003.

Meanwhile, the insect cell High Five line (BTI-TN-5B1-4): (Invitrogen Corporation) was slowly conditioned from the fetal bovine serum-contained medium to a serum-free medium (Ultimate Insect Serum-Free Medium: Invitrogen Corporation) over a period of about 1 month. Then, using the gene transfer lipid Insectin-Plus (Invitrogen Corporation), the plasmid pBOP003 was transferred for the transformation of the High Five line conditioned to the serum-free medium. The sample was incubated in the serum-free medium at 27°C for 48 hours, followed by further incubation that extended 4 to 7 days until confluent cells were obtained on the serum-free medium with the additional 400 µg/mL antibiotic zeocin (Invitrogen Corporation).

The sample was centrifuged at 1500 x g for 5 min, and the supernatant was collected. The HBsAg particles in the medium were measured for the presence or absence of expression, using the IMx kit (Dainabot Co. Ltd.). The result confirmed the expression of HBsAg particles. Further, with the primary antibody fixed into the agarose beads of IMx, the particles in the medium were immunoprecipitated. The precipitated protein was then subjected to SDS-PAGE, followed by Western blotting, detecting the protein by an anti FLAG M2 antibody. As a result, a band with molecular amount of 80kDa was detected, and expression of the fusion protein in the intended form was confirmed. Further, the fluorescence spectrum of EGFP was detected by excitation light 480nm with a flurophotometer. This confirmed that the original structures of HBsAg L protein and EGFP were kept in the particles of the expressed fusion protein.

The gene sequence of the HBsAg L protein fused with EGFP, and its amino-acid sequence are denoted by the sequence numbers 13 and 14, respectively.

### (Example D) Preparation of HBsAg particles fused with human interferon ω (IFNω)

### (1) Preparation of a plasmid expressing a fusion protein of IFNω and HBsAg

The plasmid pGT65-hIFN-α (InvivoGen) contains a gene fragment encoding IFNω. The gene fragment was used as a model to amplify the gene fragment encoding IFNω by the general PCR method.

The two kinds of PCR primer used here were oligonucleotides of the sequence number 3 and the sequence number 4. These primers are designed to contain Agel site in the upstream side and contains a restriction enzyme NotI site in the downstream side.

The PCR product was electrophorased on agarose, and a band containing the target cDNA was collected to be subcloned to a pCR2.1-TOPO vector (Invitrogen Corporation), using the TOPO TA Cloning kit (Invitrogen Corporation). The inserted base sequence was confirmed based on the document attached to the product: pORF-hIFNa v.11, and the cDNA fragment was cut out by the restriction enzymes Agel and NotI. Then, the EGFP gene of the foregoing plasmid pEGFP-N1 was replaced with the cDNA fragment using the Agel site and the NotI site, thereby constructing a plasmid pBOP004.

The FLAG tag gene and HBsAg gene were inserted in the plasmid pB0004 by the same method as that described in Example C(1), thereby constructing a plasmid pBOP005. In this construction, the genes inserted in the downstream side of the CMV promoter of the plasmid respectively encode proteins that are fused with, from the amino-terminus, chicken-lysozyme-derived secretory signal, HBVsAg L protein, FLAG tag, and IFNω.

### (2) Transfer of plasmid to monkey-kidney-derived cells COS-7, and the expression of the plasmid

After checking the base sequence of the genes, the plasmid pBOP005 was transferred to COS-7 cells derived from an African grivet kidney, using the gene transfer device gene pulser (Bio-Rad Laboratories, Inc.). After the transfer, the sample was inoculated in 16-holes well plates in an amount of 1×10⁴ cell for each plate, and was cultivated overnight in a Dulbecco-modified medium D-MEM containing 10% fetal bovine serum. Next, day, the medium was replaced with a serum-free medium CHO-SFMII (Gibco-BRL), and further cultivated for four days. Then the medium containing COS-7 was collected.

The S-antigenicity in the medium was confirmed by IMx kit (Dainabot Co. Ltd.) and particles were detected. Further, the particles in the medium were immunoprecipitated using the primary antibody fixed to the agarose beads. The precipitated protein was then subjected to SDS-PAGE, followed by Western blotting, detecting the protein by an anti FLAG M2 antibody. As a result, a band with molecular amount of 70kDa was detected, and expression of the fusion protein in the intended form was confirmed. This ensured that the original structures of HBsAg L protein and IFNω were kept in the particles of the expressed fusion protein.

### (3) Transfer of plasmid to yeast cells, and the expression of the plasmid

Further, to express the fusion protein in the yeast cells, a plasmid pBOP005 was cut at that site recognizing the restriction enzyme NotI, which exists on the side of the translation stop codon 3' of the IFNω gene, and the adhesion end was smoothed by a E. coli DNA polymerase large fragment. Then, by inserting XhoI linker 5'-CCTCCGAGG-3', the smoothed end was bonded and closed, thus constructing a plasmid. Then the site encoding the fusion protein was cut out from the plasmid by a restriction enzyme Xhol. Then, the HBsAg L protein contained in the plasmid pGLDLIIP39-RcT was replaced with the cut out site to form a plasmid pBOP006. This plasmid was used to transform yeast (Saccharomyces Cerevisiae AH22R-strain), and the resulting recombinant yeast was cultivated in synthetic mediums High-Pi and 8S5N-P400, thereby expressing a HBsAg L protein fused with a IFNω protein.

From the recombinant yeast in stationary growth phase (about 72 hours), the whole cell extract was obtained with the Yeast Protein Extraction Reagent (product of Pierce Chemical Co., Ltd.). Then, the HBsAg was tested for S-antigenicity with an IMx kit. Further, with the primary antibody fixed into the agarose beads of IMx, the particles in the medium were immunoprecipitated. The precipitated protein was then subjected to SDS-PAGE, followed by Western blotting, detecting the protein by an anti FLAG antibody. As a result, a band with molecular amount of 70kDa was detected. This confirmed that the original structures of HBsAg L protein and human interferon ω were kept in the particles of the expressed fusion protein in the yeast.

### (4) Transfer of plasmid to insect cells, and the expression of the plasmid

Next, the XhoI fragment of the gene encoding the fusion protein was cut out from the plasmid pBOP006, and the adhesion end was smoothed by a E. coli DNA polymerase large fragment. Then, the smoothed end was inserted in EcoRV site of vector pIZT/VS-His (used for stable expression in insect cells) (Invitrogen Corporation) to close the ring. After confirming the base sequence, the plasmid was named pBOP007.

Meanwhile, the insect cell High Five line (BTI-TN-5B1-4): (Invitrogen Corporation) was slowly conditioned from the fetal bovine serum-contained medium to a serum-free medium (Ultimate Insect Serum-Free Medium: Invitrogen Corporation) over a period of about 1 month. Then, using the gene transfer lipid Insectin-Plus (Invitrogen Corporation), the plasmid pBOP007 was transferred for the transformation of the High Five line conditioned to the serum-free medium. The sample was incubated in the serum-free medium at 27°C for 48 hours, followed by further incubation that extended 4 to 7 days until confluent cells were obtained on the serum-free medium with the additional 400 µg/mL antibiotic zeocin (Invitrogen Corporation).

The sample was centrifuged at 1500 × g for 5 min, and the supernatant was collected. The HBsAg particles in the medium were measured for the presence or absence of expression, using the IMx kit (Dainabot Co. Ltd.). The result confirmed the expression of HBsAg particles. Further, with the primary antibody fixed into the agarose beads of IMx, the particles in the medium were immunoprecipitated. The precipitated protein was then subjected to SDS-PAGE, followed by Western blotting, detecting the protein by an anti FLAG M2 antibody. As a result, a band with molecular amount of 70kDa was detected, and expression of the fusion protein in the intended form was confirmed. This confirmed that the original structures of HBsAg L protein and human interferon ω were kept in the particles of the expressed fusion protein.

The gene sequence of the HBsAg L protein fused with human interferon ω, and its amino-acid sequence are denoted by the sequence numbers 15 and 16, respectively.

### (Example E) Preparation of HBsAg particles fused with human interferon β1 (IFNβ1)

### (1) Preparation of a plasmid expressing a fusion protein of IFNβ1 and HBsAg

The plasmid pGT65-hIFN-α (InvivoGen) contains a gene fragment encoding IFNβ1. The gene fragment was used as a model to amplify the gene fragment encoding IFNβ1 by the general PCR method.

The two kinds of PCR primer used here were an oligonucleotide with a sequence number 5 for sense-strand, and an oligonucleotide with a sequence number 6 for antisense-strand. These primers are designed to contain AgeI site in the upstream side and contains a restriction enzyme NotI site in the downstream side.

The PCR product was electrophorased on agarose, and a band containing the target cDNA was collected to be subcloned to a pCR2.1-TOPO vector (Invitrogen Corporation), using the TOPO TA Cloning kit (Invitrogen Corporation). The inserted base sequence was confirmed based on the reference (GenBank accession no. M28622), and the cDNA fragment was cut out by the restriction enzymes Agel and NotI. Then, the EGFP gene of the foregoing plasmid pEGFP-N1 was replaced with the cDNA fragment using the AgeI site and the NotI site, thereby constructing a plasmid.

The FLAG tag gene and HBsAg gene were inserted in the plasmid by the same method as that described in Example C(1), thereby constructing a plasmid pBOP008. In this construction, the genes inserted in the downstream side of the CMV promoter of the plasmid respectively encode proteins that are fused with, from the amino-terminus, chicken-lysozyme-derived secretory signal, HBVsAg L protein, FLAG tag, and IFNβ 1.

### (2) Transfer of plasmid to monkey-kidney-derived cells COS-7, and the expression of the plasmid

After checking the base sequence of the genes, the plasmid pBOP008 was transferred to COS-7 cells derived from an African grivet kidney, using the gene transfer device gene pulser (Bio-Rad Laboratories, Inc.). After the transfer, the sample was inoculated in 16-holes well plates in an amount of 1×10⁴ cell for each plate, and was cultivated overnight in a Dulbecco-modified medium D-MEM containing 10% fetal bovine serum. Next, day, the medium was replaced with a serum-free medium CHO-SFMII (Gibco-BRL), and further cultivated for another week. Then the medium containing COS-7 was collected.

The S-antigenicity in the medium was confirmed by IMx kit (Dainabot Co. Ltd.) and particles were detected. Further, the particles in the medium were immunoprecipitated using the primary antibody fixed to the agarose beads. The precipitated protein was then subjected to SDS-PAGE, followed by Western blotting, detecting the protein by an anti FLAG M2 antibody. As a result, a band with molecular amount of 70kDa was detected, and expression of the fusion protein in the intended form was confirmed. This ensured that the original structures of HBsAg L protein and IFNβ1 were kept in the particles of the expressed fusion protein.

### (3) Transfer of plasmid to yeast cells, and the expression of the plasmid

Further, to express the fusion protein in the yeast cells, a plasmid pBOP008 was cut at that site recognizing the restriction enzyme NotI, which exists on the side of the translation stop codon 3' of the IFNβ1 gene, and the adhesion end was smoothed by a E. coli DNA polymerase large fragment. Then, by inserting Xhol linker 5'-CCTCCGAGG-3', the smoothed end was bonded and closed, thus constructing a plasmid. Then the site encoding the fusion protein was cut out from the plasmid by a restriction enzyme XhoI. Then, the HBsAg L protein contained in the plasmid pGLDLIIP39-RcT was replaced with the cut out site to form a plasmid pBOP009. This plasmid was used to transform yeast (Saccharomyces Cerevisiae AH22R-strain), and the resulting recombinant yeast was cultivated in synthetic mediums High-Pi and 8SSN-P400, thereby expressing a HBsAg L protein fused with a IFNβ1 protein.

From the recombinant yeast in stationary growth phase (about 72 hours), the whole cell extract was obtained with the Yeast Protein Extraction Reagent (product of Pierce Chemical Co., Ltd.). Then, the HBsAg was tested for S-antigenicity with an IMx kit. Further, with the primary antibody fixed into the agarose beads of IMx, the particles in the medium were immunoprecipitated. The precipitated protein was then subjected to SDS-PAGE, followed by Western blotting, detecting the protein by an anti FLAG antibody. As a result, a band with molecular amount of 70kDa was detected. This confirmed that the original structures of HBsAg L protein and human interferon β1 were kept in the particles of the expressed fusion protein in the yeast.

### (4) Transfer of plasmid to insect cells, and the expression of the plasmid

Next, the XhoI fragment of the gene encoding the fusion protein was cut out from the plasmid pBOP009, and the adhesion end was smoothed by a E. coli DNA polymerase large fragment. Then, the smoothed end was inserted in EcoRV site of vector pIZT/V5-His (used for stable expression in insect cells) (Invitrogen Corporation) to close the ring. After confirming the base sequence, the plasmid was named pBOP0010.

Meanwhile, the insect cell High Five line (BTI-TN-5B1-4): (Invitrogen Corporation) was slowly conditioned from the fetal bovine serum-contained medium to a serum-free medium (Ultimate Insect Serum-Free Medium: Invitrogen Corporation) over a period of about 1 month. Then, using the gene transfer lipid Insectin-Plus (Invitrogen Corporation), the plasmid pBOP0010 was transferred for the transformation of the High Five line conditioned to the serum-free medium. The sample was incubated in the serum-free medium at 27°C for 48 hours, followed by further incubation that extended 4 to 7 days until confluent cells were obtained on the serum-free medium with the additional 400 µg/mL antibiotic zeocin (Invitrogen Corporation).

The sample was centrifuged at 1500 × g for 5 min, and the supernatant was collected. The HBsAg particles in the medium were measured for the presence or absence of expression, using the IMx kit (Dainabot Co. Ltd.). The result confirmed the expression of HBsAg particles. Further, with the primary antibody fixed into the agarose beads of IMx, the particles in the medium were immunoprecipitated. The precipitated protein was then subjected to SDS-PAGE, followed by Western blotting, detecting the protein by an anti FLAG M2 antibody. As a result, a band with molecular amount of 70kDa was detected, and expression of the fusion protein in the intended form was confirmed. This confirmed that the original structures of HBsAg L protein and human interferon β1 were kept in the particles of the expressed fusion protein.

The gene sequence of the HBsAg L protein fused with human interferon β1, and its amino-acid sequence are denoted by the sequence numbers 17 and 18, respectively.

### (Example F) Preparation of HBsAg particles fused with human hepatocyte growth factor (HGF)

### (1) Preparation of a plasmid expressing a fusion protein of HGF and HBsAg

A synthetic cDNA was made from a human-hepar-derived RNA (CloneTech) with a reverse transcriptase super script II (Gibco-BRL) using an Oligo-dT primer. The obtained cDNA was subjected to PCR using oligonucleotides of the sequence number 7 and the sequence number 8 as primers, that specifically amplify the HGF genes, thereby producing another 2.2kbp HGF genes. These primers are designed to contain AgeI site in the upstream side and contains a restriction enzyme NotI site in the downstream side.

The PCR product was electrophorased on agarose, and a band containing the target cDNA (about 2.2kbp) was collected to be subcloned to a pCR2.1-TOPO vector (Invitrogen Corporation), using the TOPO TA Cloning kit (Invitrogen Corporation).

Next, the two restriction enzyme recognizing-sites of the HGF gene are modified for easy construction of the plasmid. The following describes the procedure.

The plasmid DNA was subjected to PCR with QuickChange™ Site-Directed Mutagenesis Kit (Stratagene Corporation), using two complementary synthetic DNAs, respectively made of an oligonucleotide of the sequence number 9 and a complementary oligonucleotide of the sequence number 10, and a oligonucleotide of the sequence number 11 and a complementary oligonucleotide of the sequence number 12.

The first PCR was done with the first pair of primers, using Pfu DNA polymerase (Stratagene) as a heat-resistant DNA polymerase. The PCR was run in 30 cycles as follows: 30 second denature at 95°C, 1 minute annealing at 55°C, and 30 minute synthesis at 68°C. The PCR product was treated with restriction enzyme DpnI and transformed into E. coli DH5α. Then, the resulting E. coli DH5α was cultivated, and vector DNA was extracted from the resultant colonies, and the extract was screened for mutant plasmid based on the base sequence. Next, using the obtained plasmid as a model, the same process was repeated with the second pair of primers. Eventually, obtained was a plasmid pBOP011 carrying a human HGFcDNA in which the two Xhol-recognizing sites are deleted, but still having the same amino-acid coded by the HGFcDNA.

After checking the base sequence of the genes based on the reference (GenBank accession no. M29145), the cDNA fragment was cut out by the restriction enzymes Agel and NotI. Then, the EGFP gene of the foregoing plasmid pEGFP-N1 was replaced with the cDNA fragment using the AgeI site and the NotI site, thereby constructing a plasmid.

The FLAG tag gene and HBsAg gene were inserted in the plasmid by the same method as that described in Example C(1), thereby constructing a plasmid pBOP012. In this construction, the genes inserted in the downstream side of the CMV promoter of the plasmid respectively encode proteins that are fused with, from the amino-terminus, chicken-lysozyme-derived secretory signal, HBVsAg L protein, FLAG tag, and human HGF.

### (2) Transfer of plasmid to monkey-kidney-derived cells COS-7, and the expression of the plasmid

After checking the base sequence of the genes, the plasmid pBOP012 was transferred to COS-7 cells derived from an African grivet kidney, using the gene transfer device gene pulser (Bio-Rad Laboratories, Inc.). After the transfer, the sample was inoculated in 16-holes well plates in an amount of 1×10⁴ cell for each plate, and was cultivated overnight in a Dulbecco-modified medium D-MEM containing 10% fetal bovine serum. Next, day, the medium was replaced with a serum-free medium CHO-SFMII (Gibco-BRL), and further cultivated for four days. Then the medium containing COS-7 was collected.

The S-antigenicity in the medium was confirmed by IMx kit (Dainabot Co. Ltd.) and particles were detected. Further, the particles in the medium were immunoprecipitated using the primary antibody fixed to the agarose beads. The precipitated protein was then subjected to SDS-PAGE, followed by Western blotting, detecting the protein by an anti FLAG M2 antibody. As a result, a band with molecular amount of 125kDa was detected, and expression of the fusion protein in the intended form was confirmed. This ensured that the original structures of HBsAg L protein and human HGF were kept in the particles of the expressed fusion protein.

### (3) Transfer of plasmid to yeast cells, and the expression of the plasmid

Further, to express the fusion protein in the yeast cells, a plasmid pBOP012 was cut at that site recognizing the restriction enzyme NotI, which exists on the side of the translation stop codon 3' of the Human HGF gene, and the adhesion end was smoothed by a E. coli DNA polymerase large fragment. Then, by inserting XhoI linker 5'-CCTCCGAGG-3', the smoothed end was bonded and closed, thus constructing a plasmid. Then the site encoding the fusion protein was cut out from the plasmid by a restriction enzyme XhoI. Then, the HBsAg L protein contained in the plasmid pGLDLIIP39-RcT was replaced with the cut out site to form a plasmid pBOP013. This plasmid was used to transform yeast (Saccharomyces Cerevisiae AH22R-strain), and the resulting recombinant yeast was cultivated in synthetic mediums High-Pi and 8S5N-P400, thereby expressing a HBsAg L protein fused with a Human HGF protein.

From the recombinant yeast in stationary growth phase (about 72 hours), the whole cell extract was obtained with the Yeast Protein Extraction Reagent (product of Pierce Chemical Co., Ltd.). Then, the HBsAg was tested for S-antigenicity with an IMx kit. Further, with the primary antibody fixed into the agarose beads of IMx, the particles in the medium were immunoprecipitated. The precipitated protein was then subjected to SDS-PAGE, followed by Western blotting, detecting the protein by an anti FLAG antibody. As a result, a band with molecular amount of 125kDa was detected. This confirmed that the original structures of HBsAg L protein and human HGF were kept in the particles of the expressed fusion protein in the yeast.

### (4) Transfer of plasmid to insect cells, and the expression of the plasmid

Next, the XhoI fragment of the gene encoding the fusion protein was cut out from the plasmid pBOP013, and the adhesion end was smoothed by a E. coli DNA polymerase large fragment. Then, the smoothed end was inserted in EcoRV site of vector pIZT/V5-His (used for stable expression in insect cells) (Invitrogen Corporation) to close the ring. After confirming the base sequence, the plasmid was named pBOP014.

Meanwhile, the insect cell High Five line (BTI-TN-5B1-4): (Invitrogen Corporation) was slowly conditioned from the fetal bovine serum-contained medium to a serum-free medium (Ultimate Insect Serum-Free Medium: Invitrogen Corporation) over a period of about 1 month. Then, using the gene transfer lipid Insectin-Plus (Invitrogen Corporation), the plasmid pBOP007 was transferred for the transformation of the High Five line conditioned to the serum-free medium. The sample was incubated in the serum-free medium at 27°C for 48 hours, followed by further incubation that extended 4 to 7 days until confluent cells were obtained on the serum-free medium with the additional 400 µg/mL antibiotic zeocin (Invitrogen Corporation).

The sample was centrifuged at 1500 × g for 5 min, and the supernatant was collected. The HBsAg particles in the medium were measured for the presence or absence of expression, using the IMx kit (Dainabot Co. Ltd.). The result confirmed the expression of HBsAg particles. Further, with the primary antibody fixed into the agarose beads of IMx, the particles in the medium were immunoprecipitated. The precipitated protein was then subjected to SDS-PAGE, followed by Western blotting, detecting the protein by an anti FLAG M2 antibody. As a result, a band with molecular amount of 125kDa was detected, and expression of the fusion protein in the intended form was confirmed. This confirmed that the original structures of HBsAg L protein and human HGF were kept in the particles of the expressed fusion protein.

The gene sequence of the HBsAg L protein fused with human HGF, and its amino-acid sequence are denoted by the sequence numbers 19 and 20, respectively.(Example G) Transfer of GFP to human hepatic cancer cells by HBsAg L protein particles.

Human hepatic cancer cells HepG2 in exponential growth phase were implanted in a 3.5cm glass bottomed Petri dish with 1 × 10⁵ cells for each well, and cultivated overnight in a D-MEM containing 10% fetal bovine serum at 37°C under 5%CO2. Next day, HBsAg L protein particles fused with EGFP, that were used in Example C, were added to the dish with 10µg for each well, and further cultivated at 37°C under 5%CO2 for six hours.

Further, for negative control, human squamous-carcinoma-derived cells A431 (JCRB9009) are cultivated in the same manner.

The expression of GFP in HepG2 and A431 cells was observed by a confocal laser fluorometry microscope.

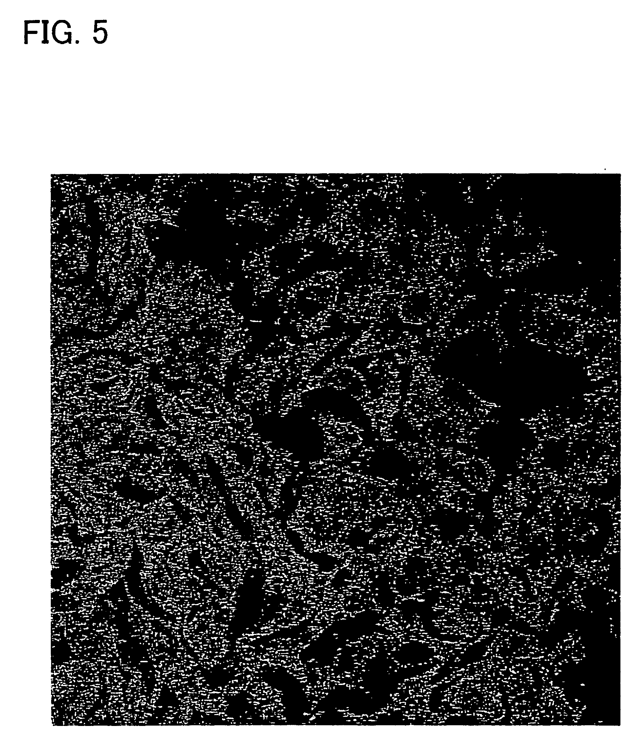

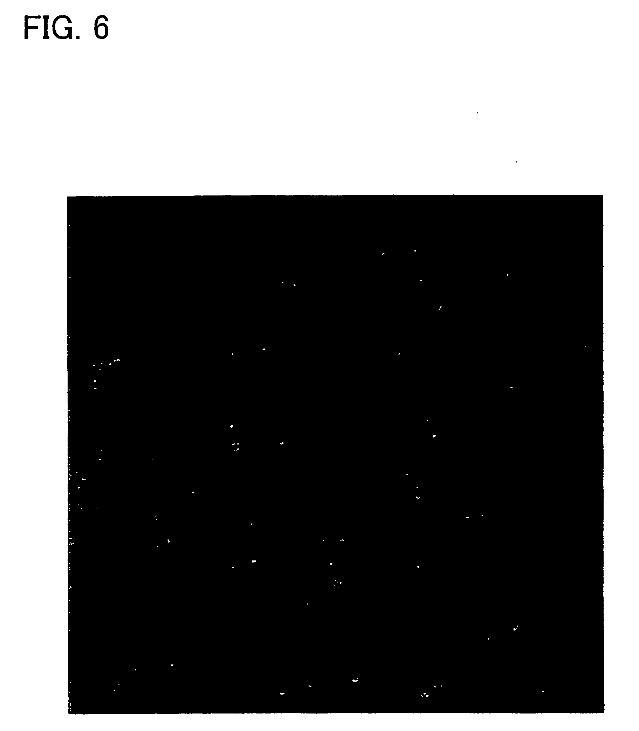

Through this observation, GFP-derived fluorescence was observed in the human hepatic cancer cells HepG2 (Figure 5); on the other hand, no fluorescence was observed in the human squamous-carcinoma-derived cells A431 (Figure 6).

As described, it was shown that the use of HBsAg L protein particles allows highly specific and efficient transfer of a protein into human hepatocytes, without changing the structure of the protein. That is, it has been proved that the substance carrier of the present invention is significantly effective.

### (Example H) Transfer of substance by HBsAg L protein particles with respect to nude mice that have been implanted with human hepatic cancer

Human hepatic-cancer-derived cells HuH-7 (JCRB0403) were hypodermically injected into nude mice (lineage: BALB/c, nu/nu, microbiological quality: SPF, male, 5 weeks of age). The injection was made in the bilateral dorsal area of the mouse with 1 × 10⁷ cells for each strain. In order to obtain a carrier mice, the mice were grown for 2 to 4 weeks until the transplanted tumor developed into a solid cancer tumor of about 2 cm diameter.

Further, for negative control, human squamous-carcinoma-derived cells A431 (JCRB9009) are cultivated in the same manner.

50 µg of the particles of HBsAg L protein fused with EGFP used in Example C were administered into the abdomen of each mouse with a 26G syringe. The mouse was killed 12 hours after the administration, and the tumor area was removed along with various organs including liver, spleen, kidney, and intestines. The tissues were fixed and embedded using the GFP resin embedding kit (Technovit7100).

Specifically, the samples were fixed by immersing them in 4% neutralized formaldehyde, and were dried in 70% EtOH at room temperature for 2 hours, 96% EtOH at room temperature for 2 hours, and 100% EtOH at room temperature for one hour. Pre-fixation was carried out for 2 hours at room temperature in a mixture containing equal amounts of 100% EtOH and Technovit7100. The samples were further immersed in Technovit7100 for no longer than 24 hours at room temperature. Out of the solution, the samples were allowed to stand for one hour at room temperature and for another one hour at 37°C for polymerization.

According to ordinary method, the sample were sliced and stained with hematoxin-eosin (common method of tissue staining). GFP fluorescence of each slice was observed with a fluorescent microscope.

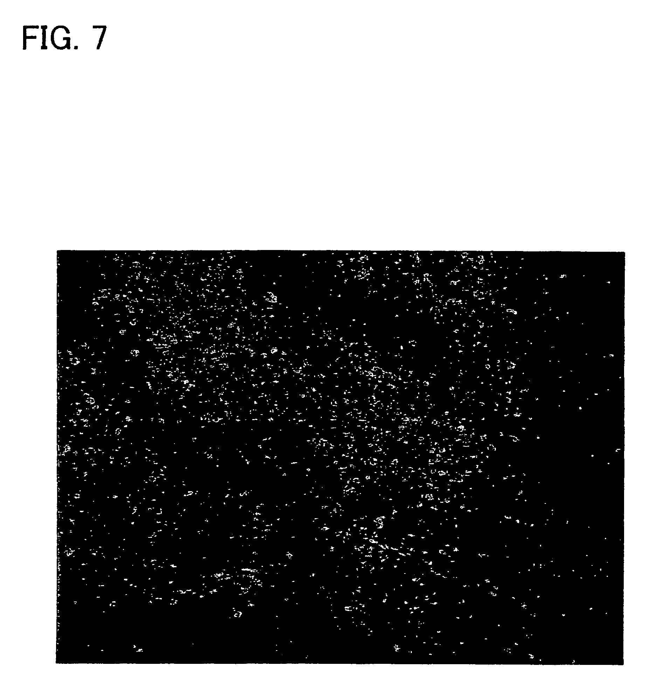

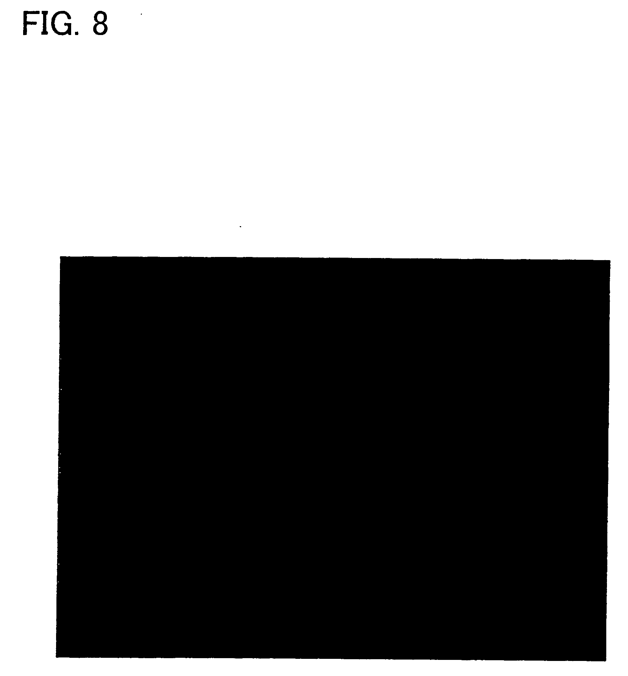

The result showed that the mouse carrying the human-hepatic-cancer-derived. cells HuH-7 had GFP fluorescence in the tumor area (Figure 7), but no fluorescence was observed in the organs removed from the same mouse, including liver, spleen, kidney, and intestines. Further, in carrier mice that have incorporated cells derived from human colon cancer WiDr, no GFP fluorescence was observed in the tumor area, or in the liver, spleen, kidney, or intestines (Tumor area: Figure 8).

With the foregoing experiments, it was shown that the use of HBsAg L protein particles allows highly specific and efficient transfer of a protein into human hepatocytes even on the laboratory animal level, without changing the structure of the protein. That is, it has been proved that the substance carrier of the present invention is significantly effective.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

### INDUSTRIAL APPLICABILITY

As described above, the present invention provides a drug enabling selective and effective transfer of a disease-treating target-cell-substance to specific diseased cells or tissues, by a convenient method, such as intravenous injection. The invention is a great leap forward from conventional gene therapy in that it does not require any surgical operation, and that the risk of side effect is greatly reduced. Further, since the target-cell-substance is fused with the protein that forms particles, it may be encapsulated in the particles upon preparation of the particles, thus offering easy manufacturing.

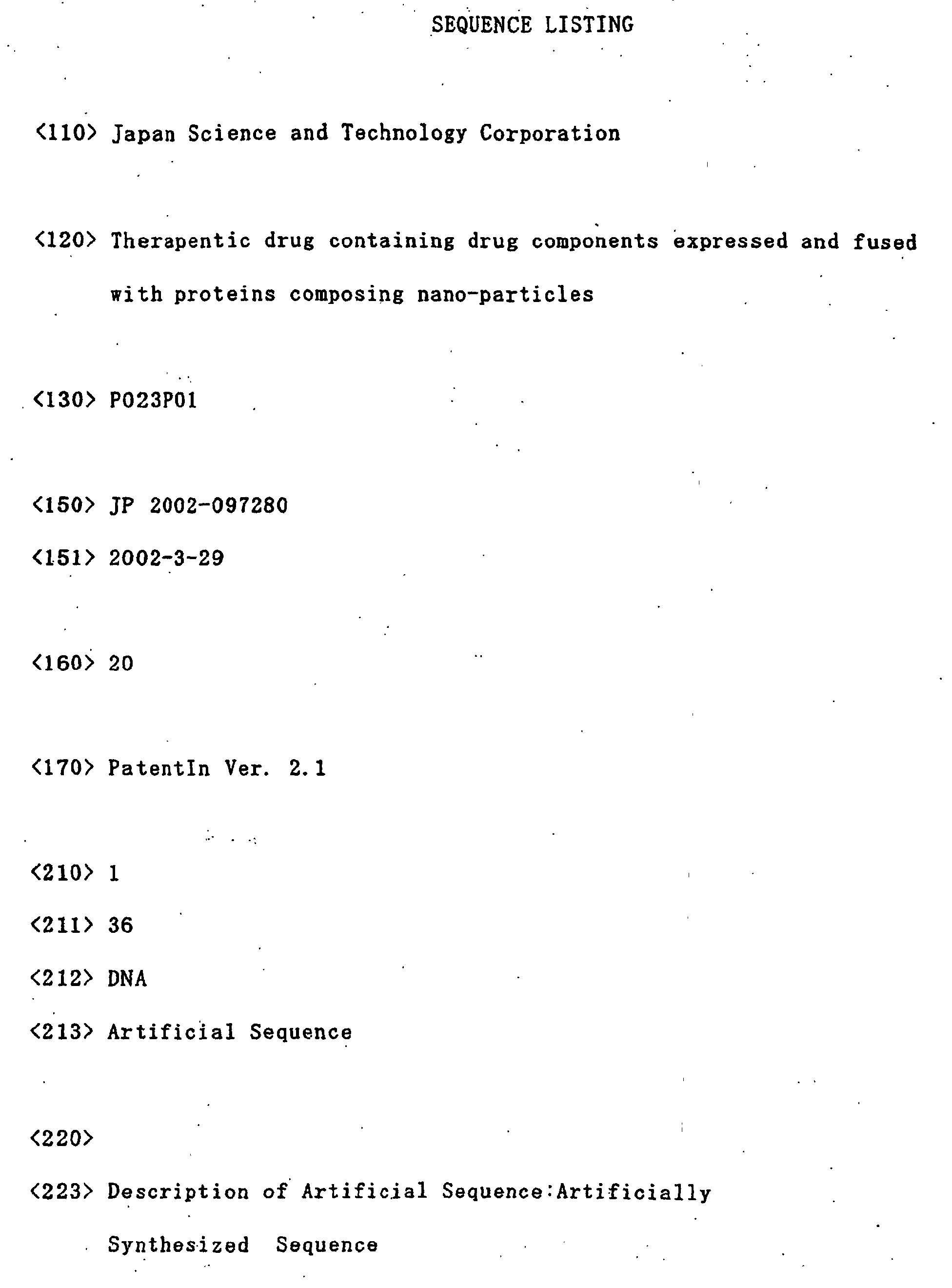

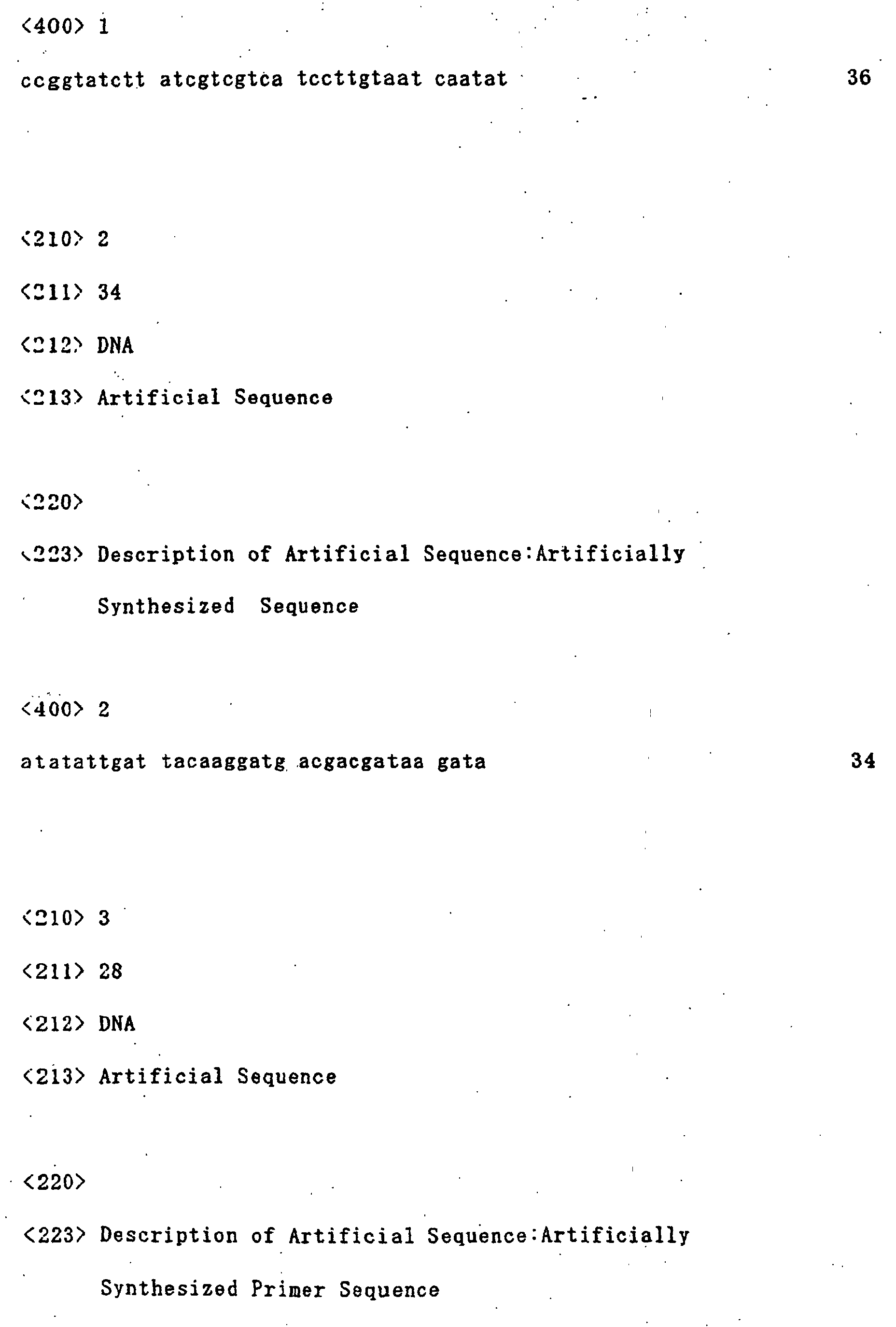

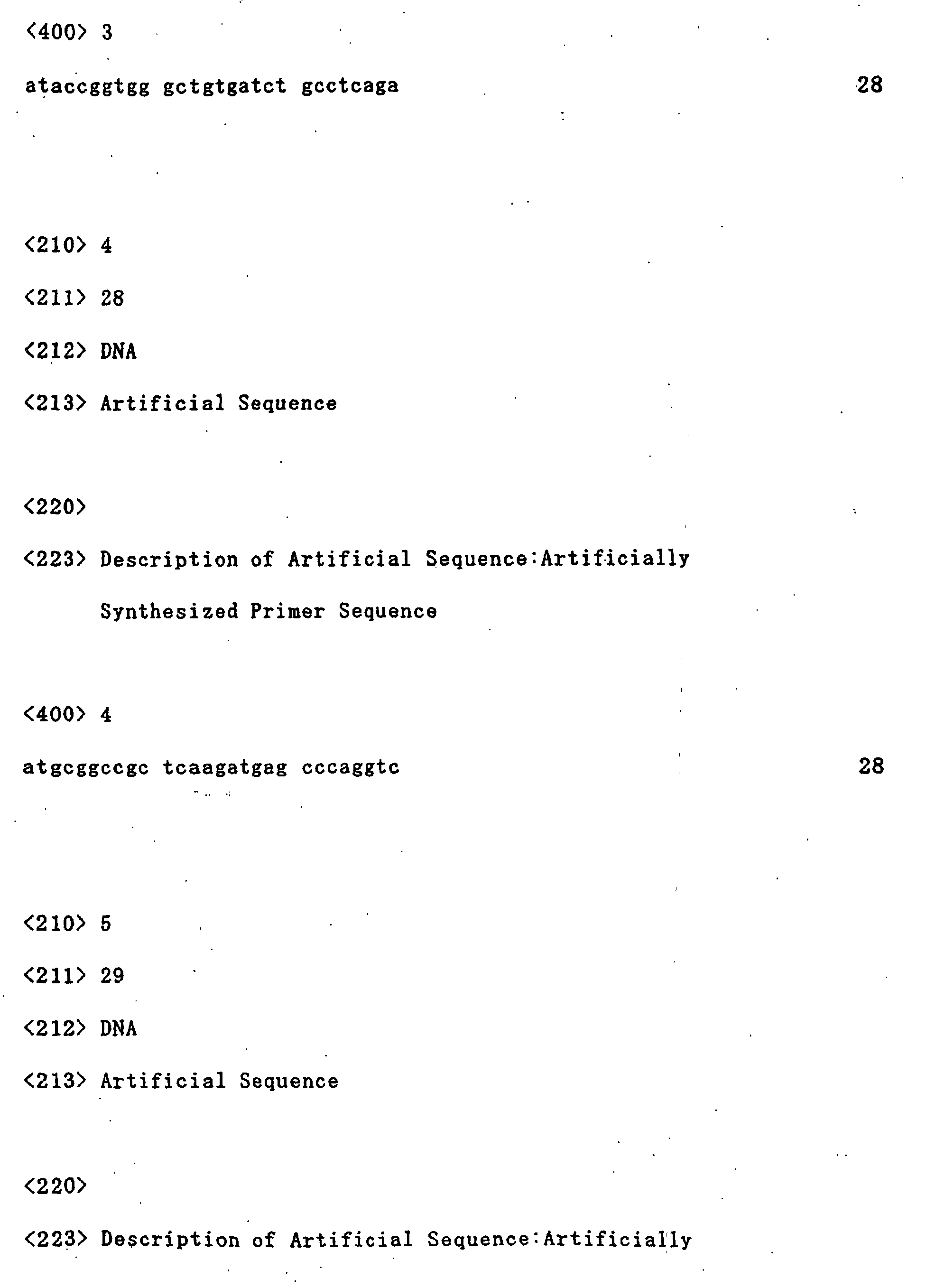

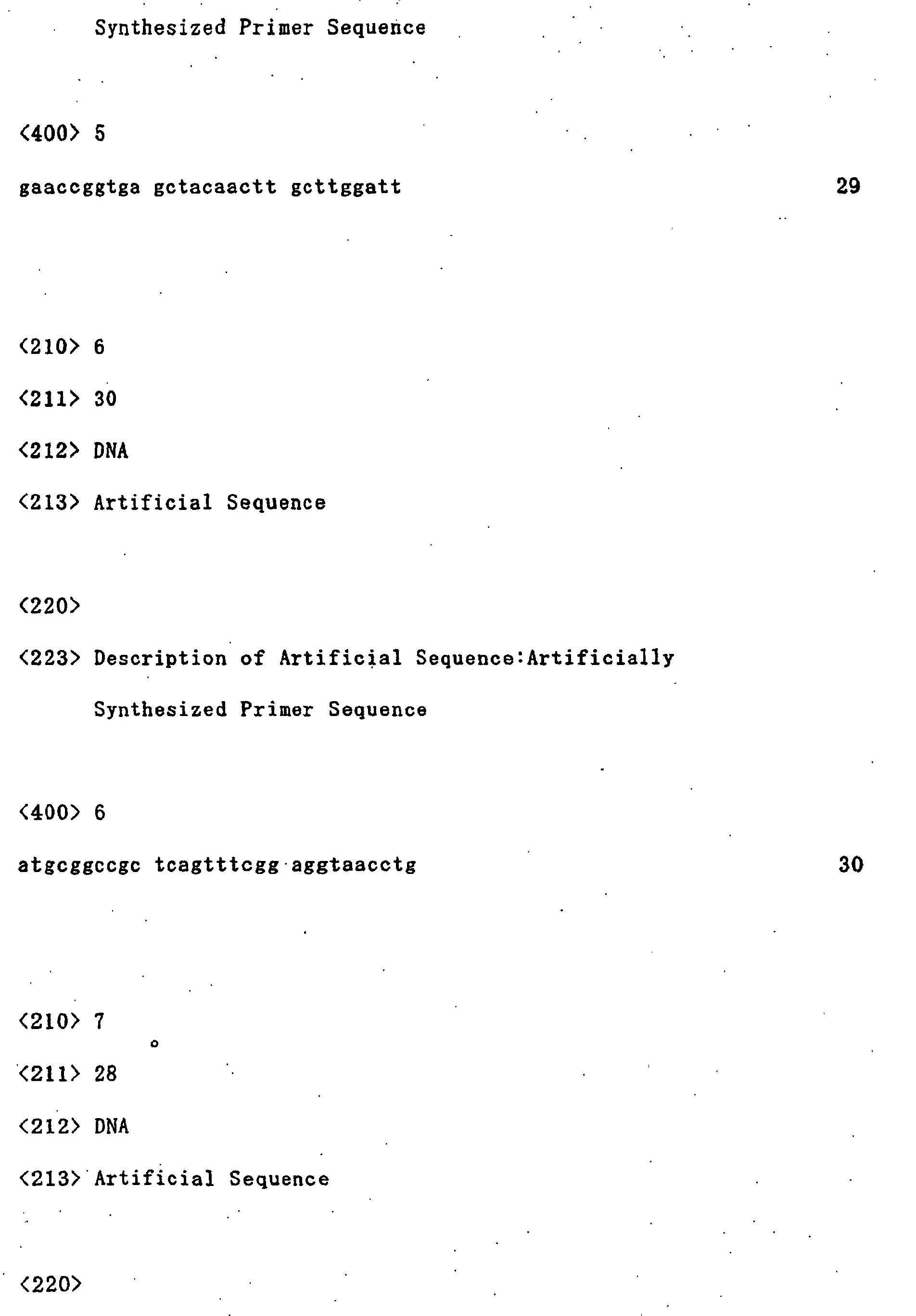

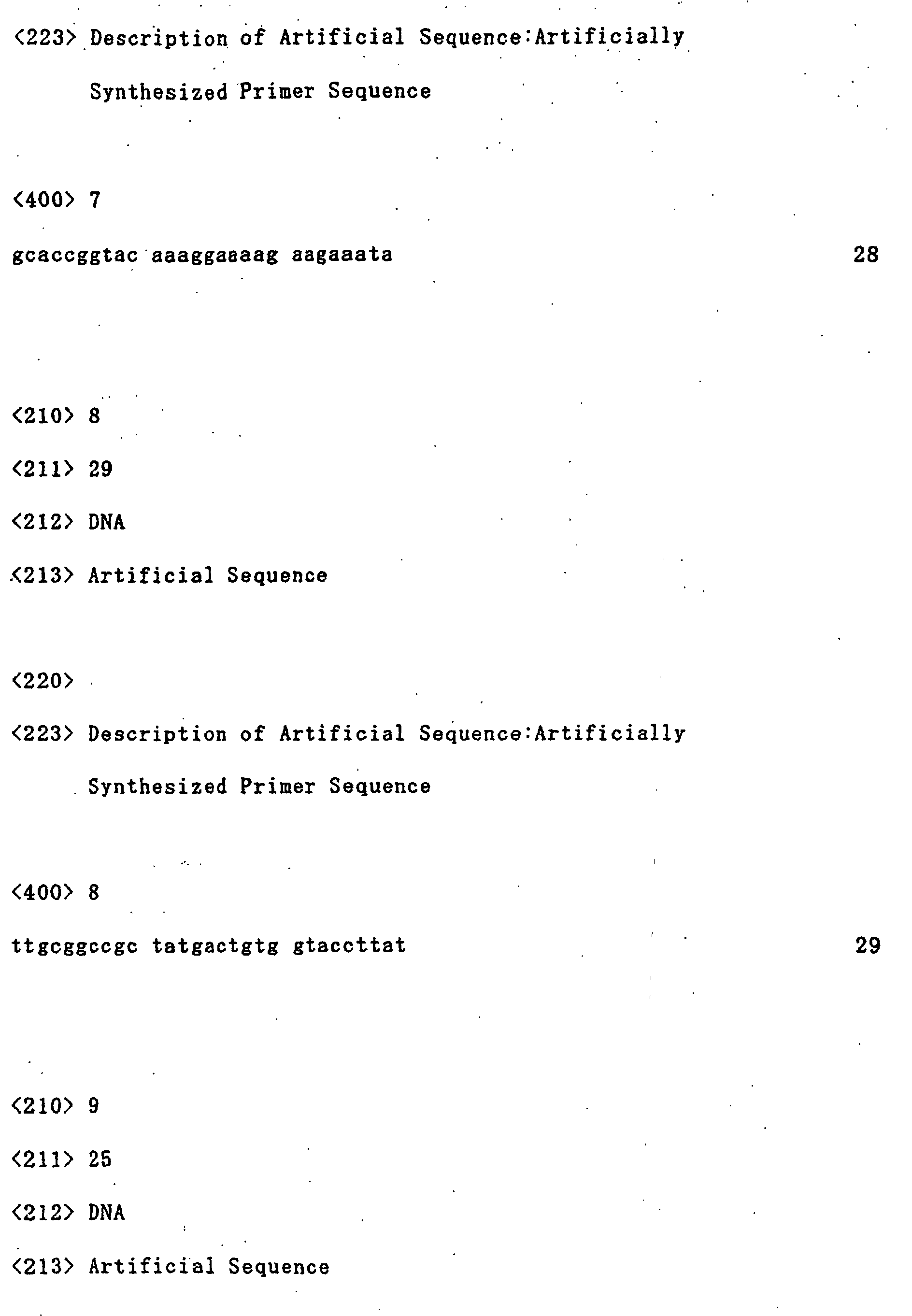

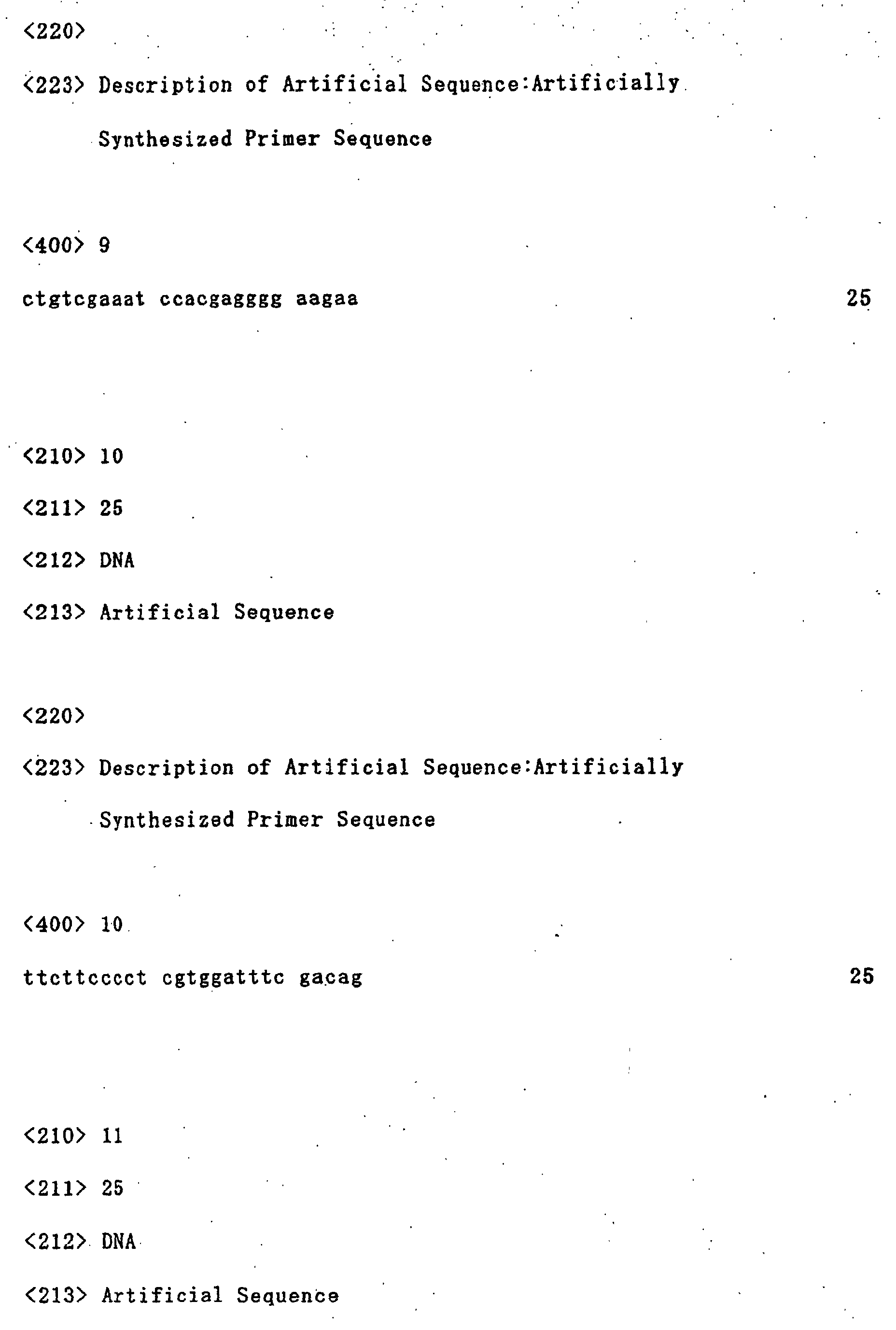

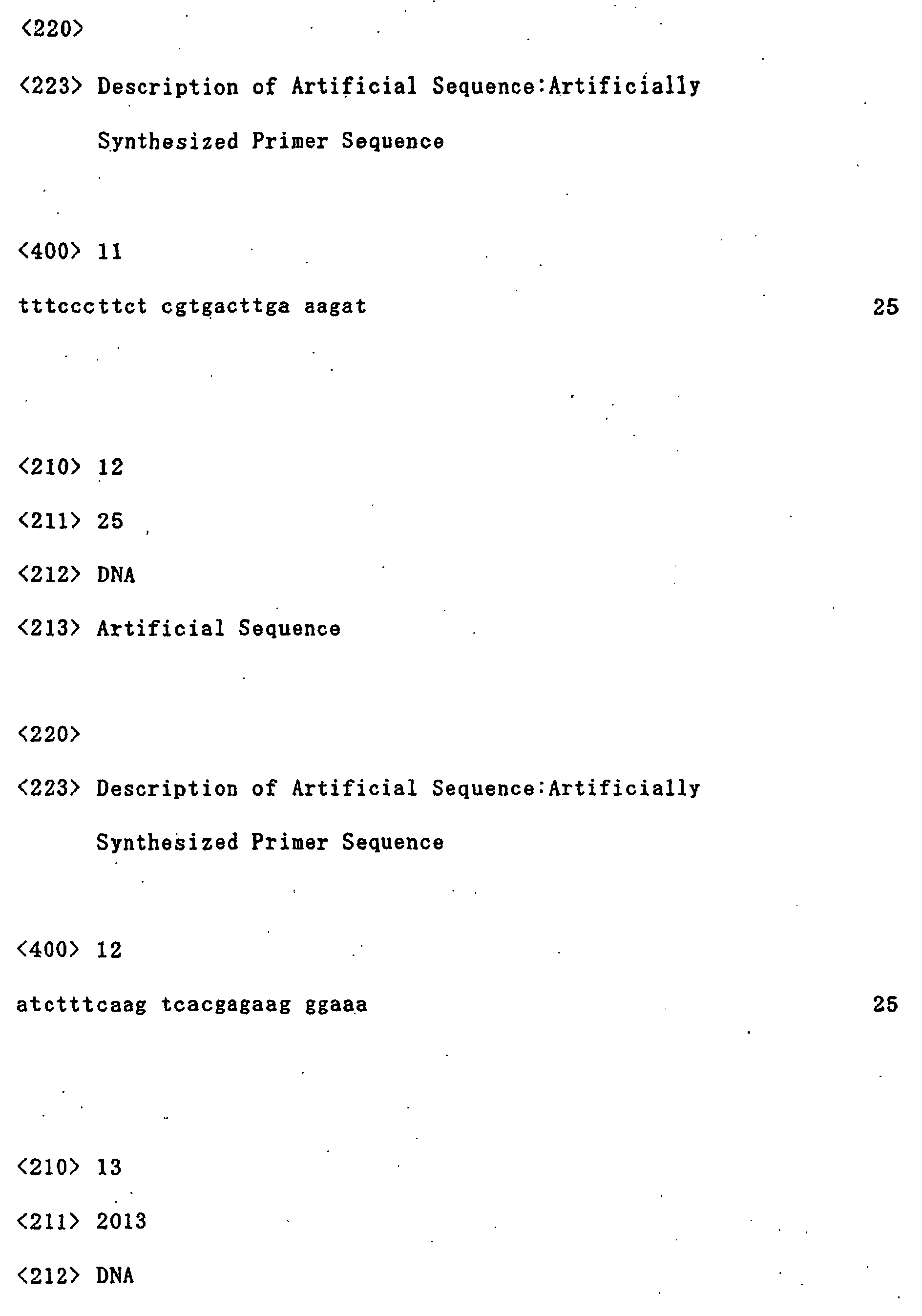

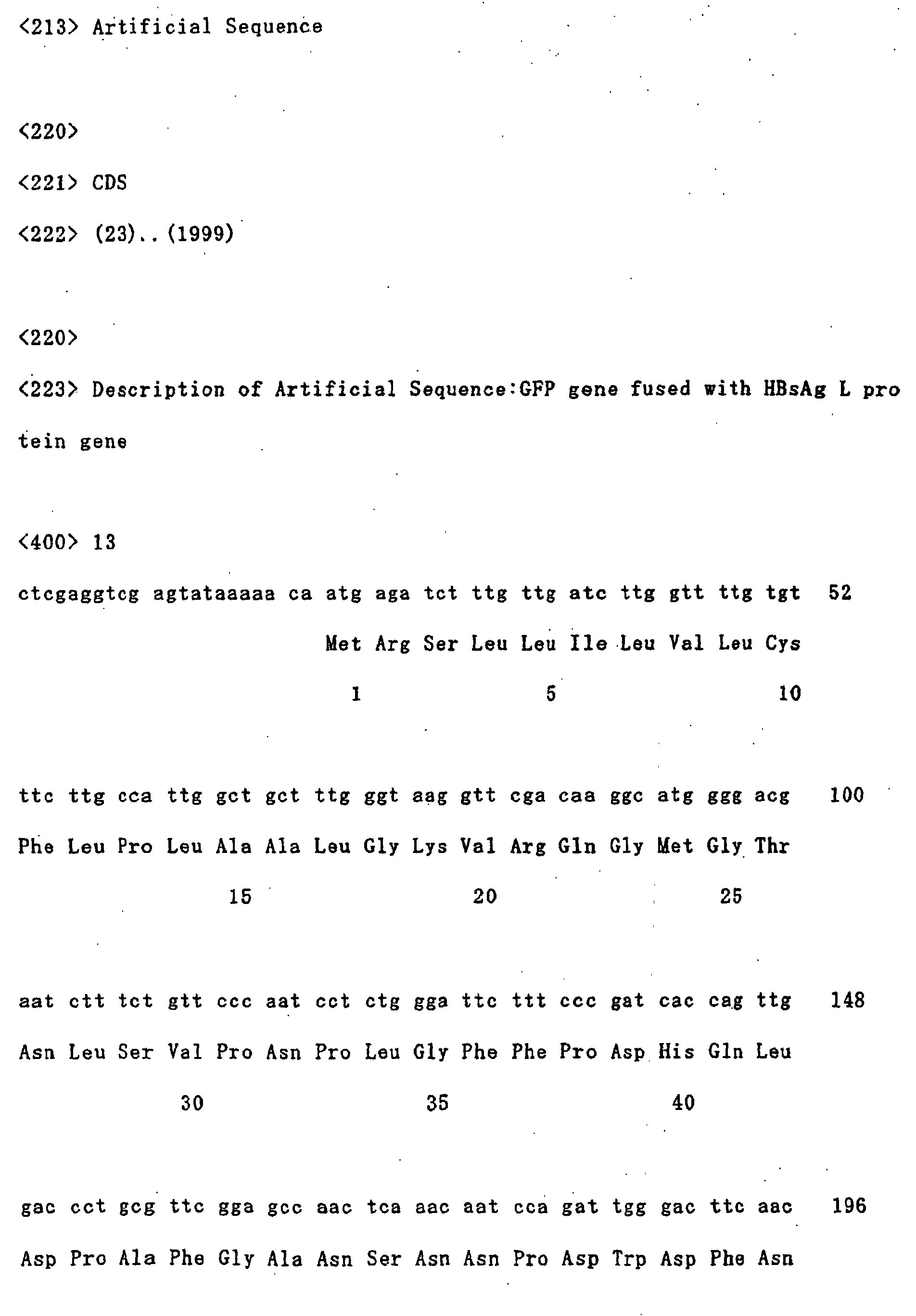

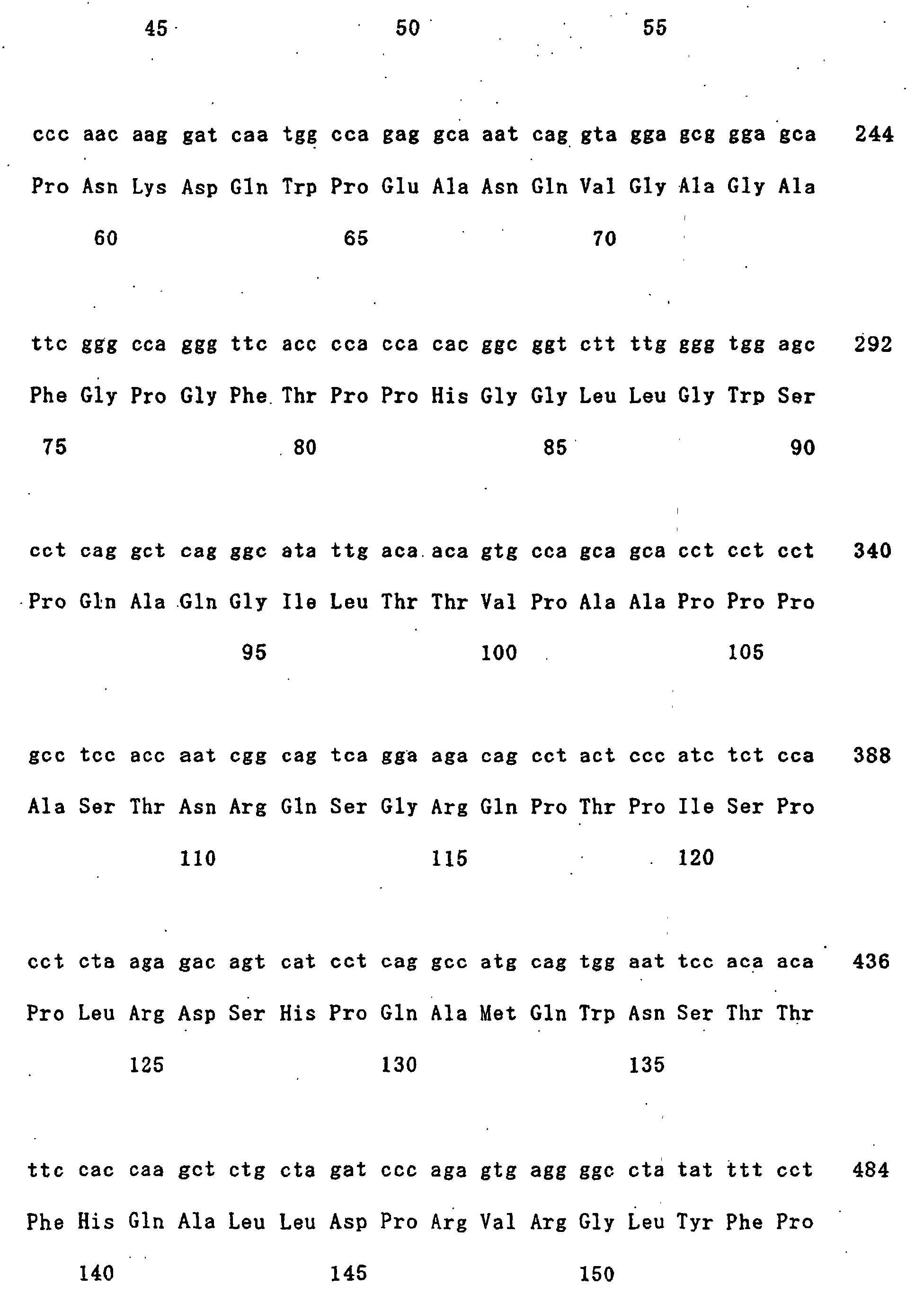

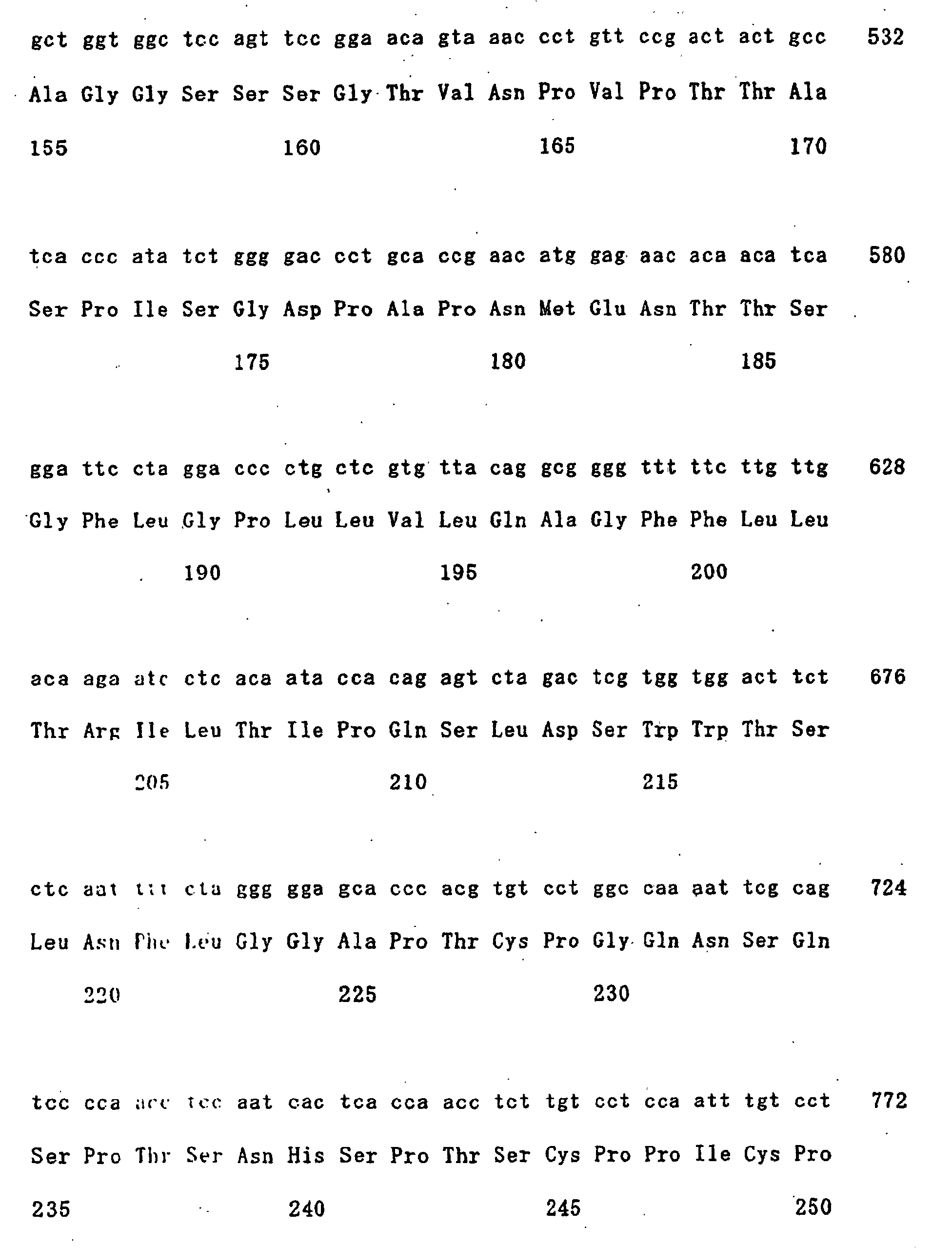

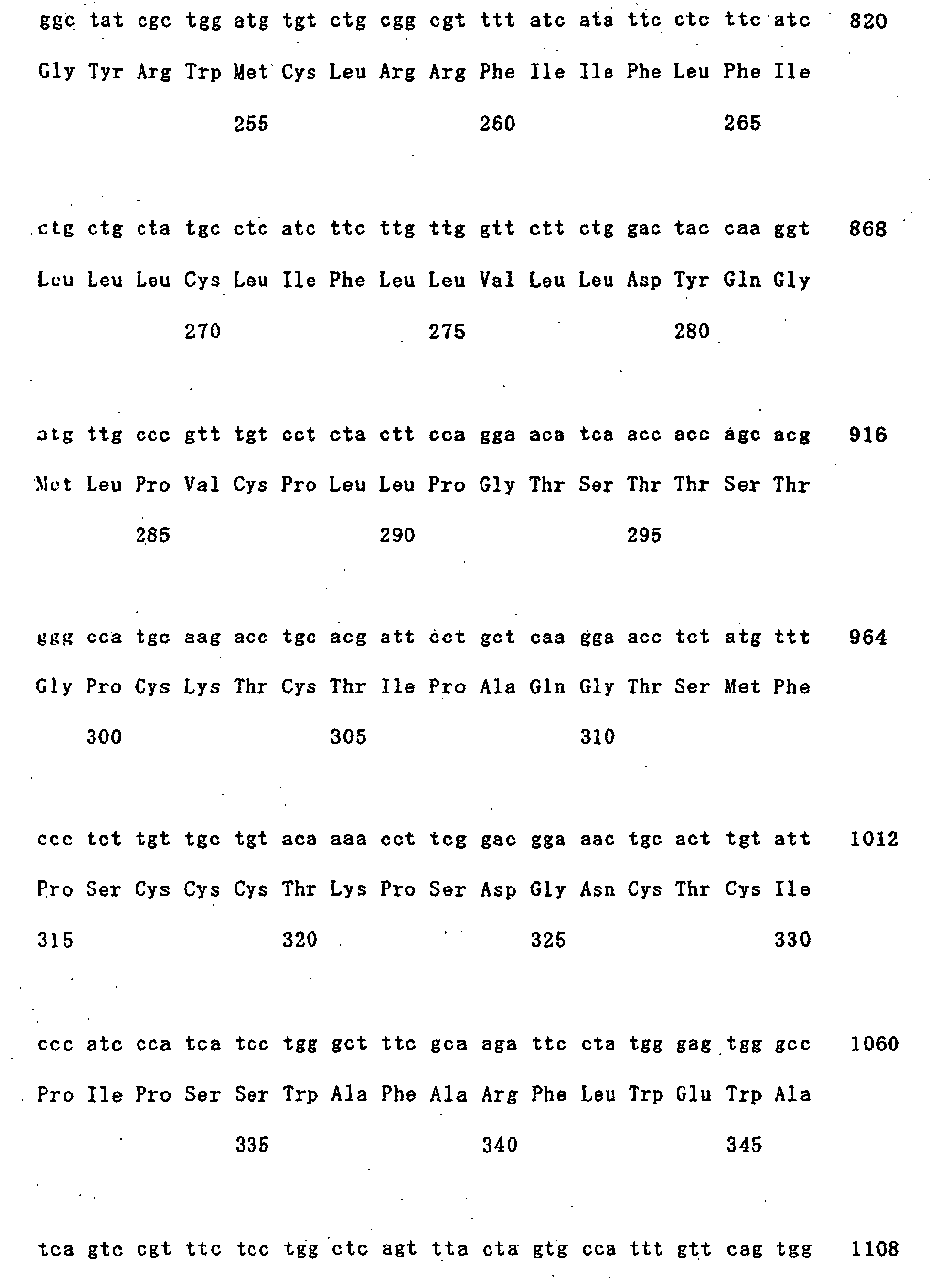

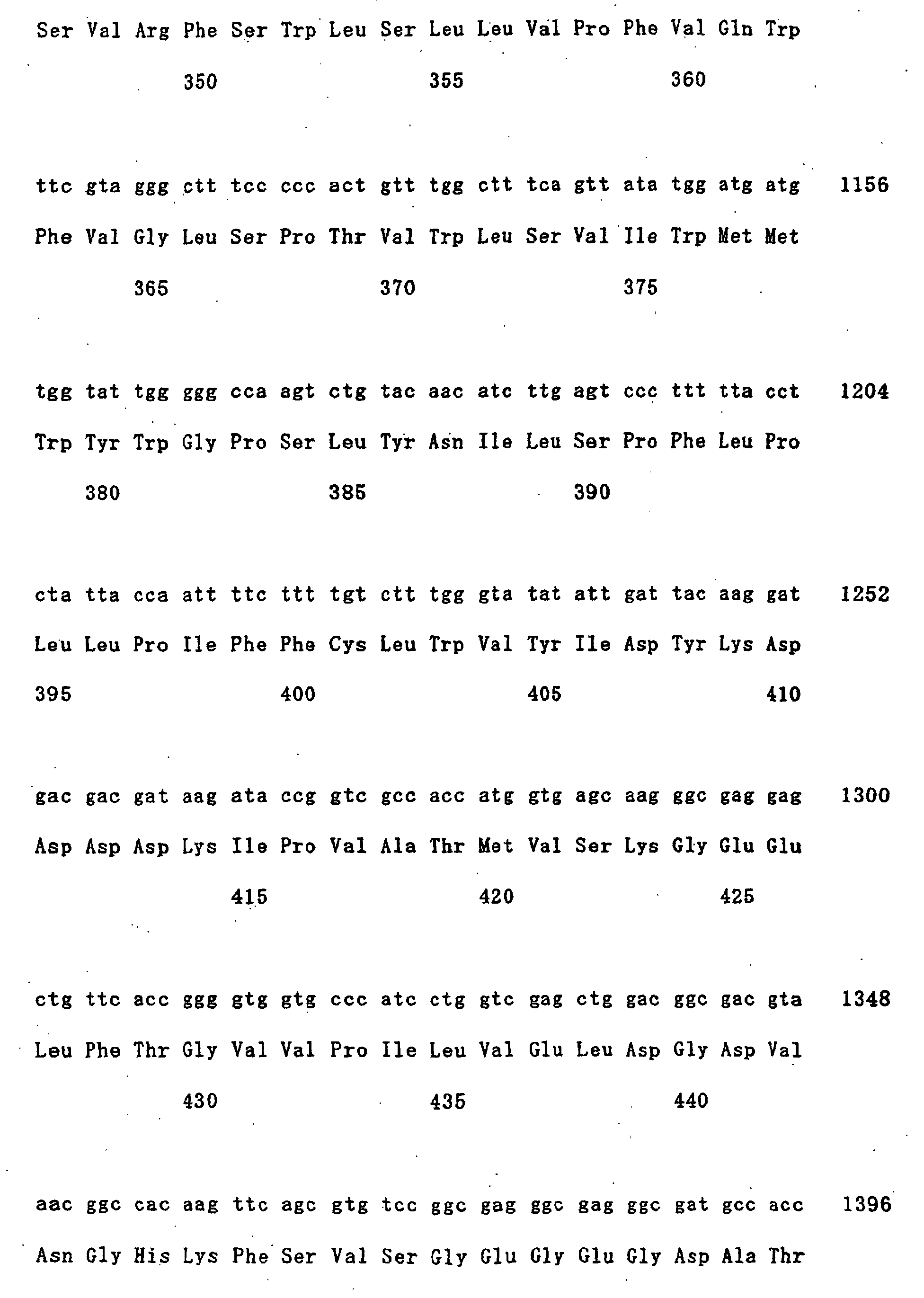

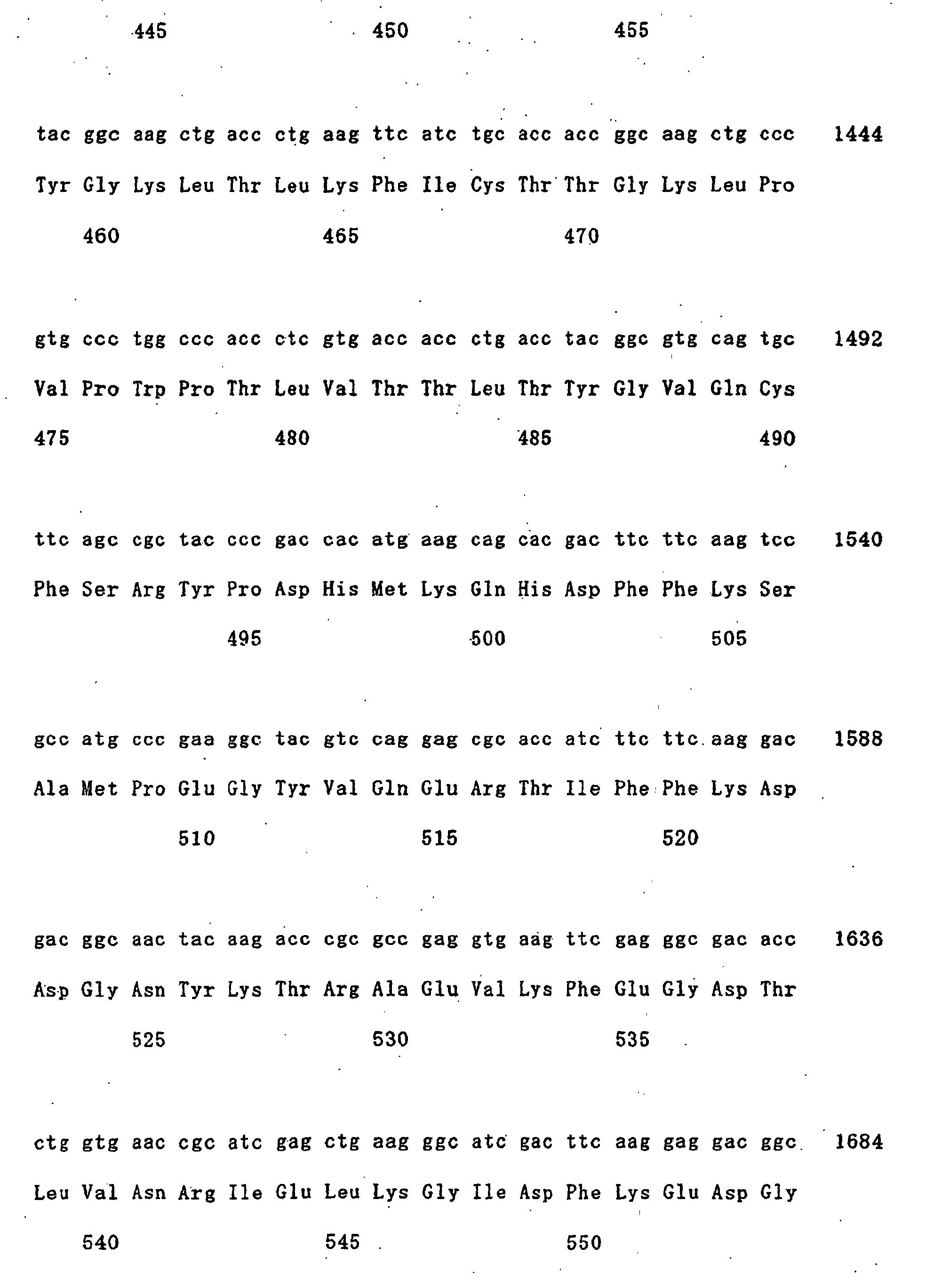

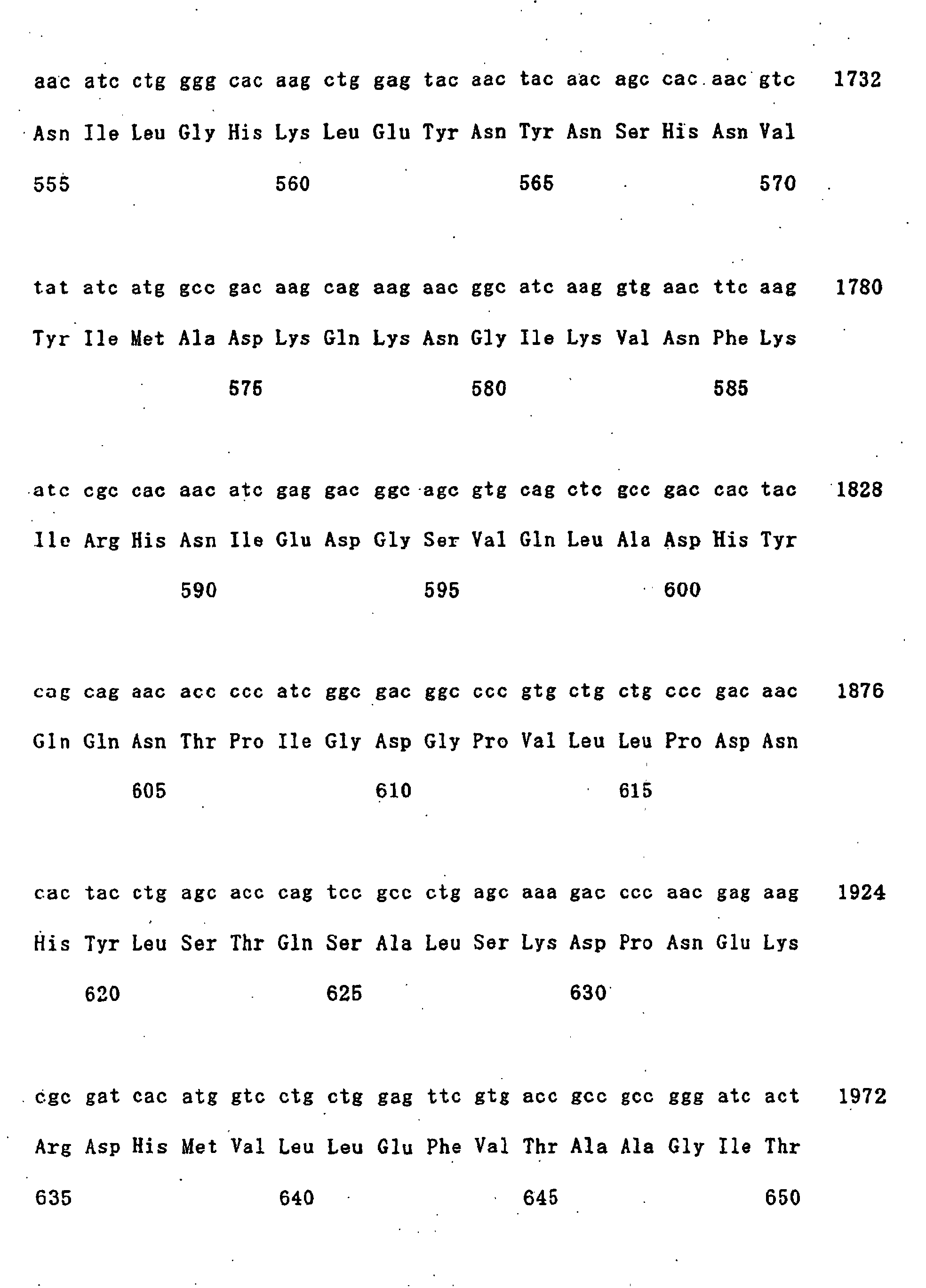

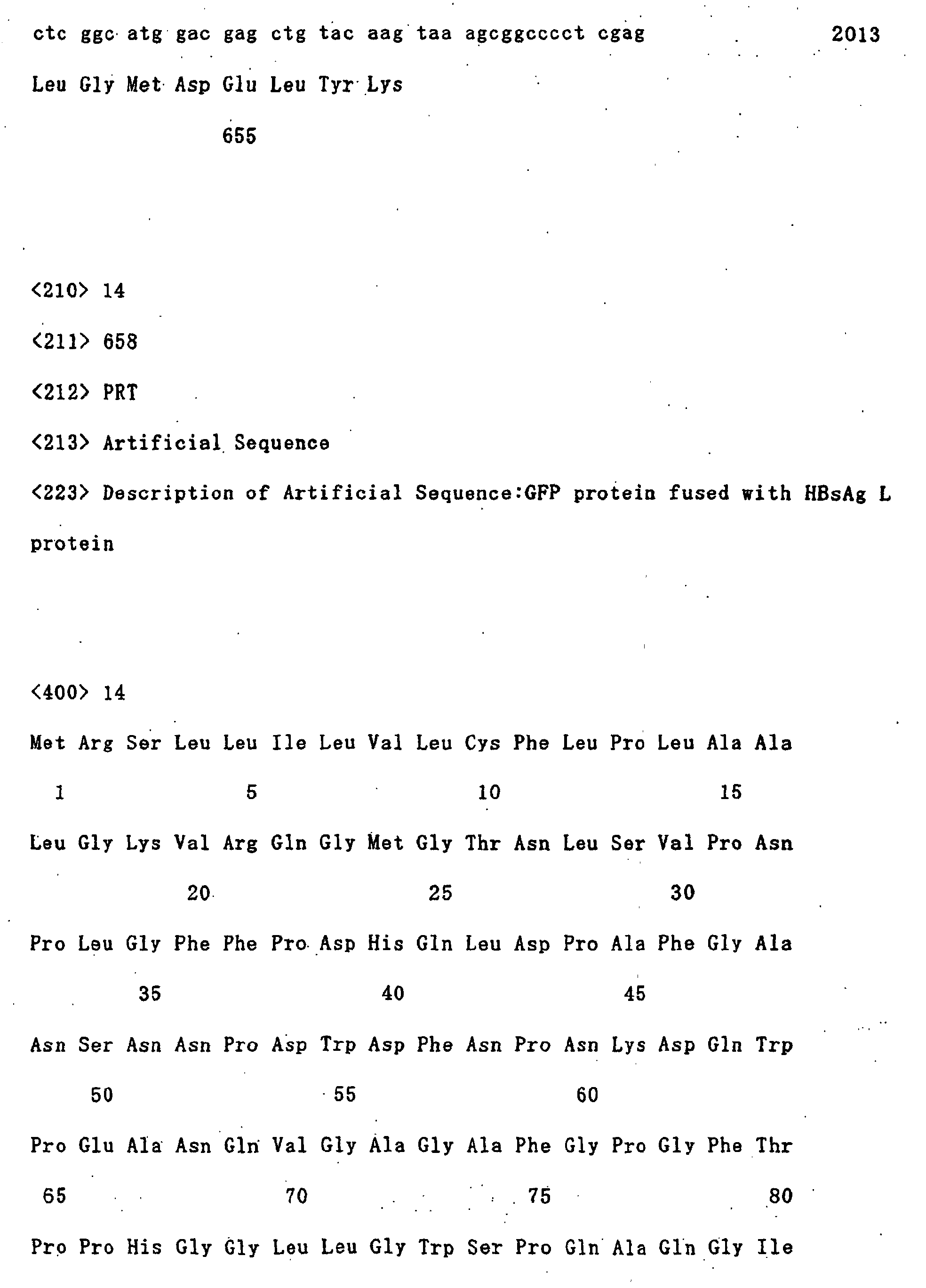

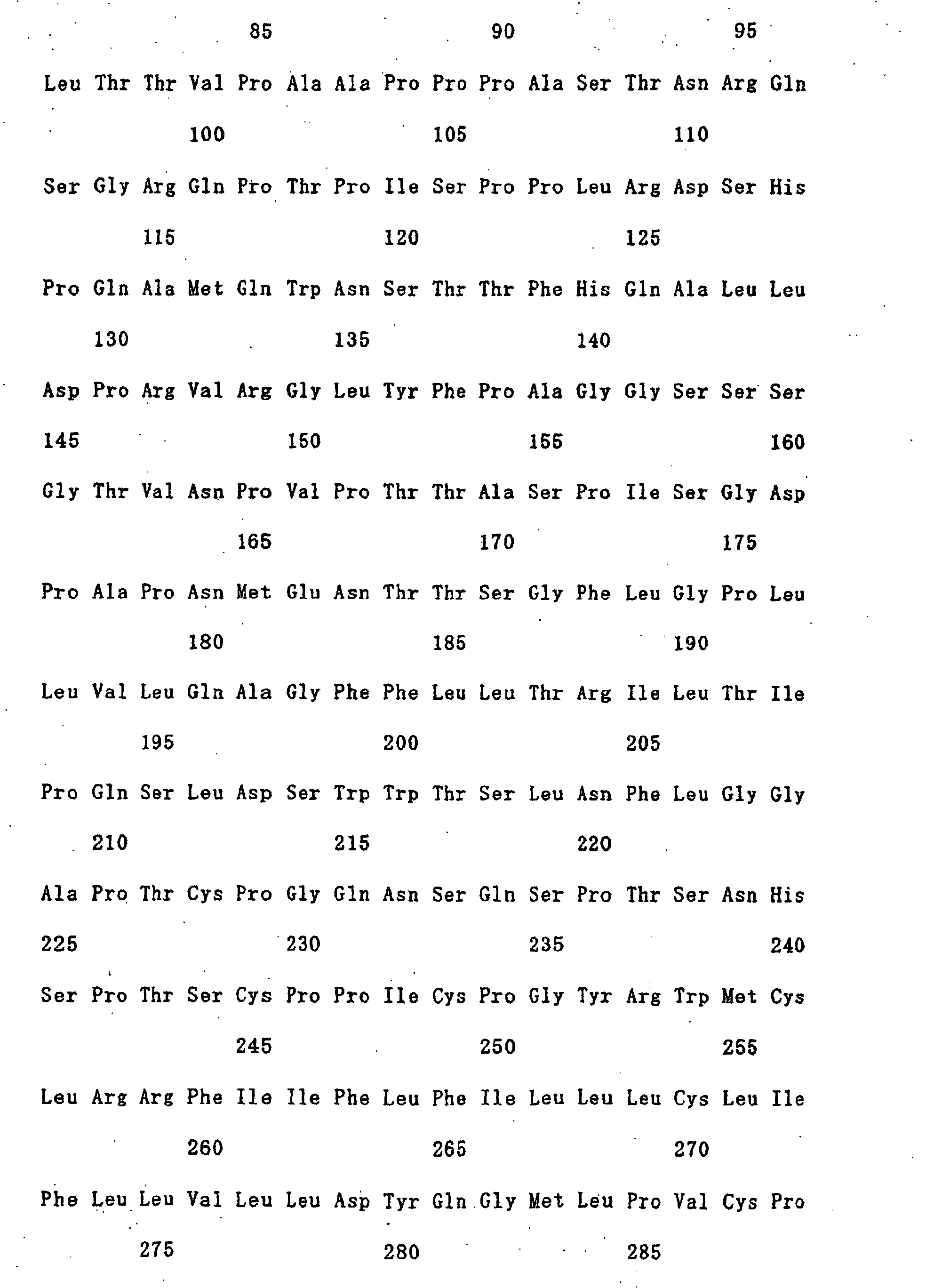

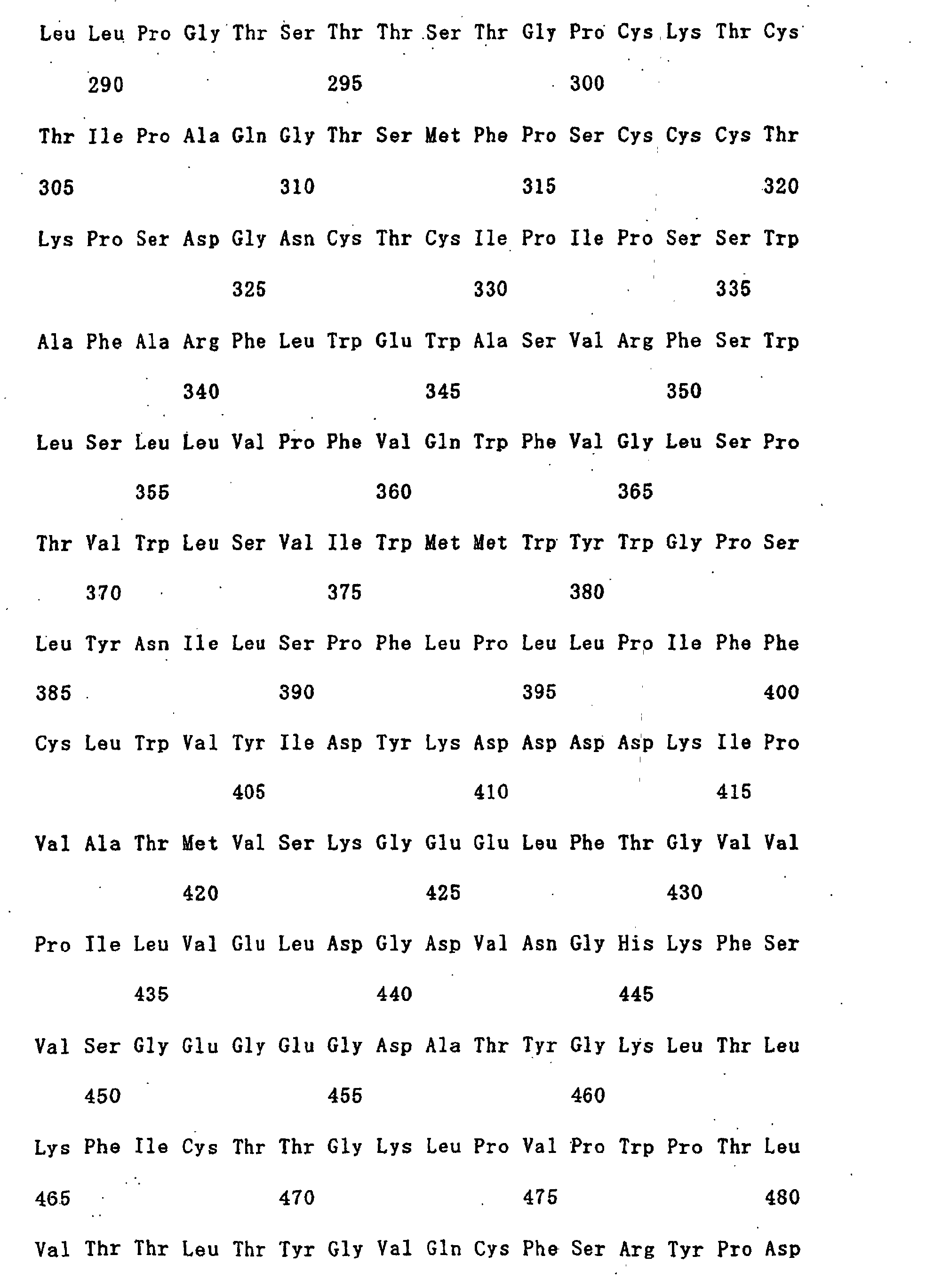

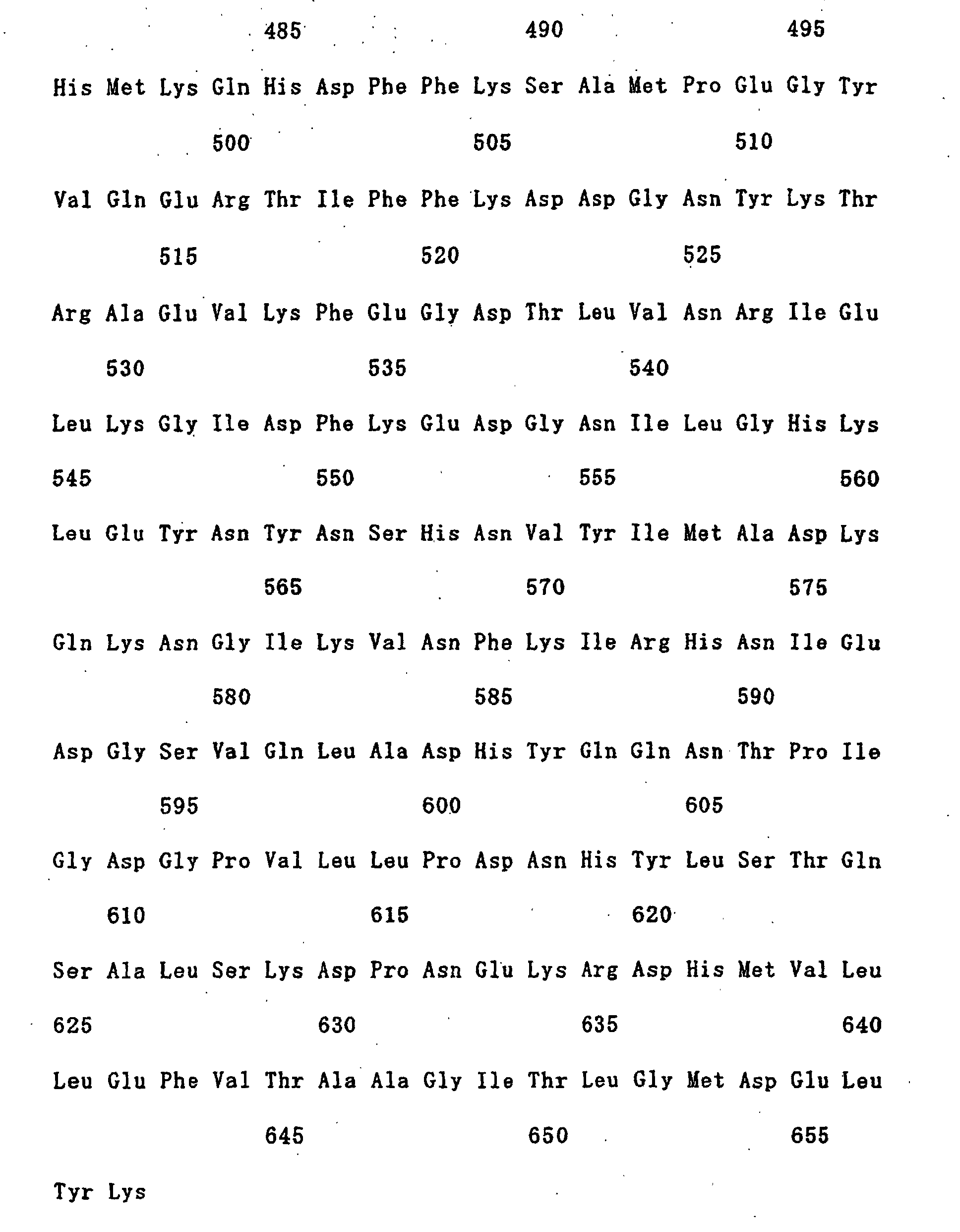

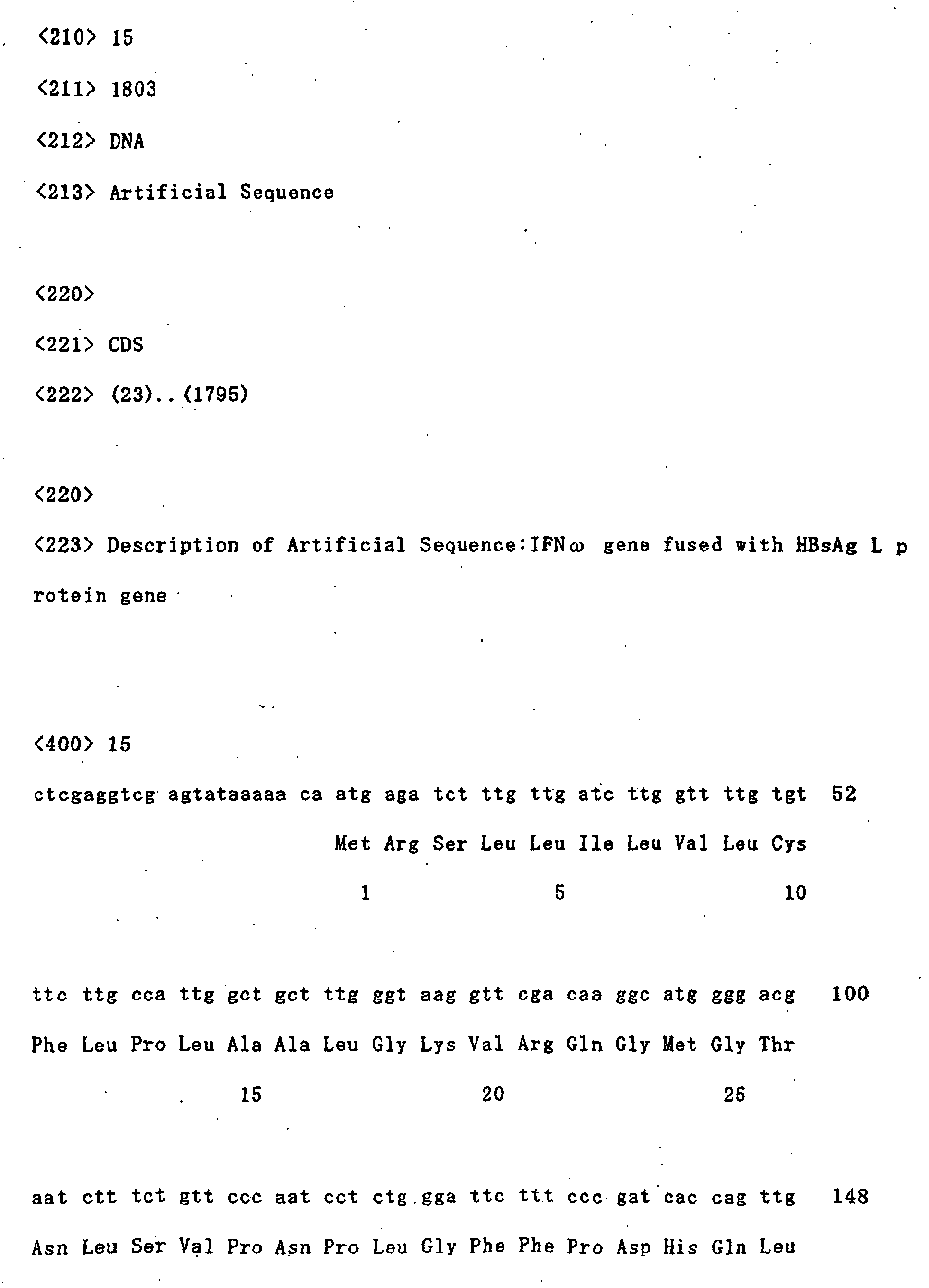

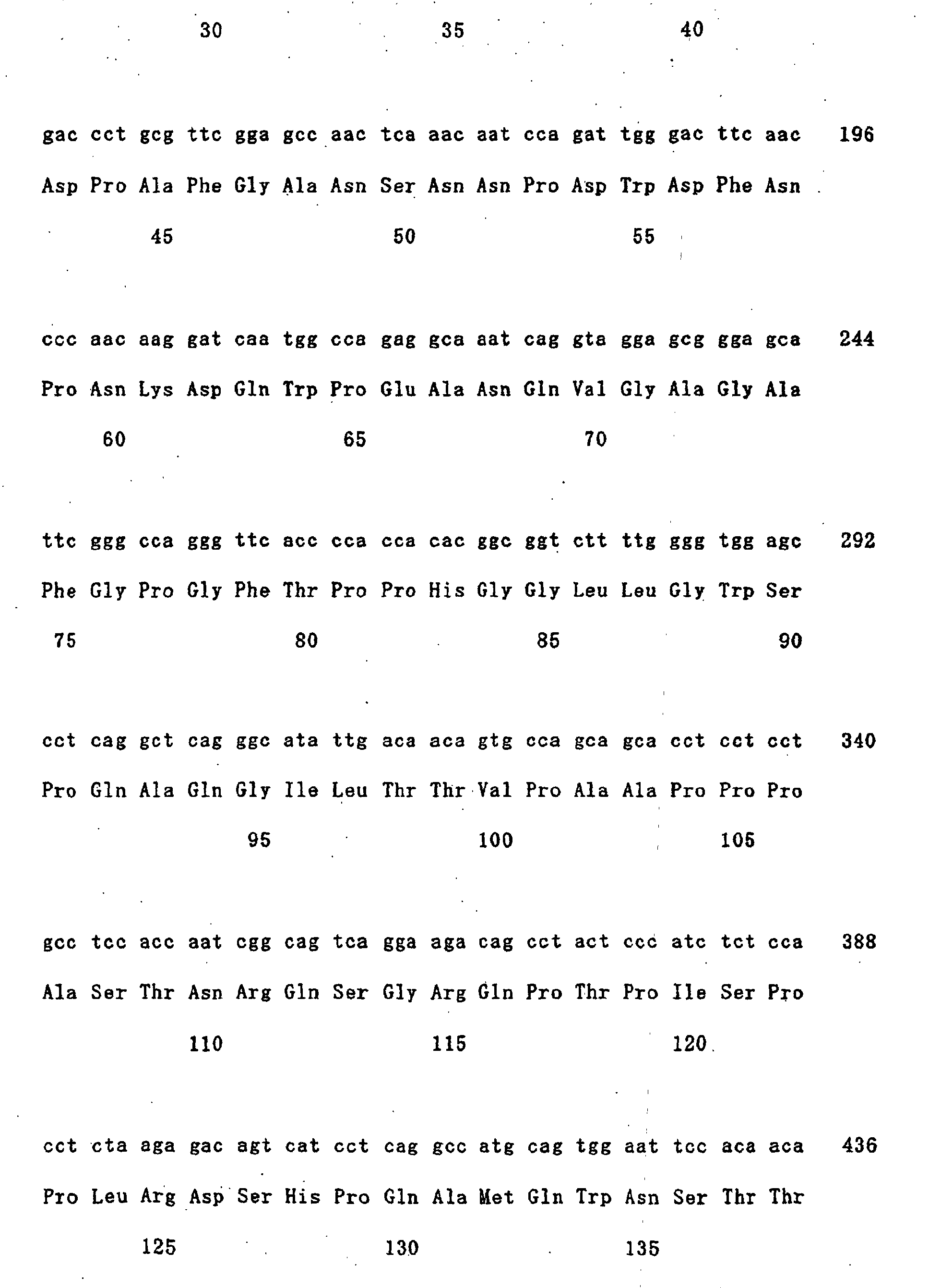

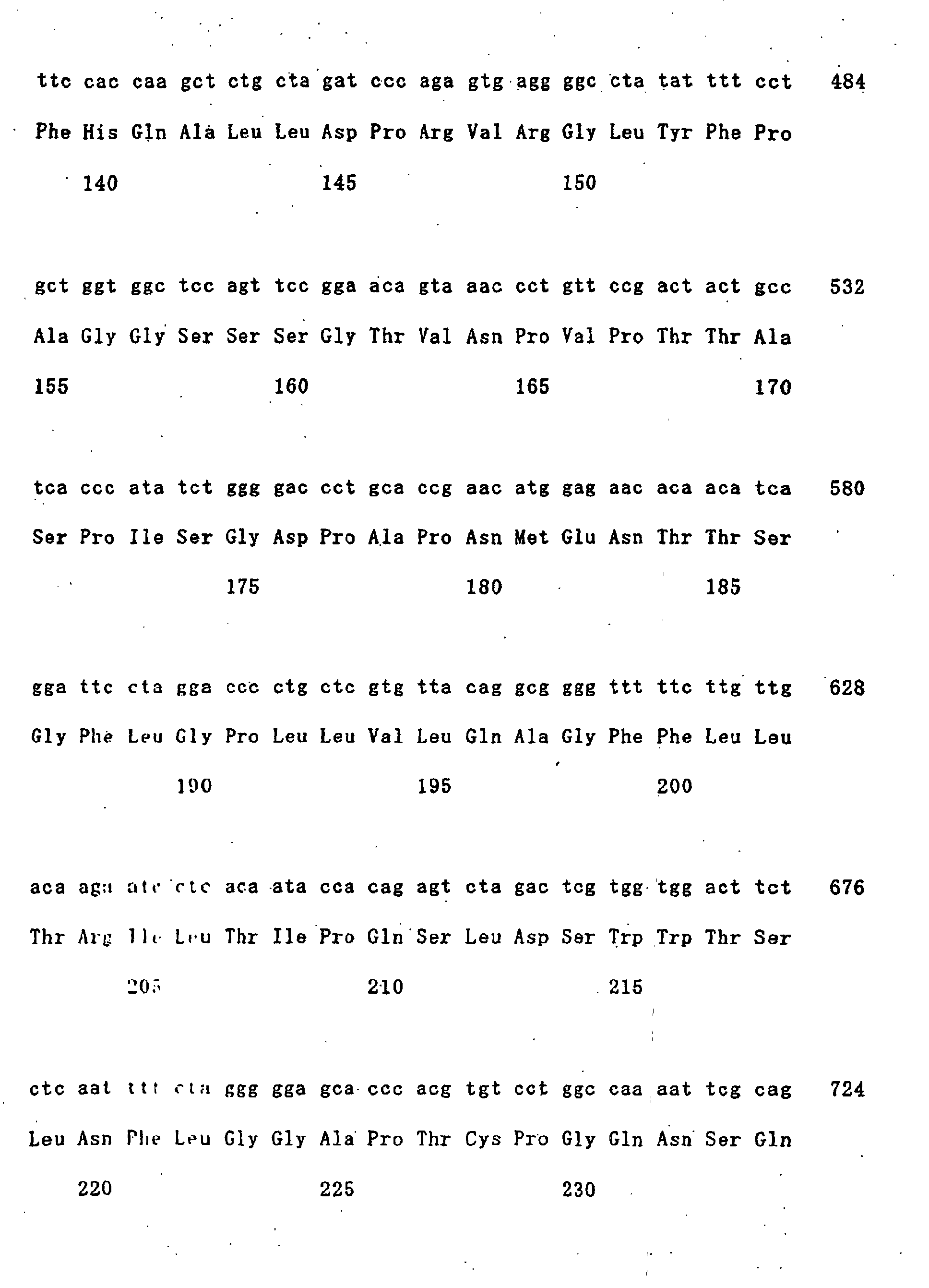

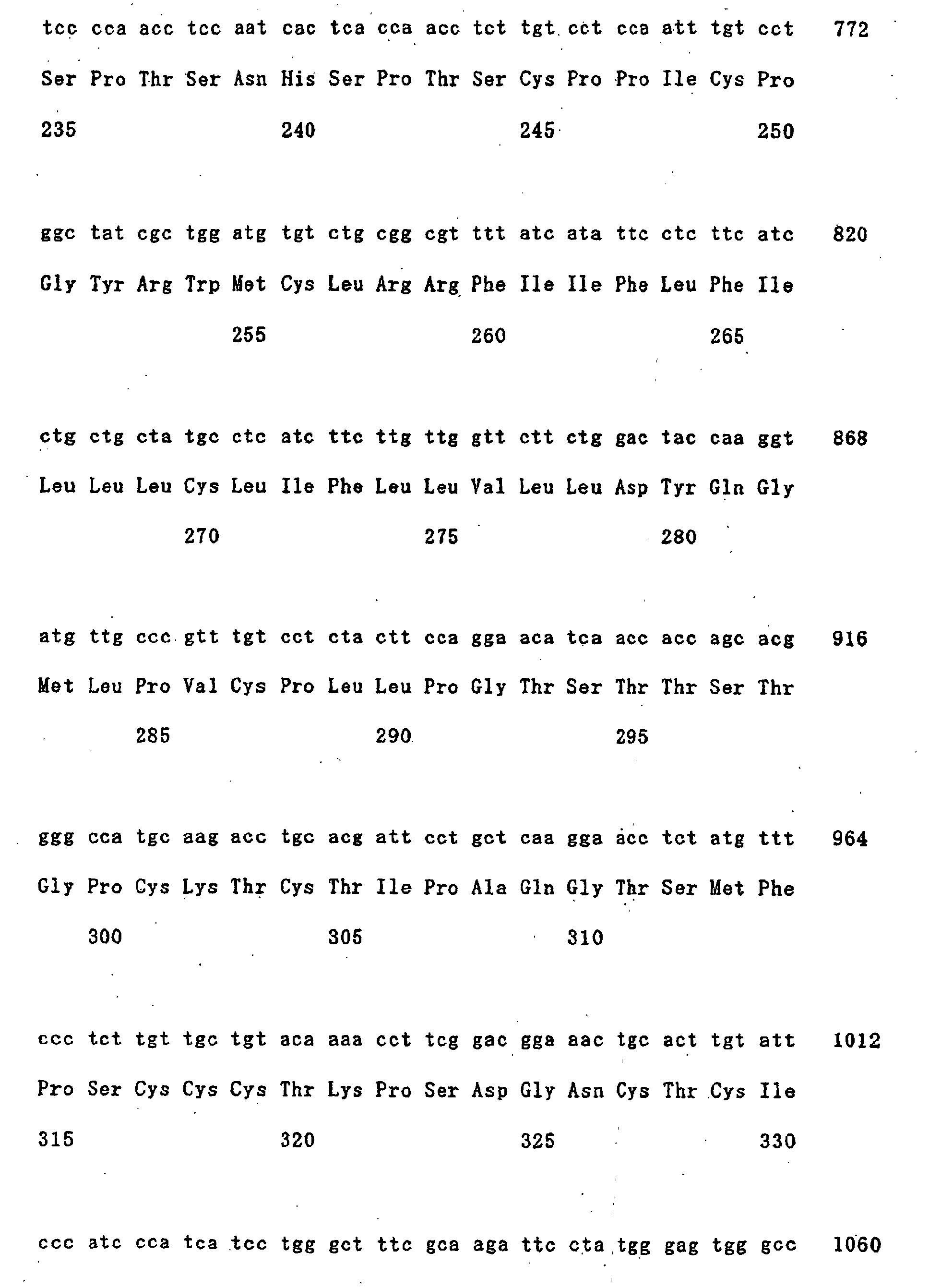

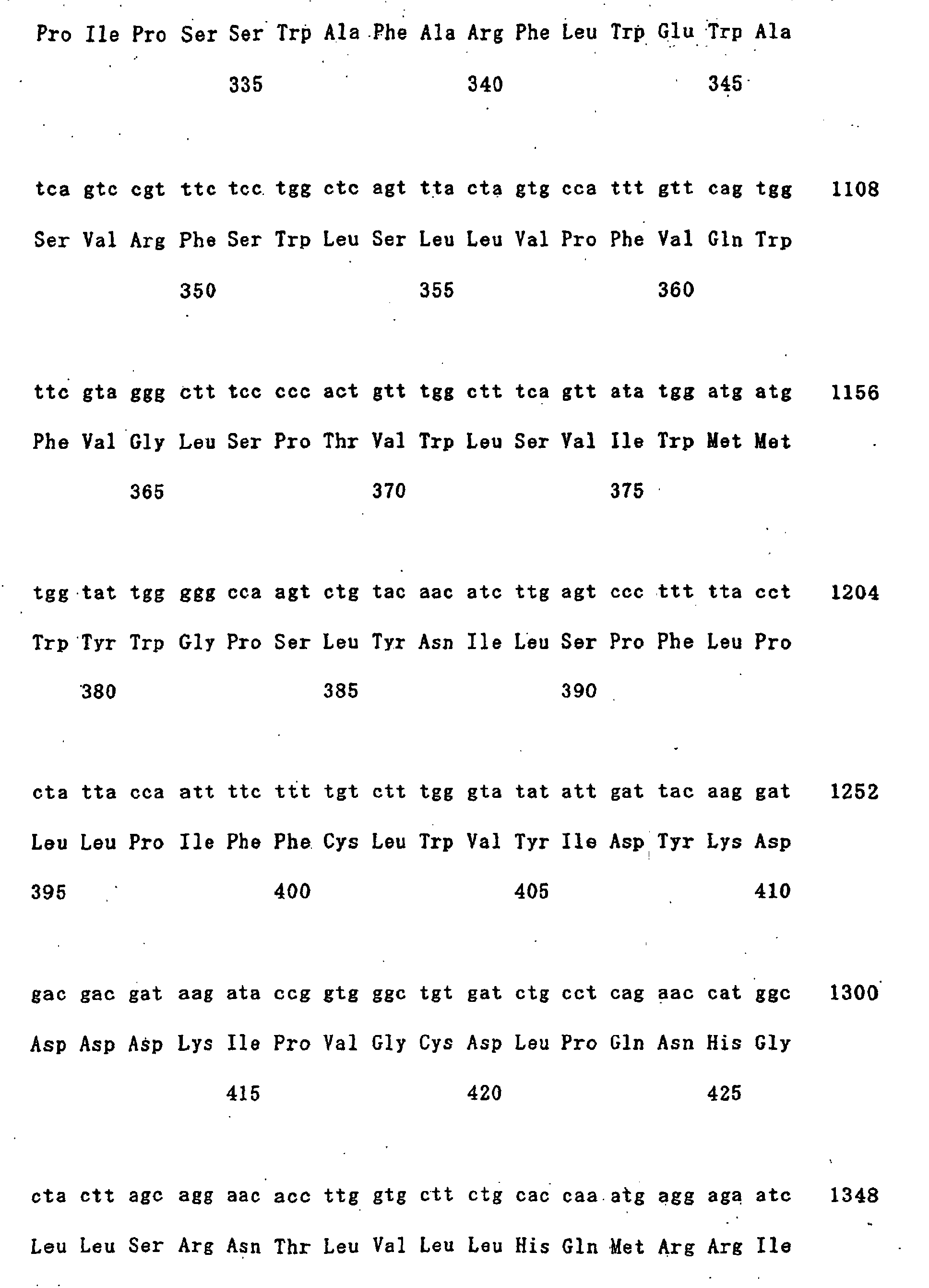

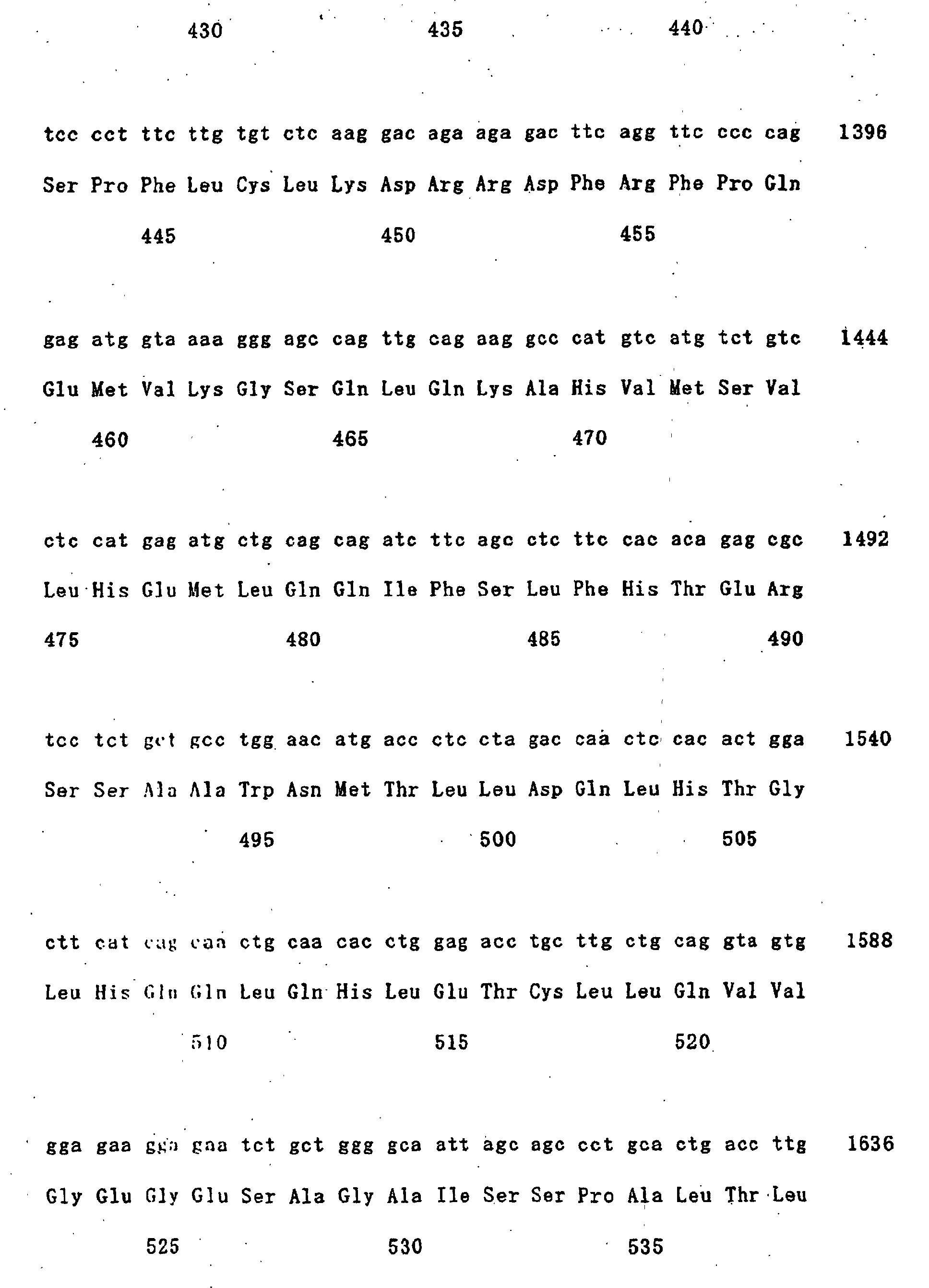

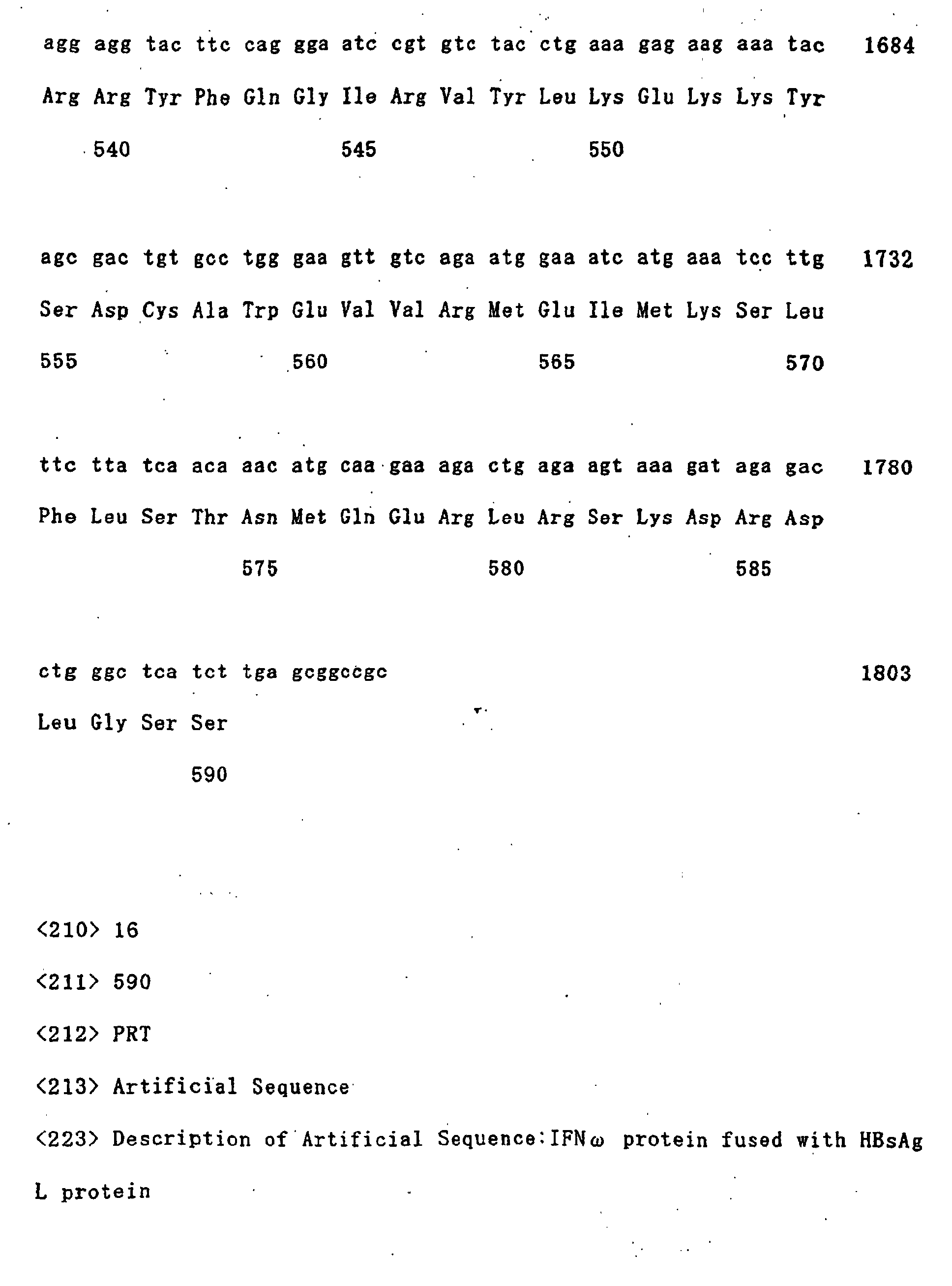

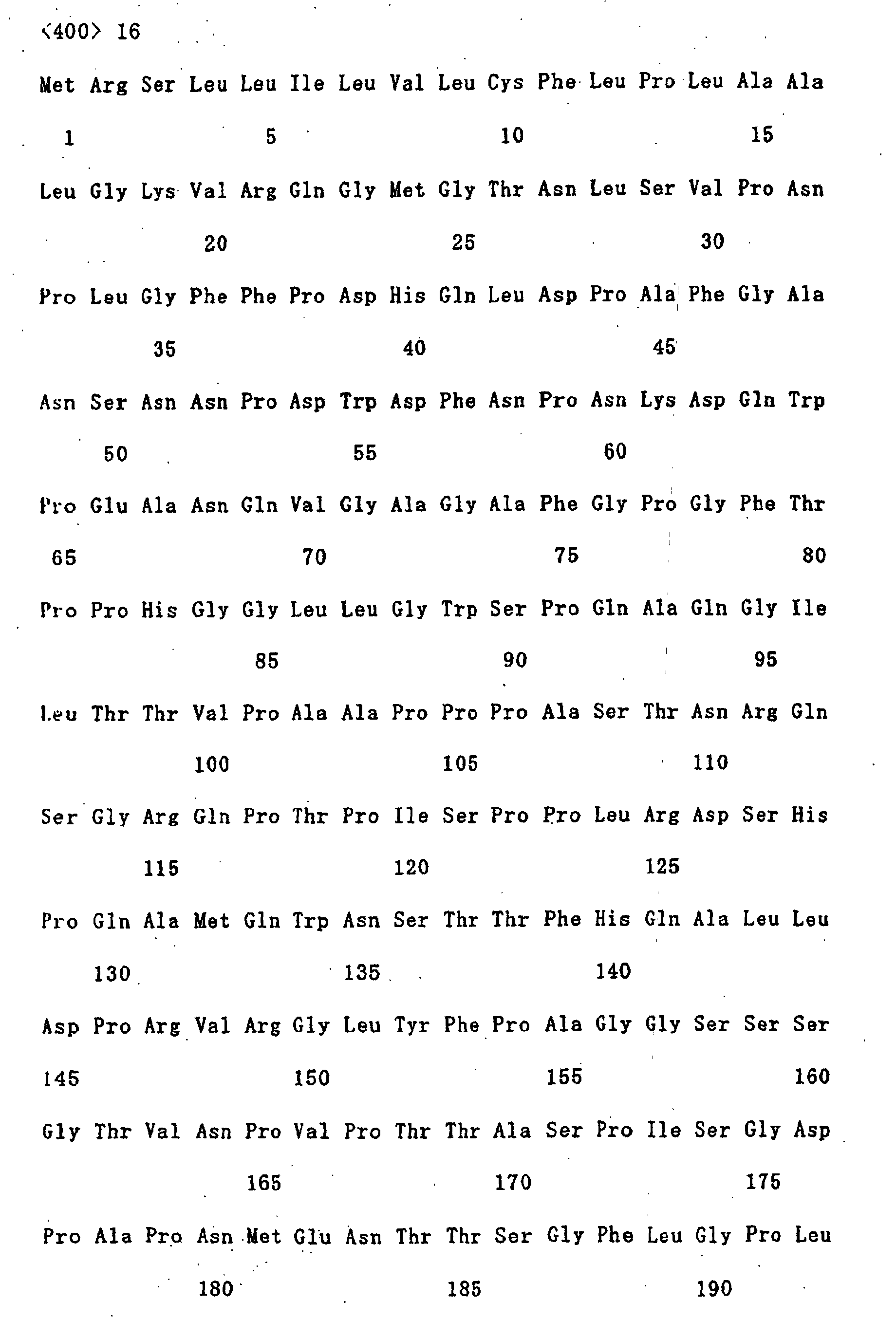

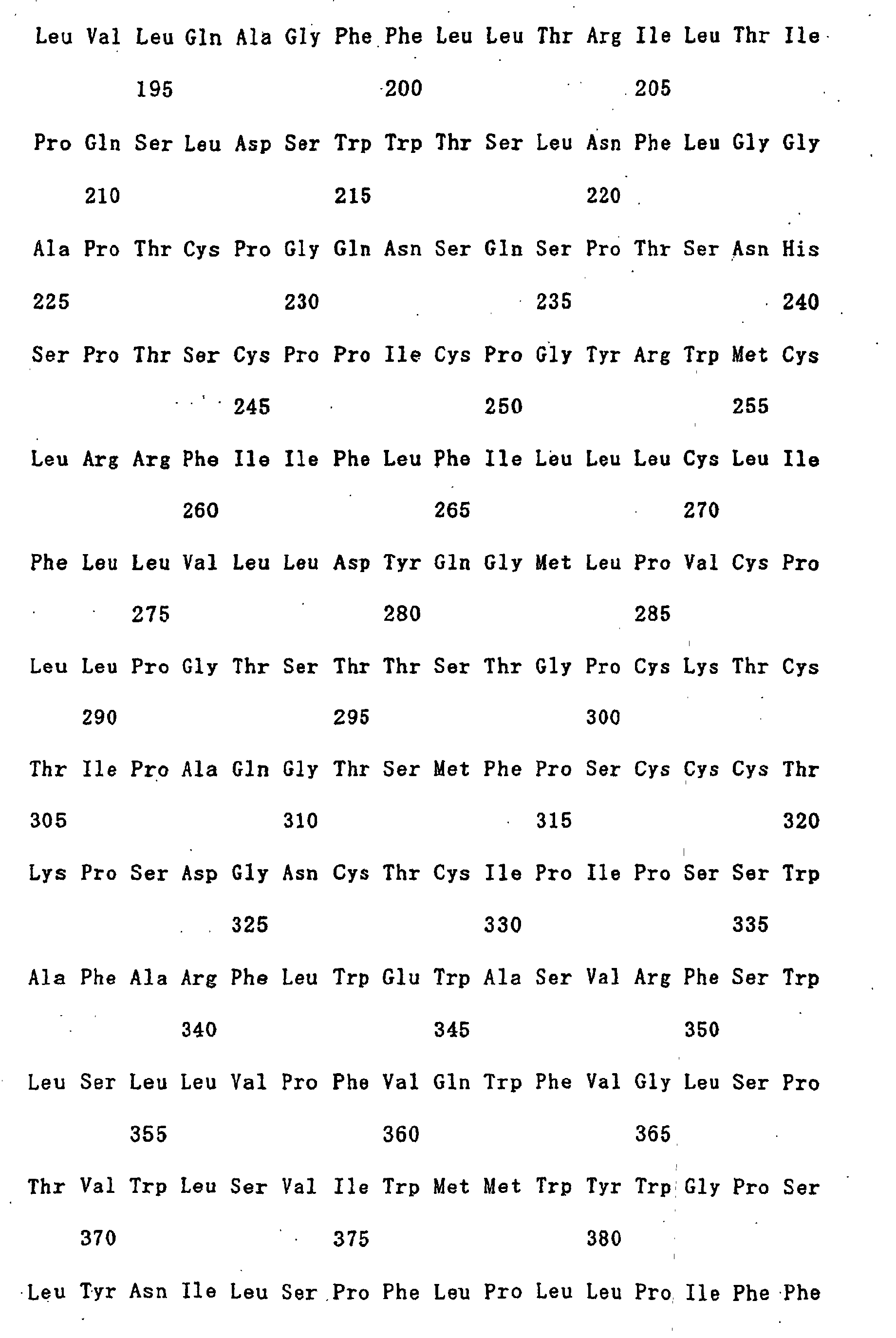

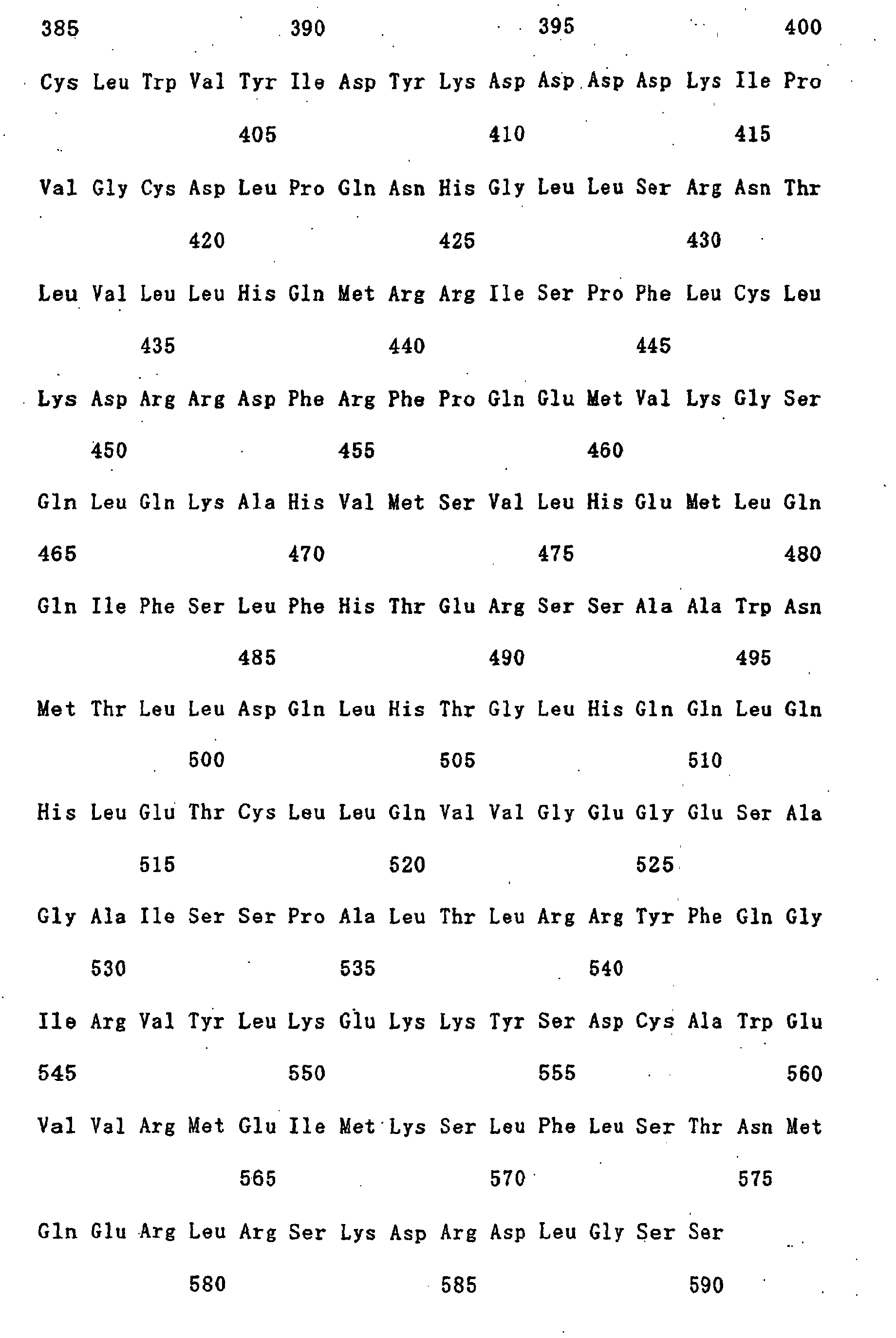

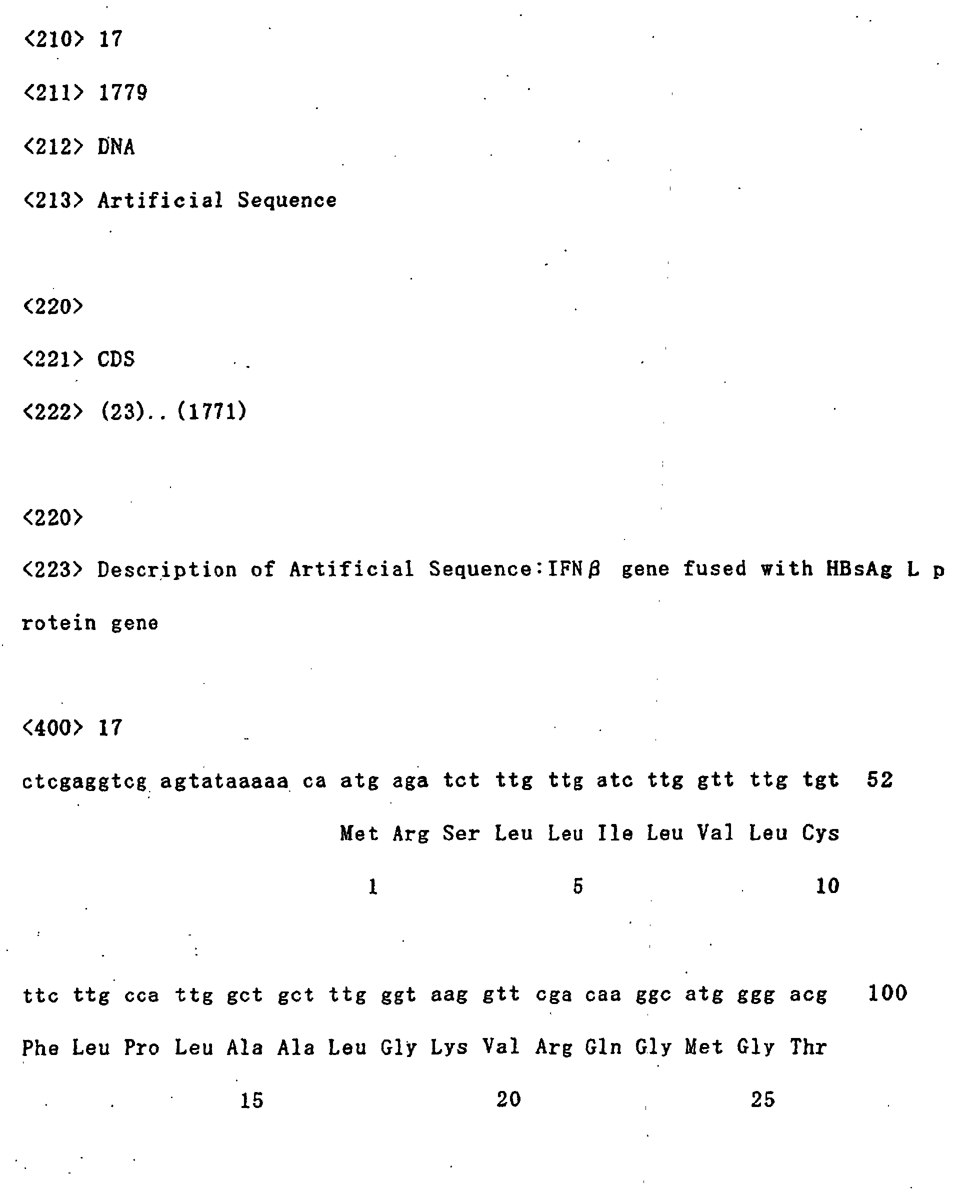

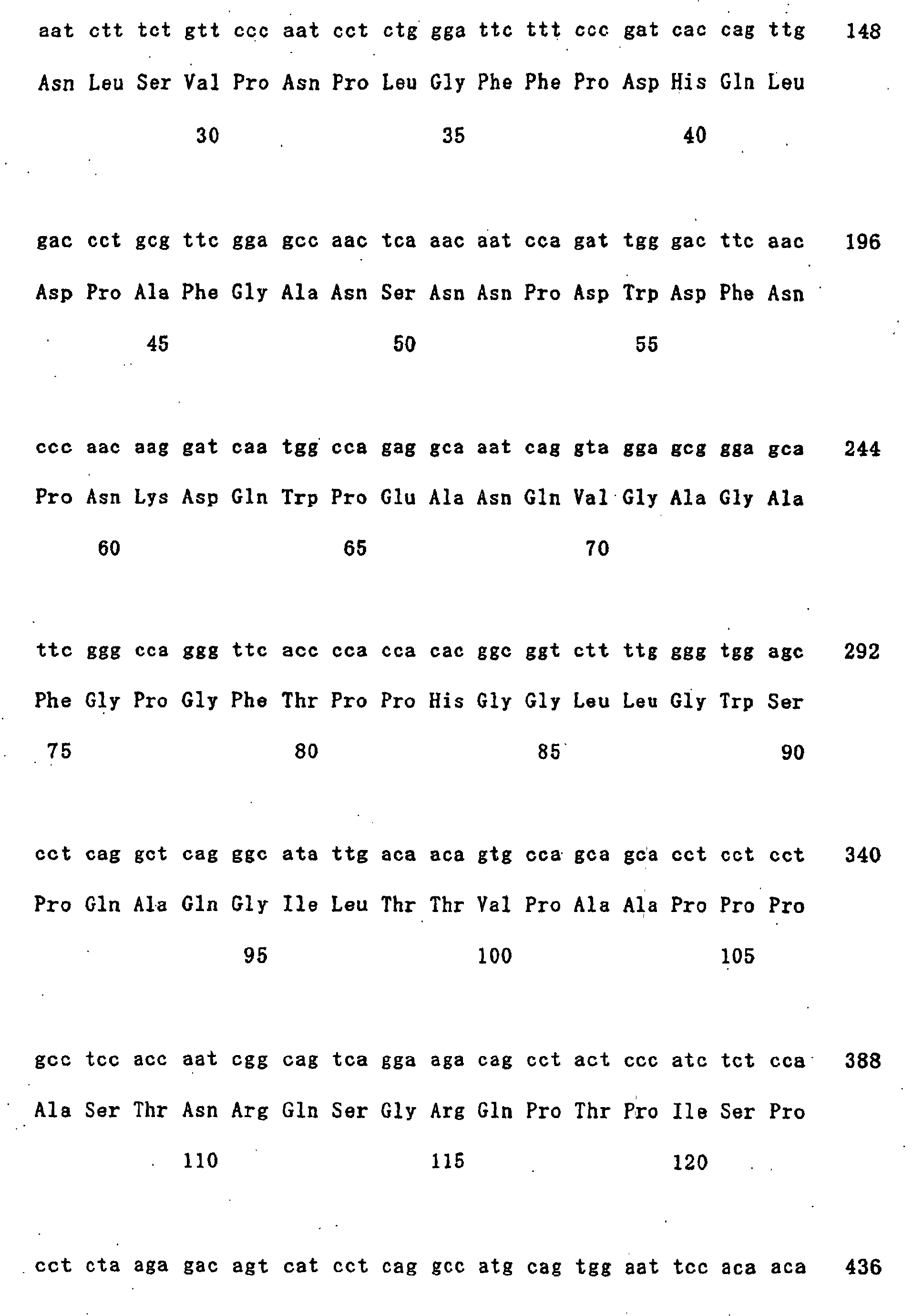

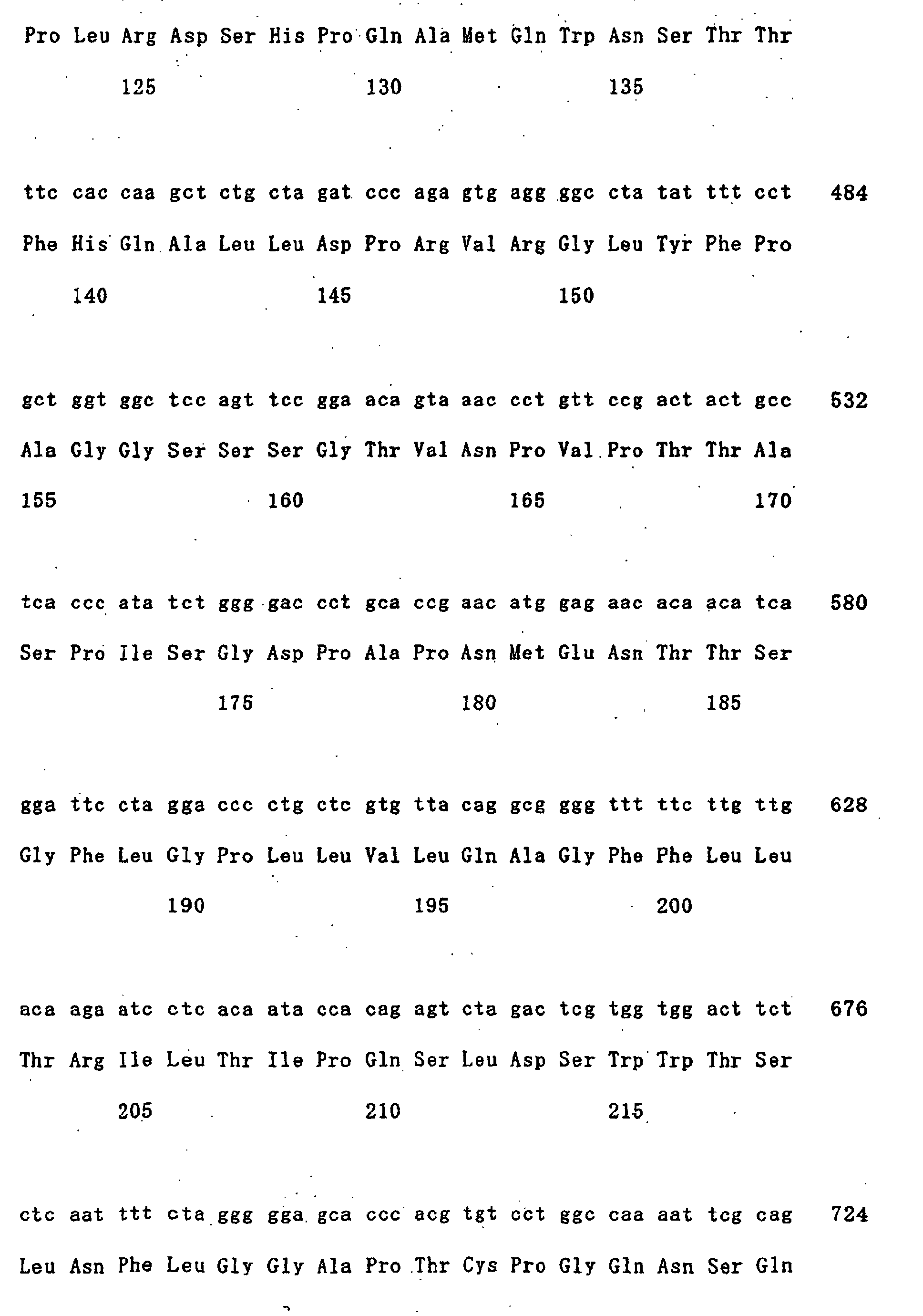

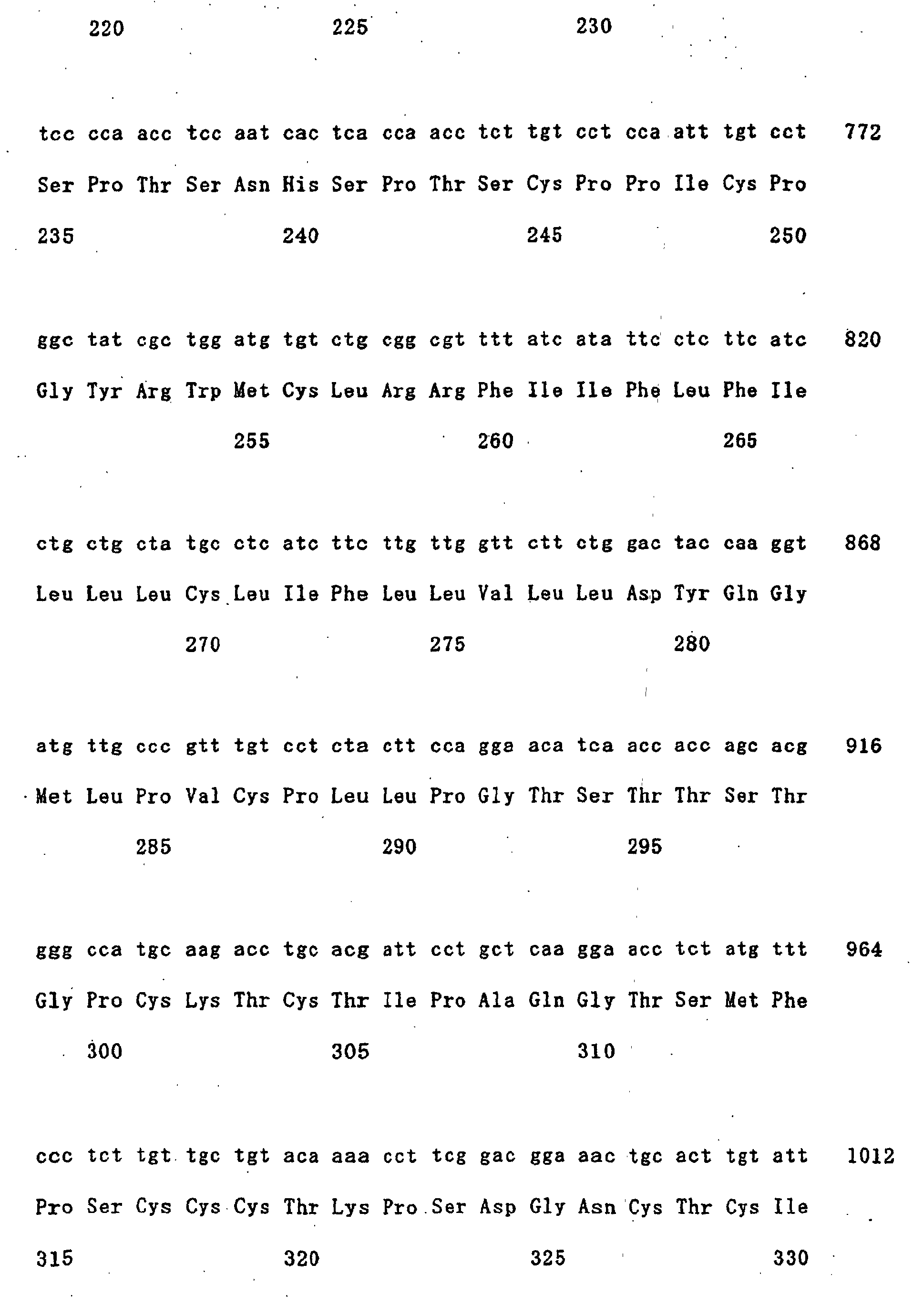

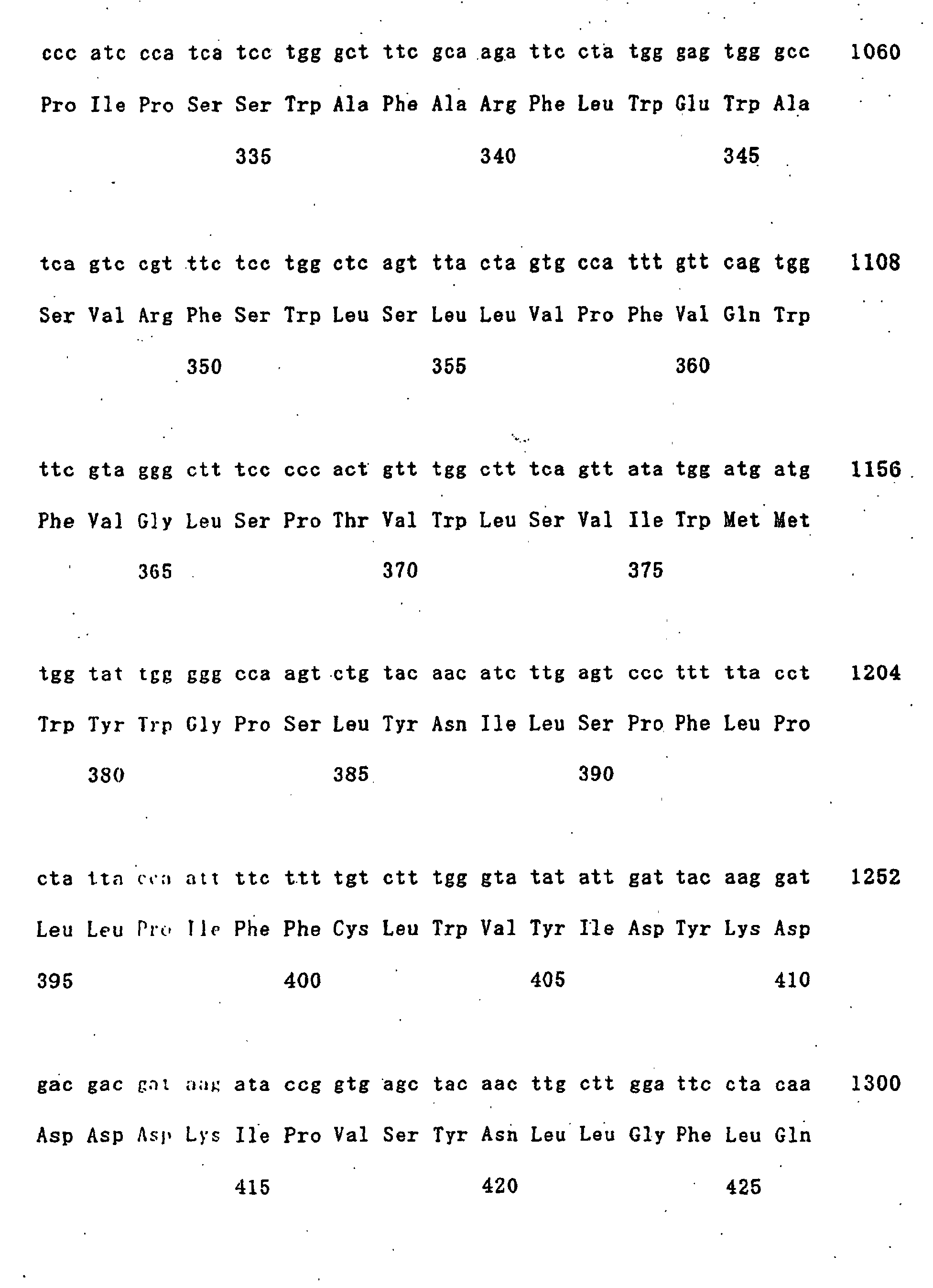

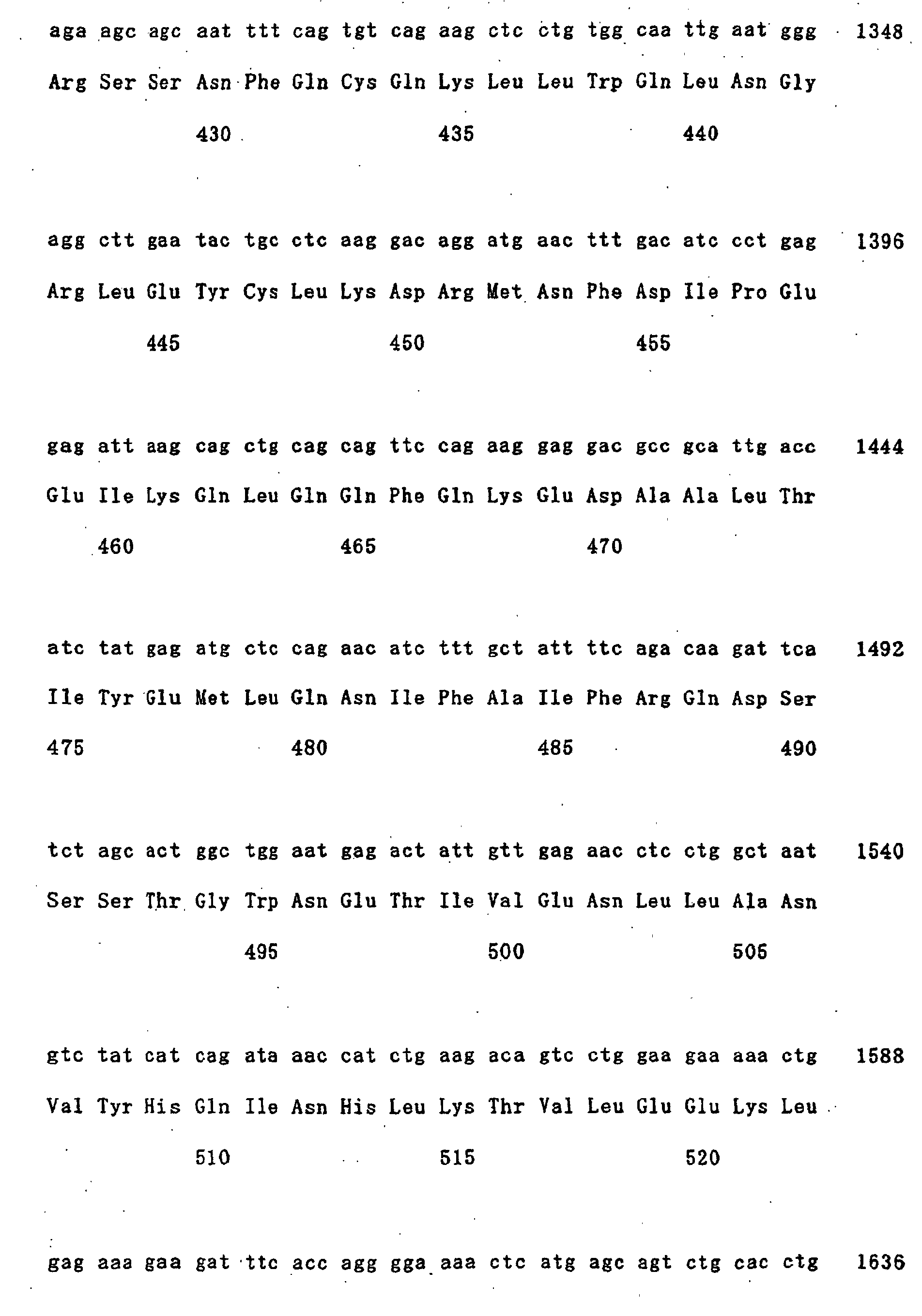

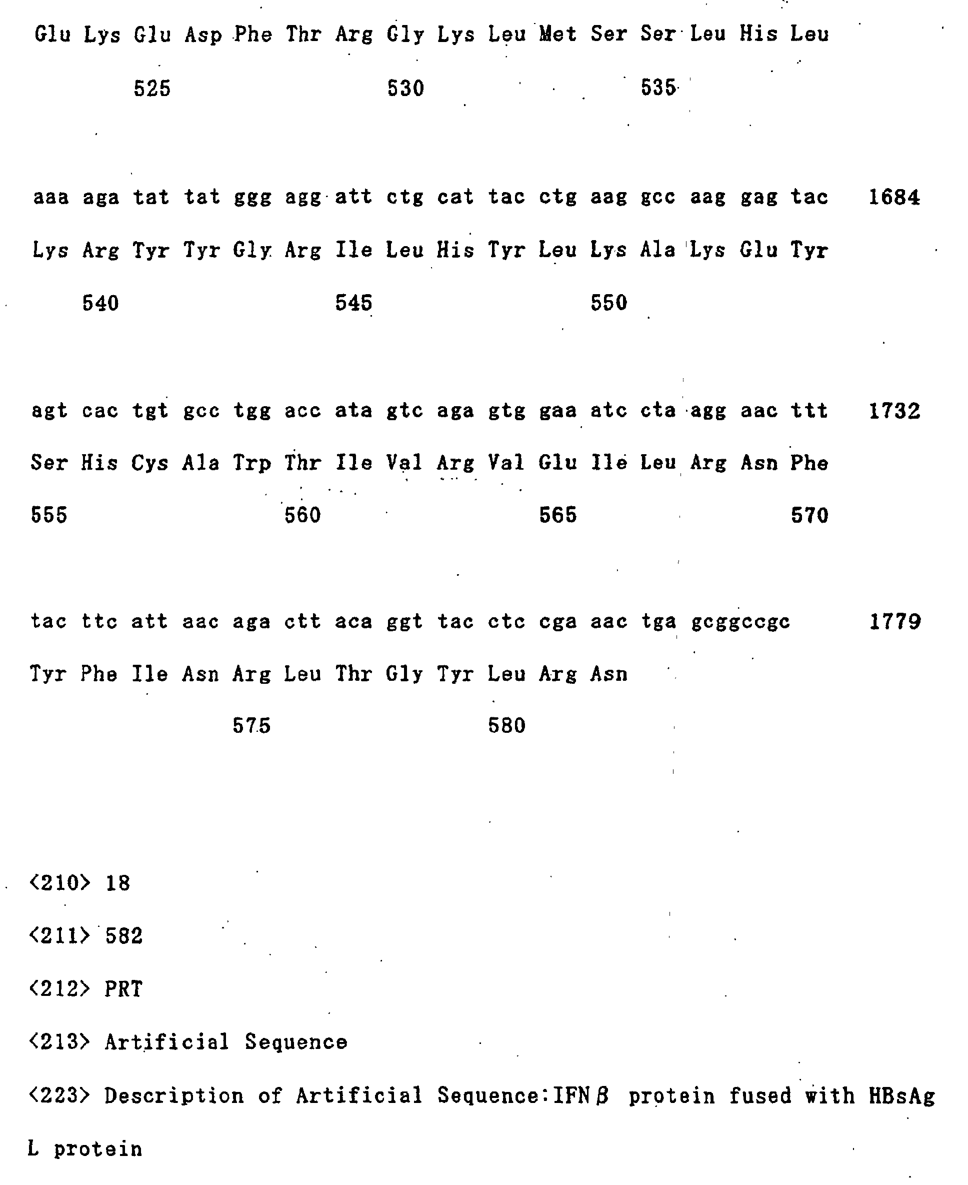

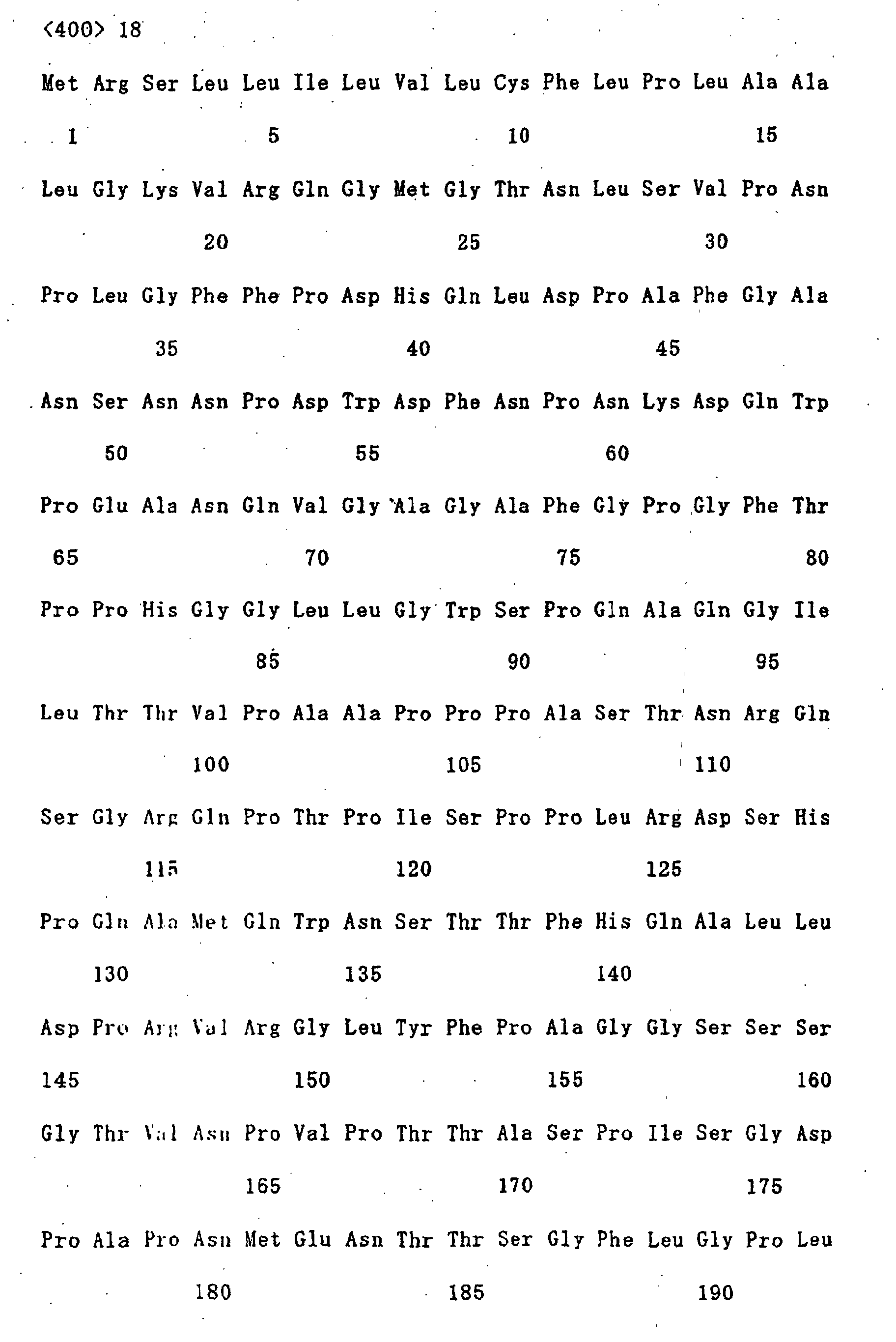

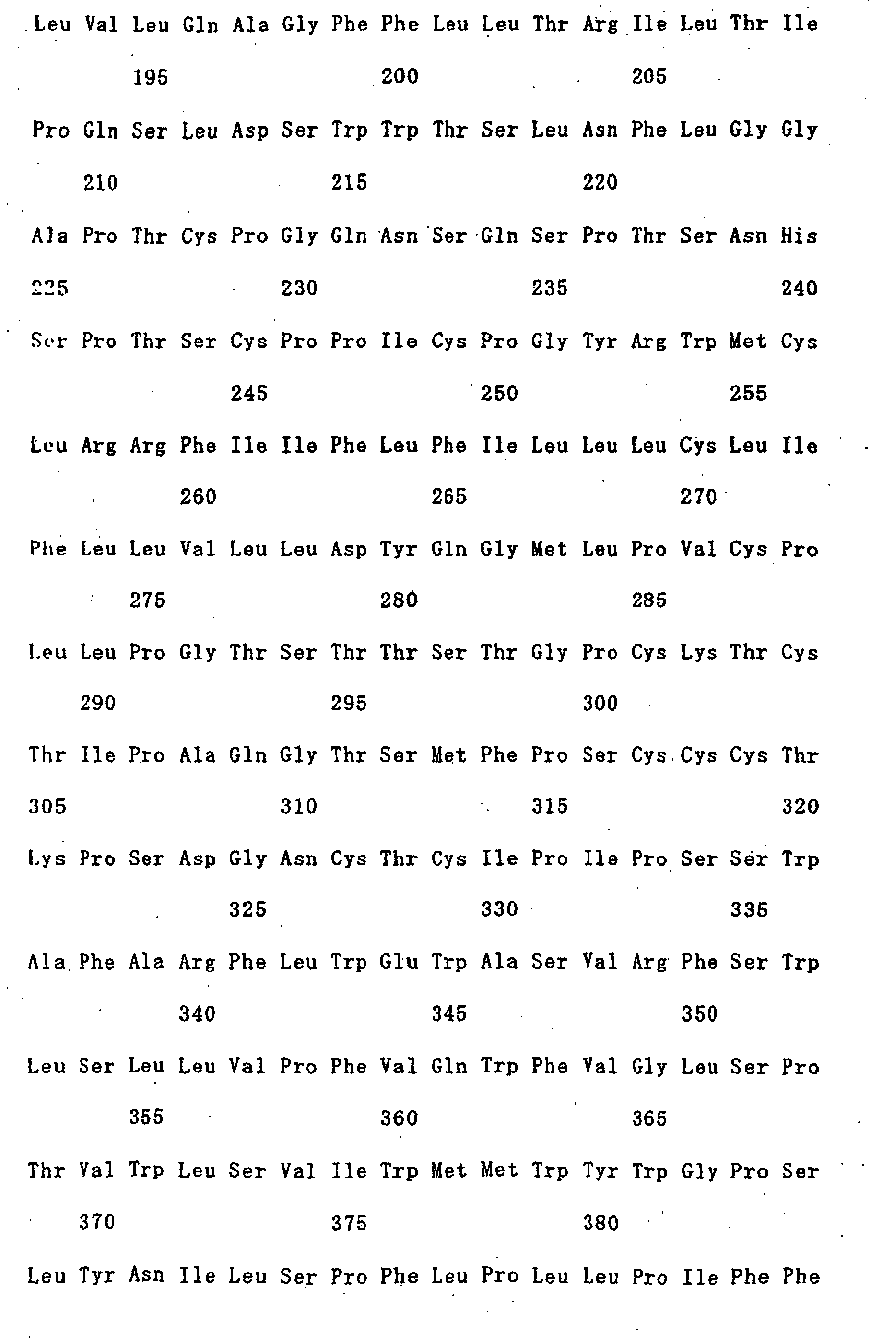

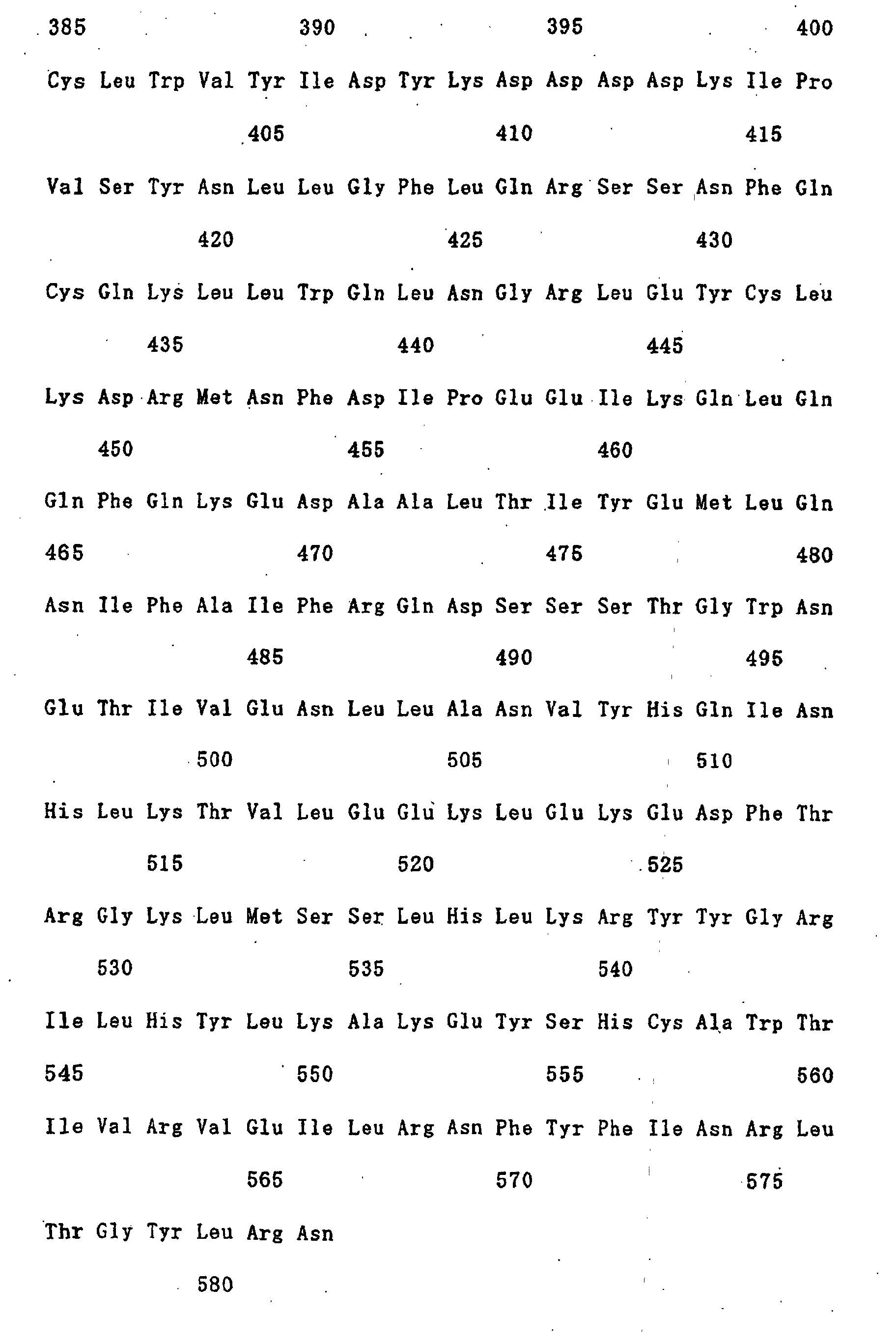

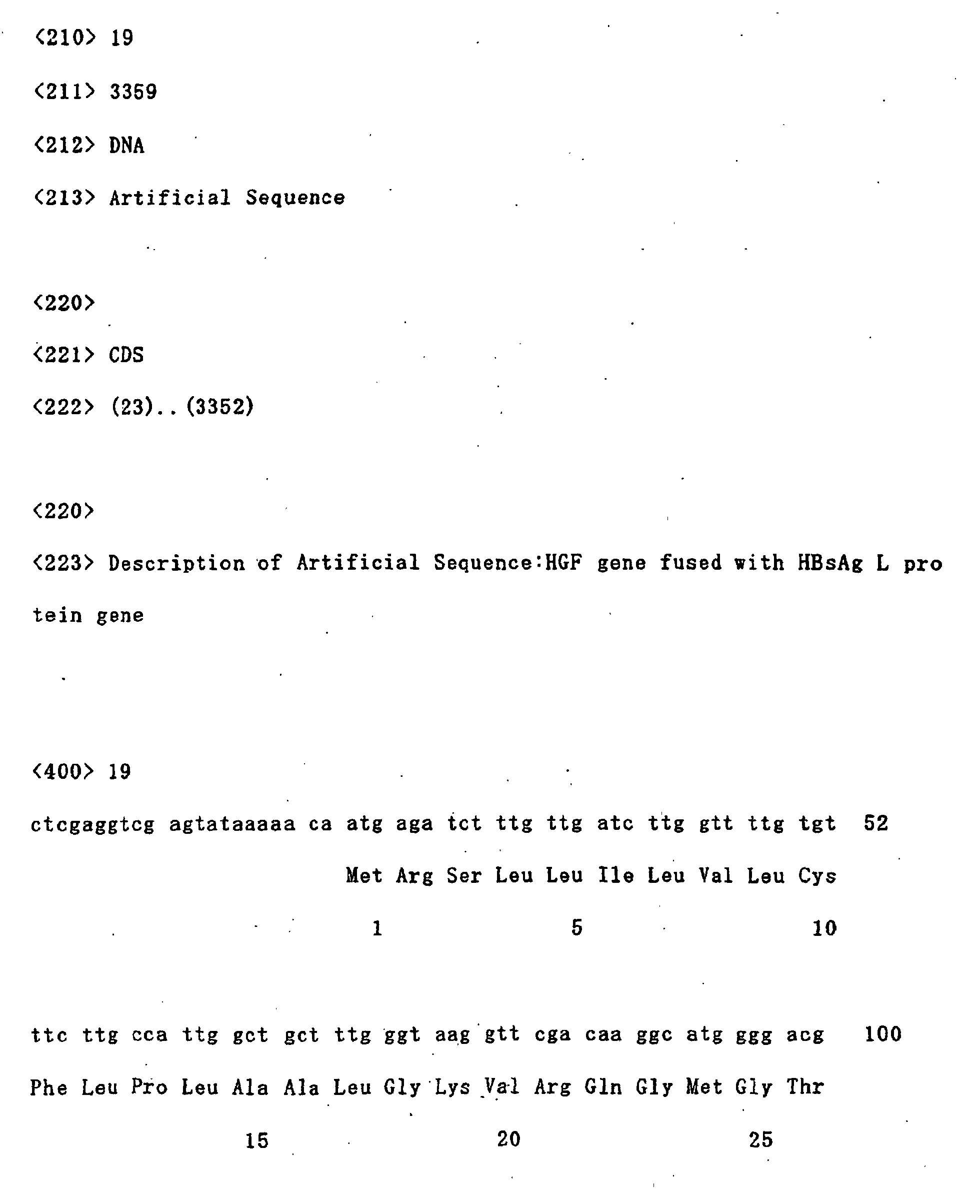

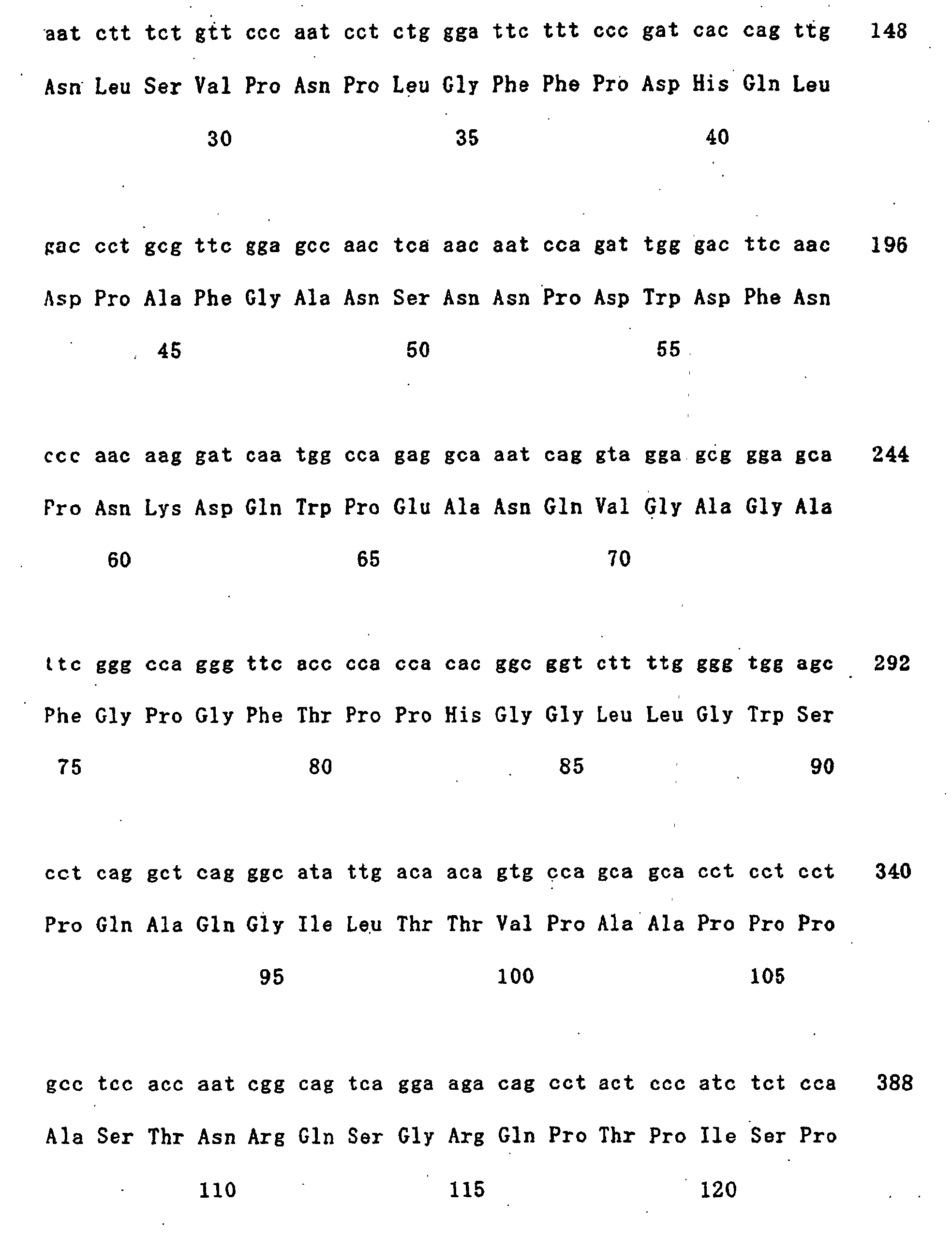

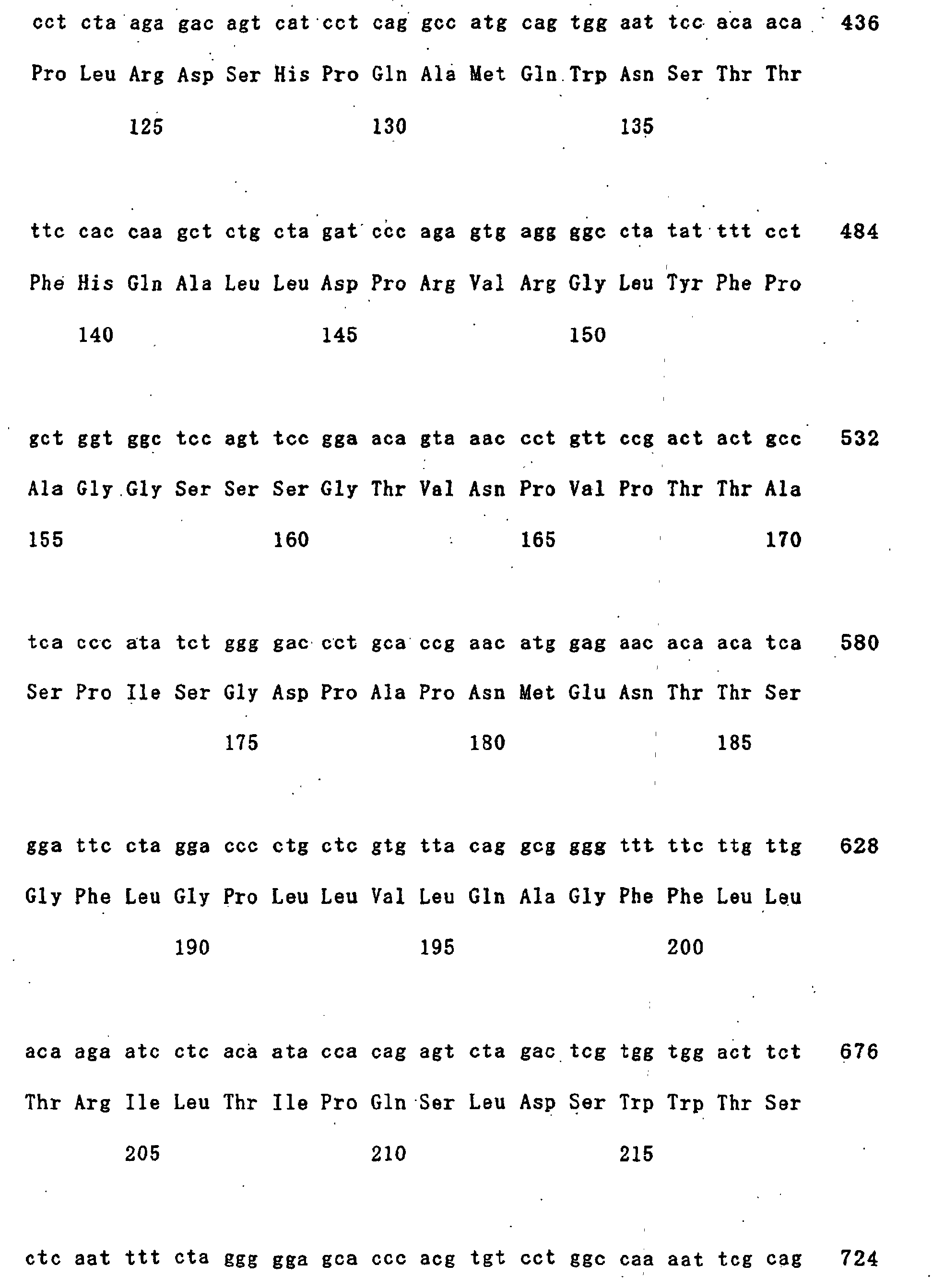

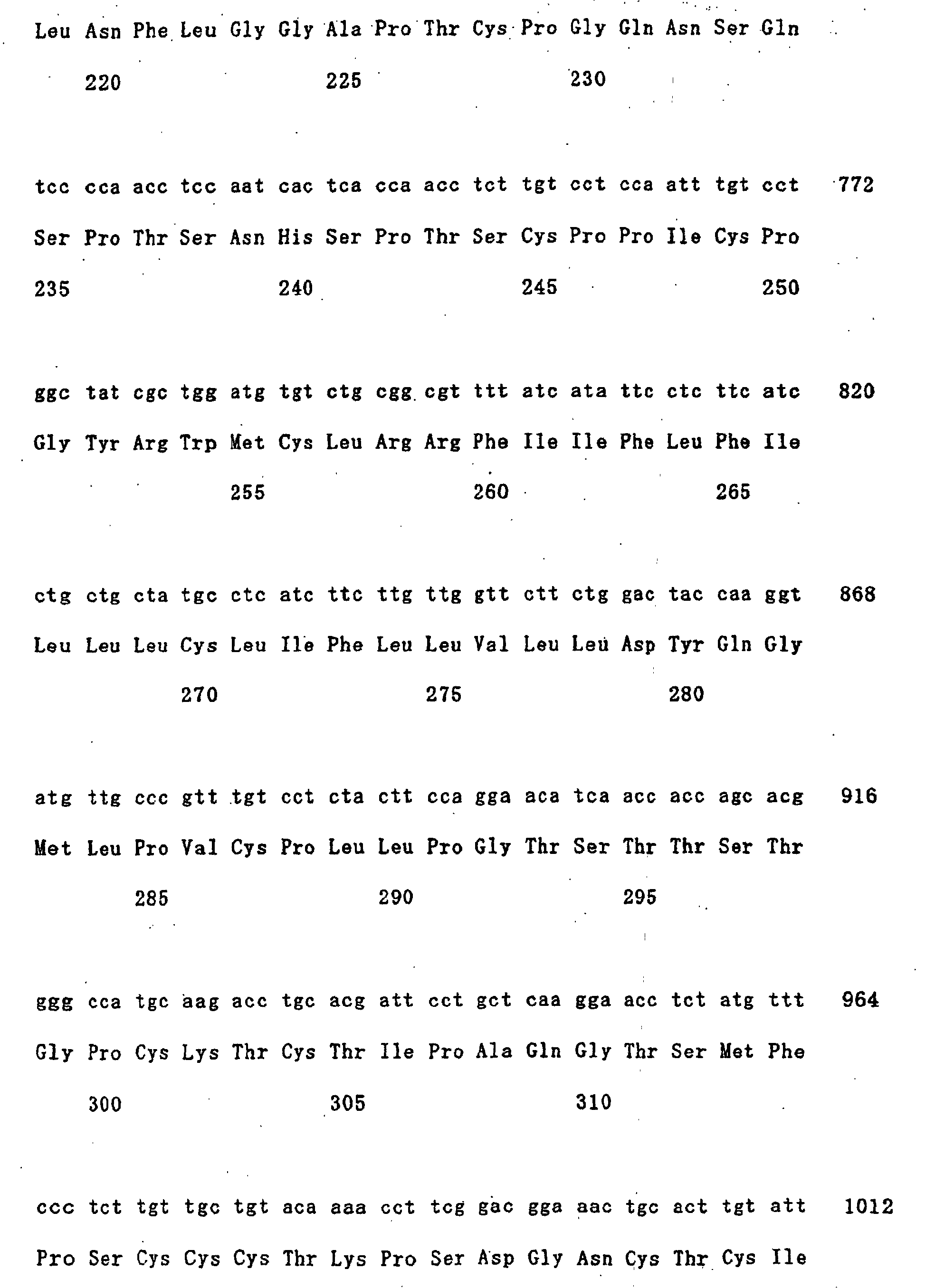

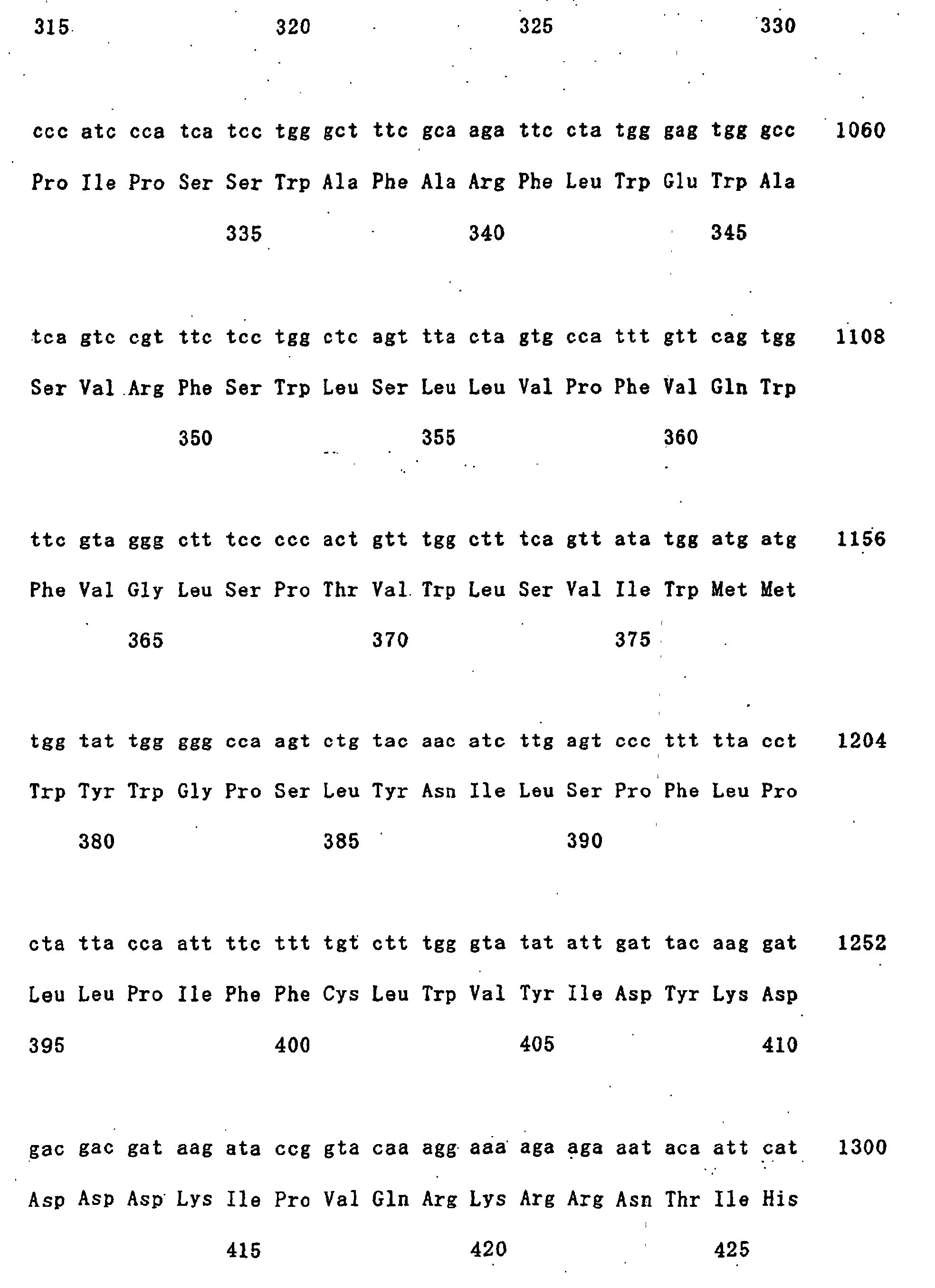

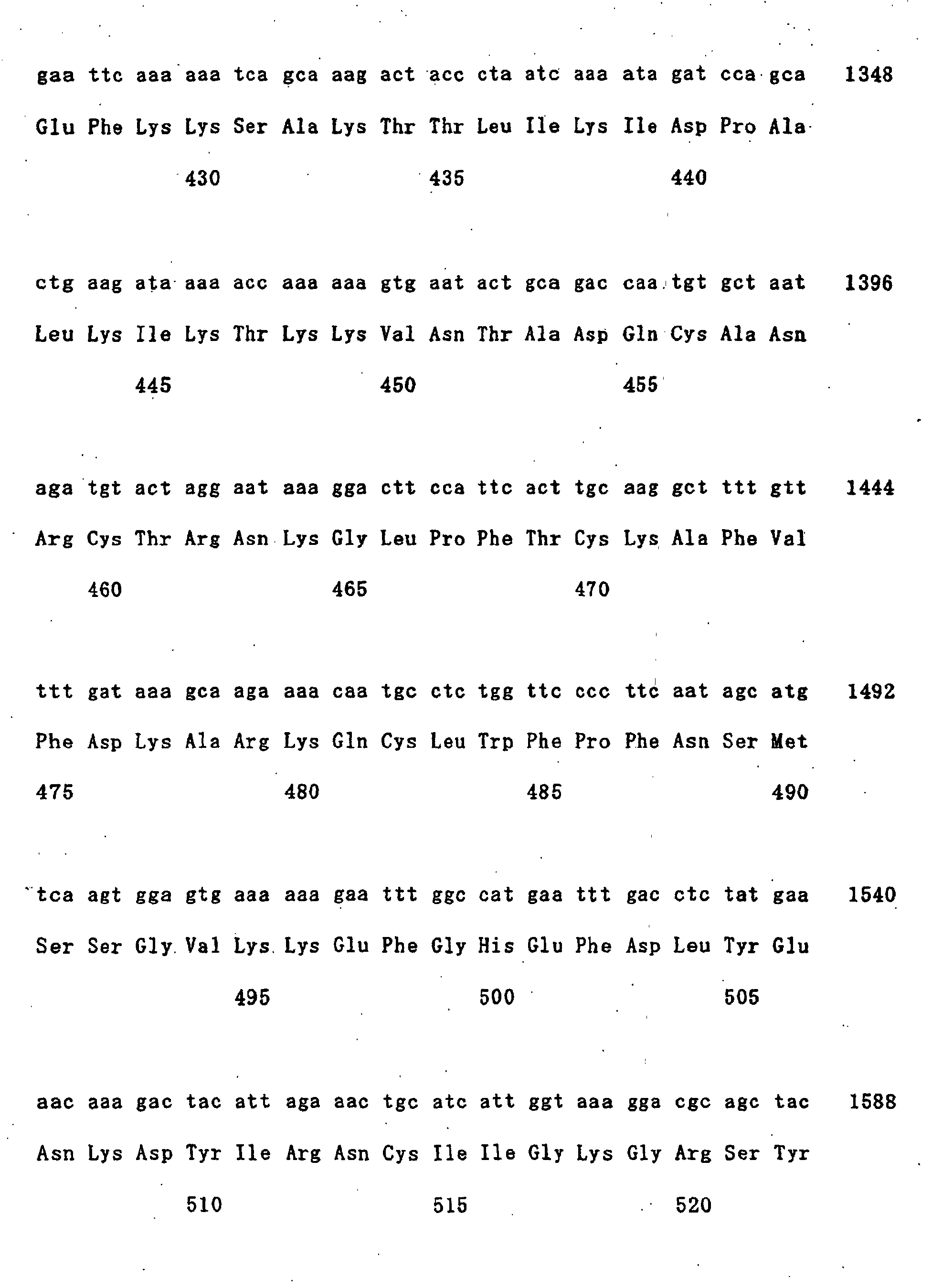

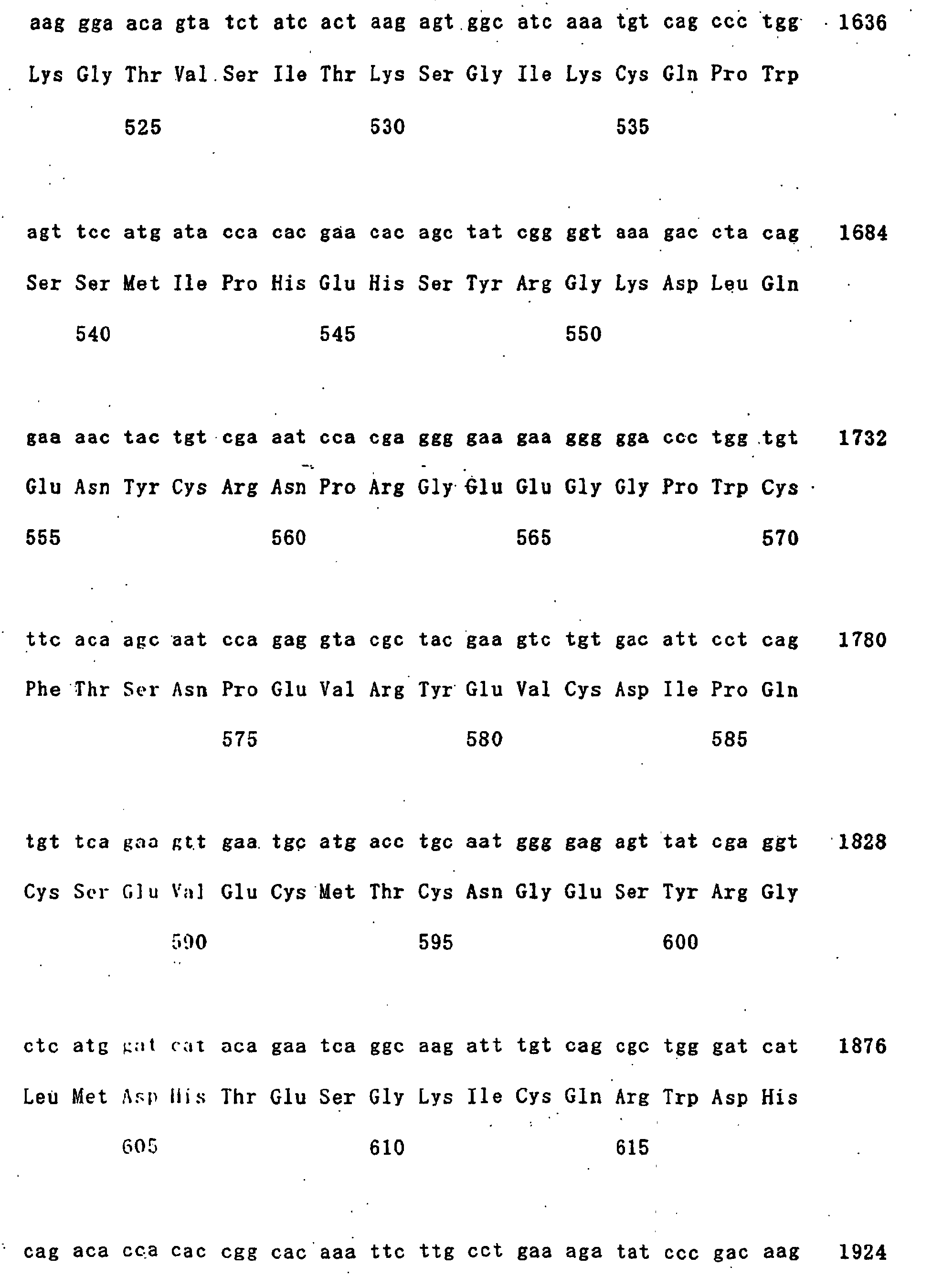

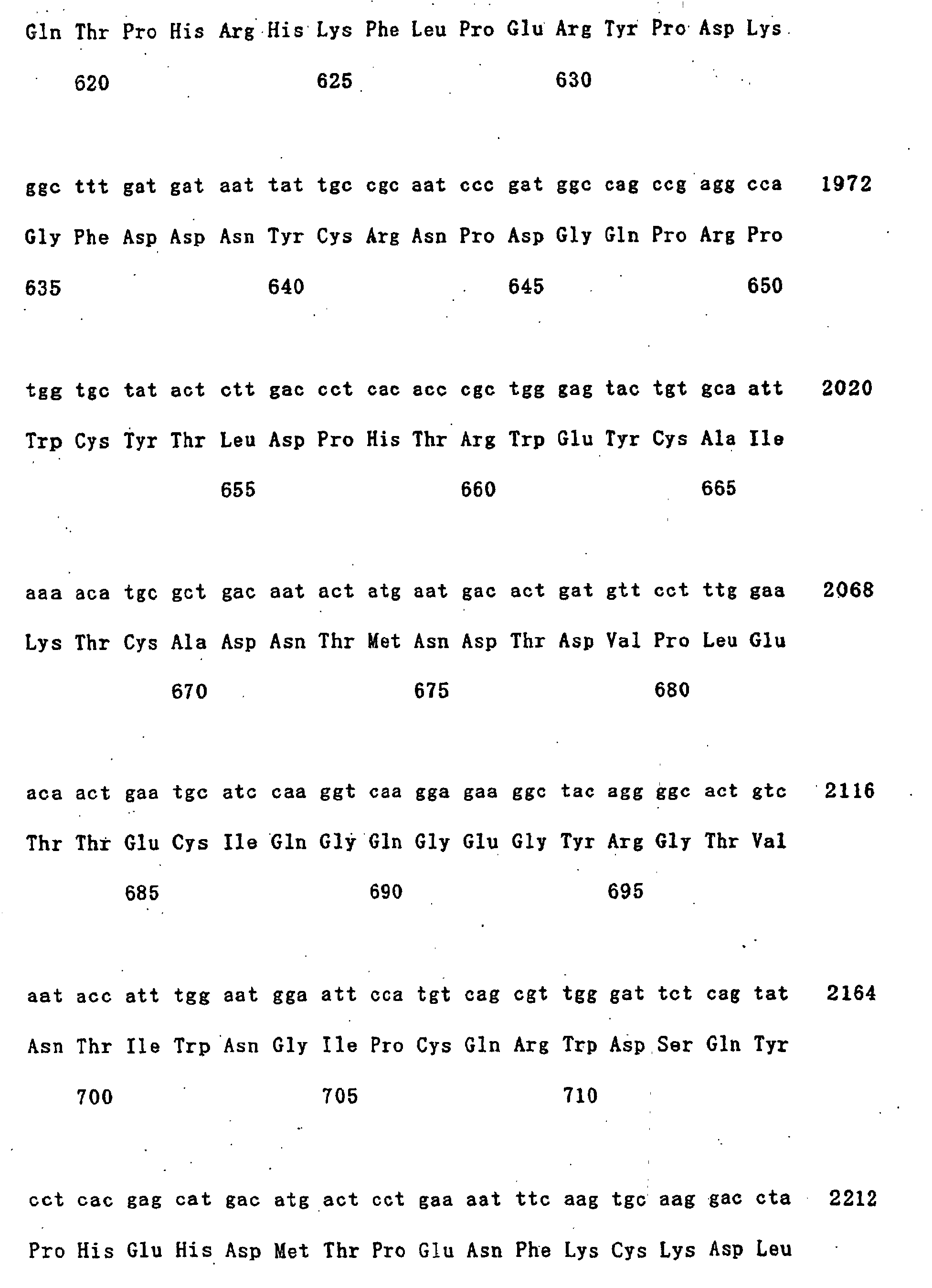

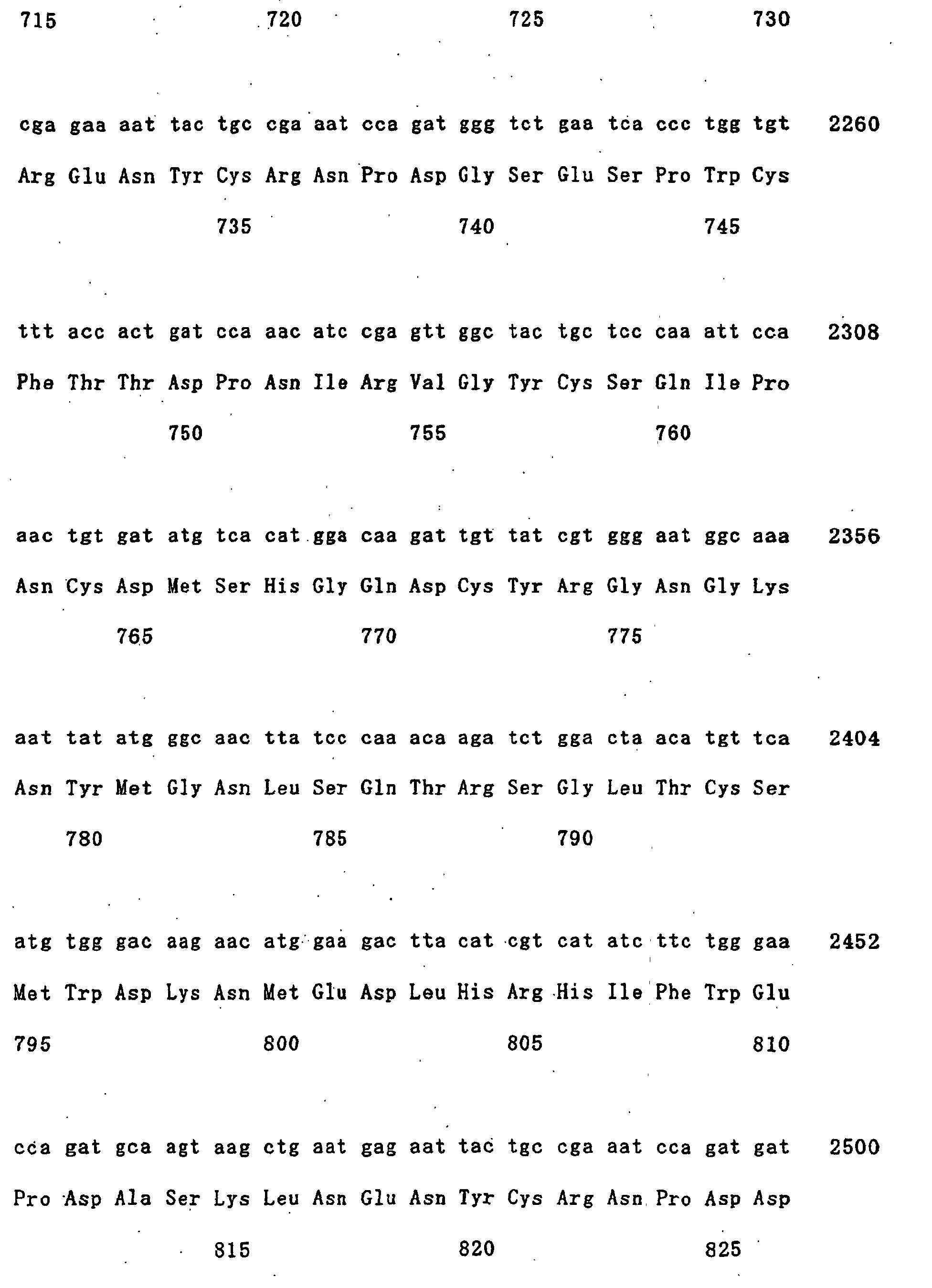

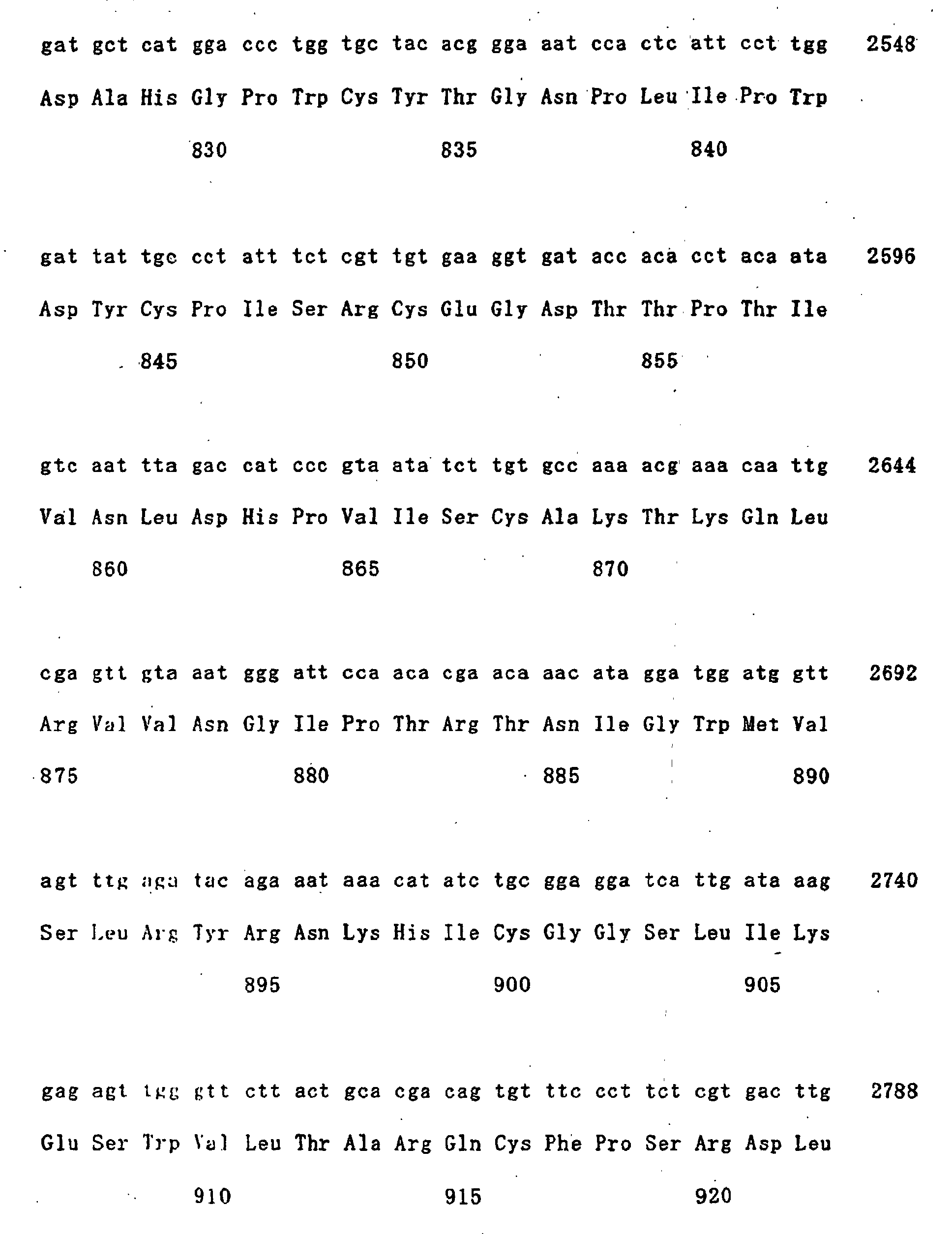

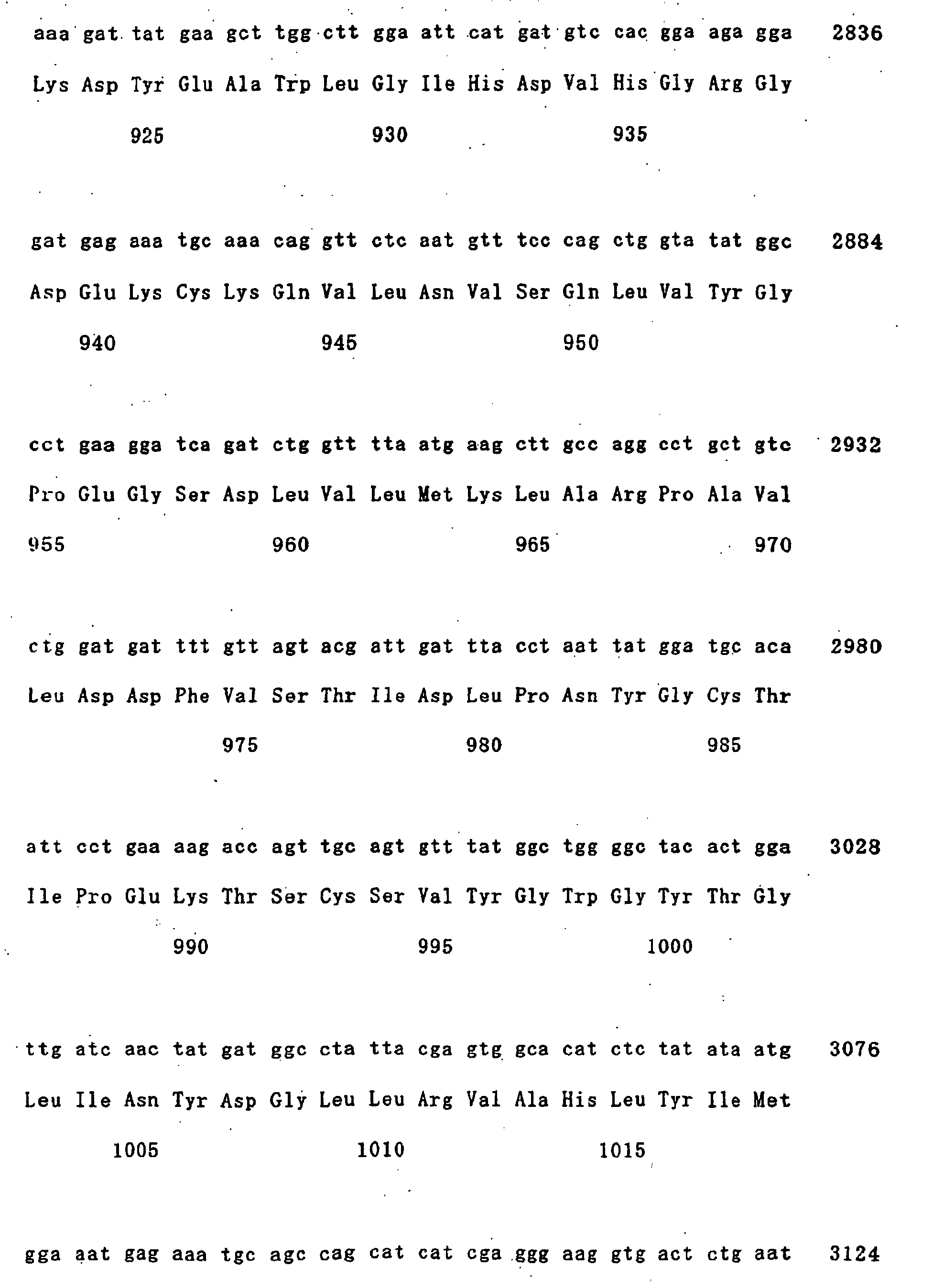

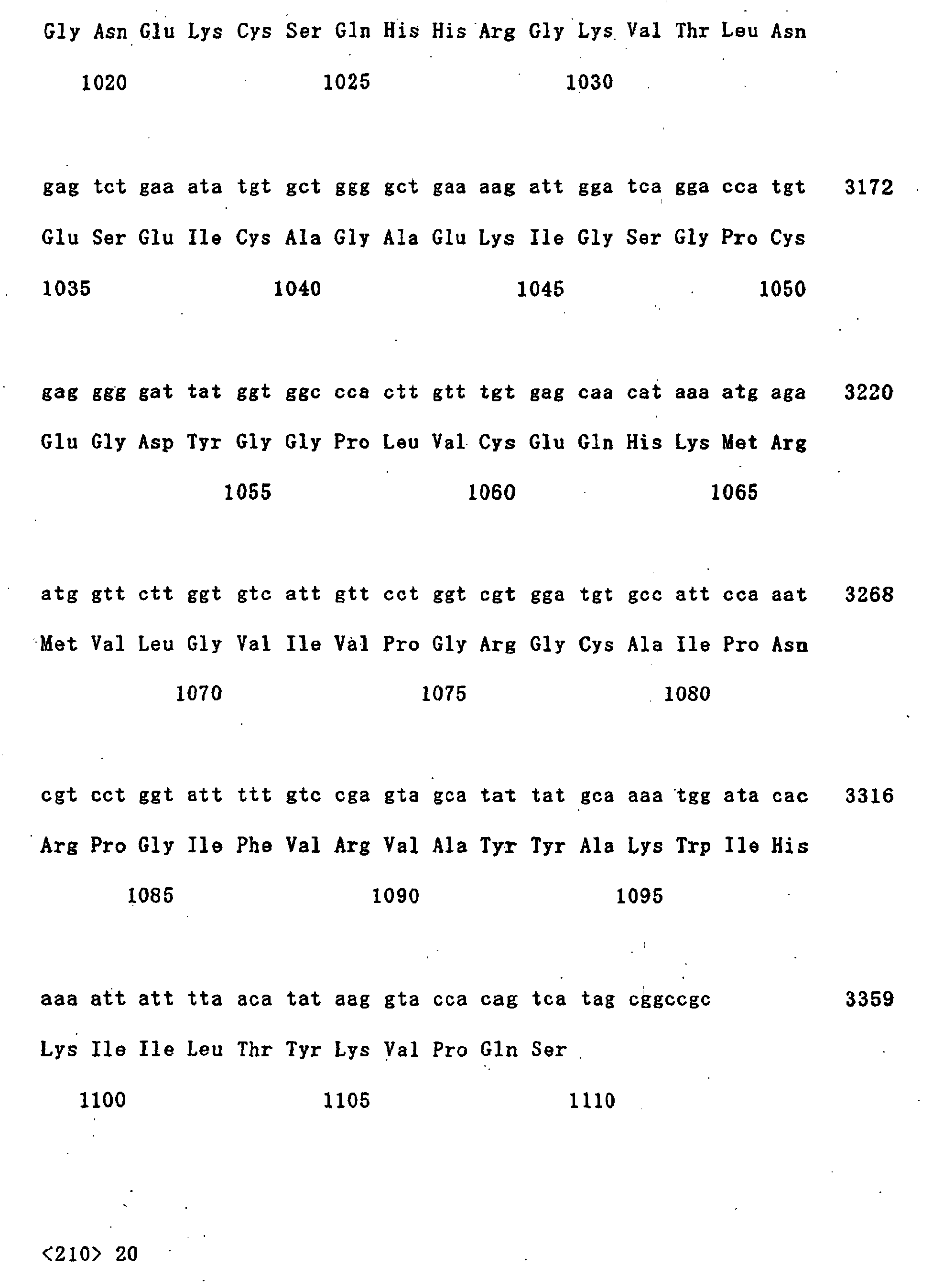

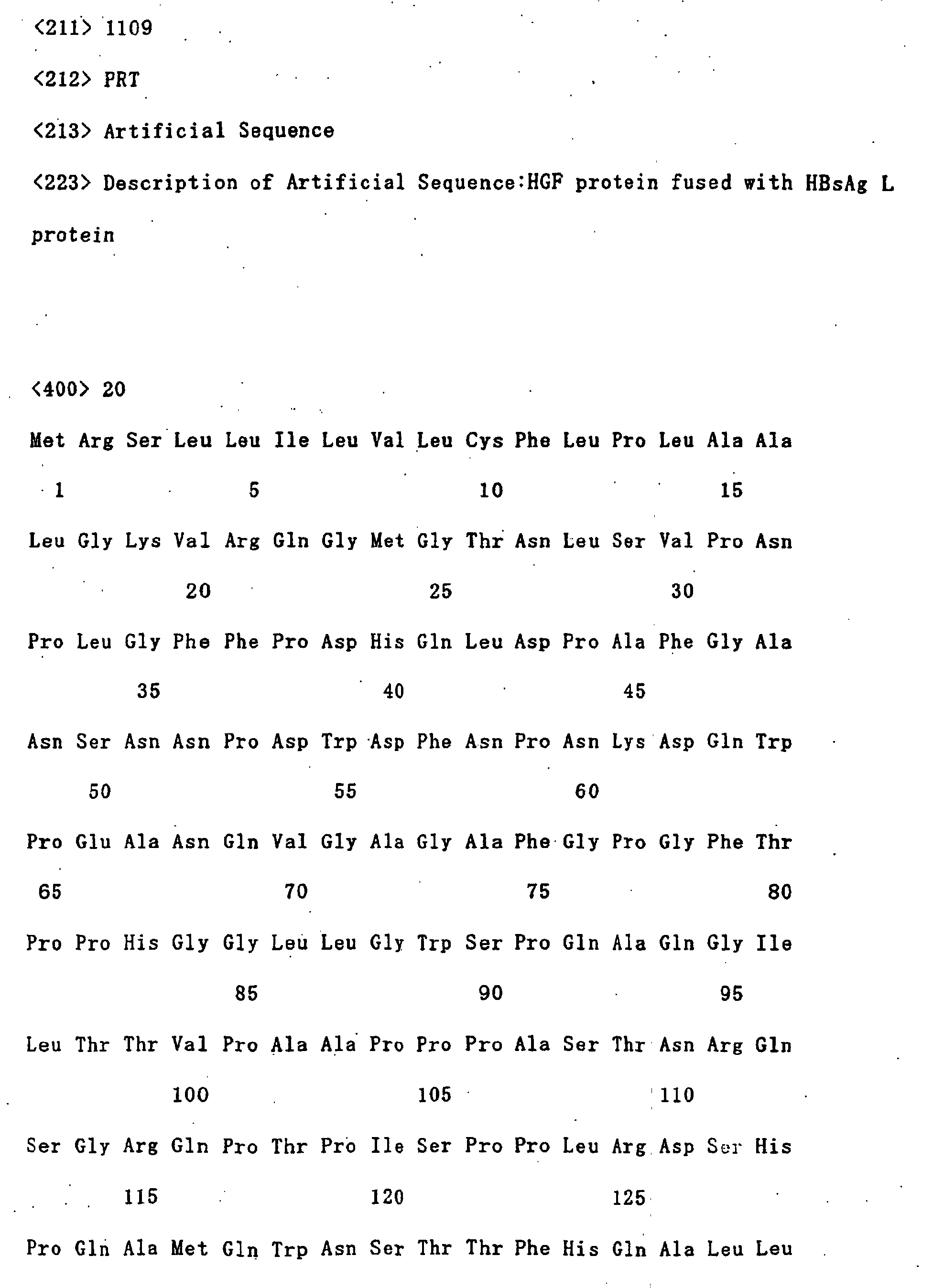

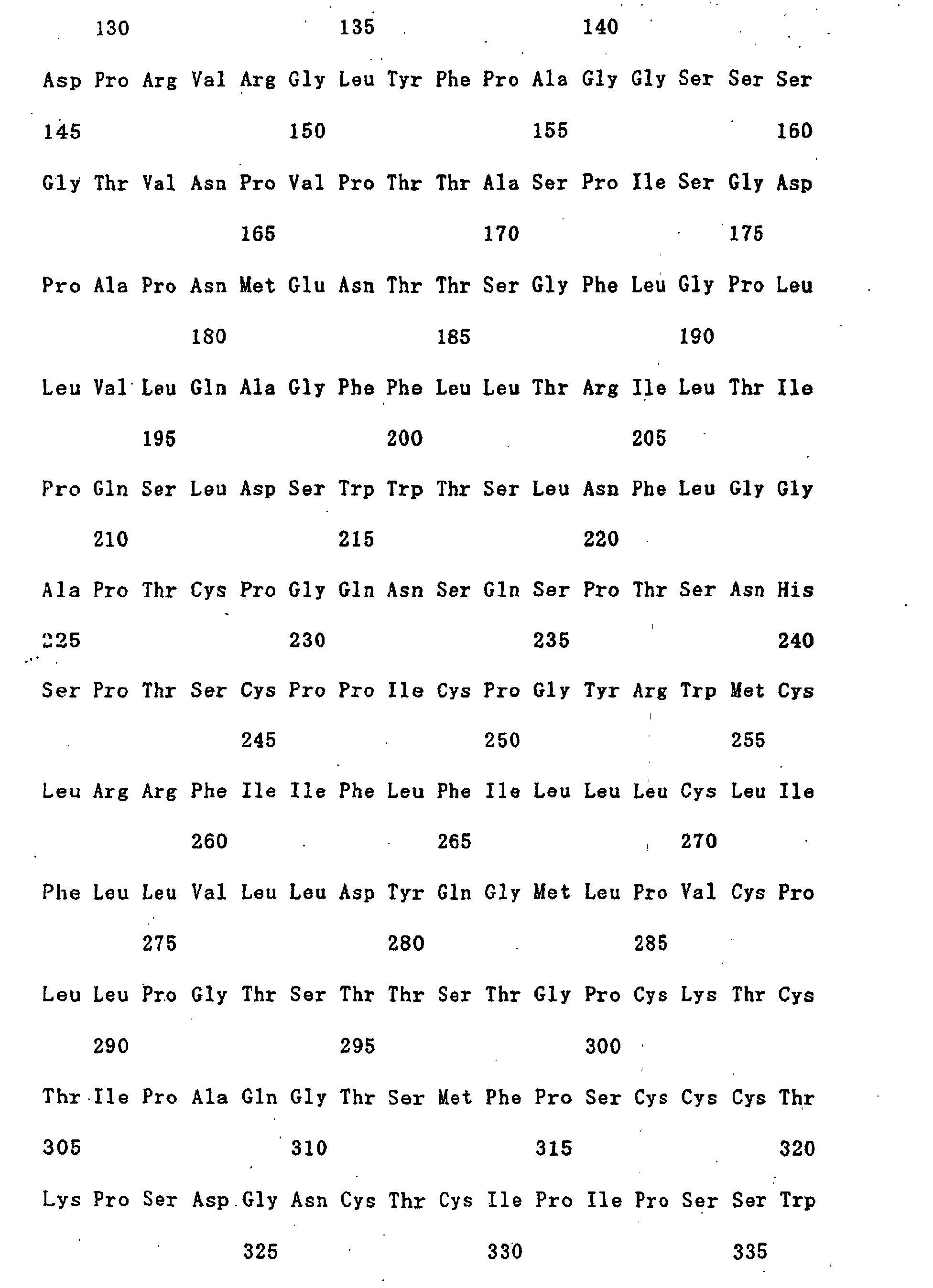

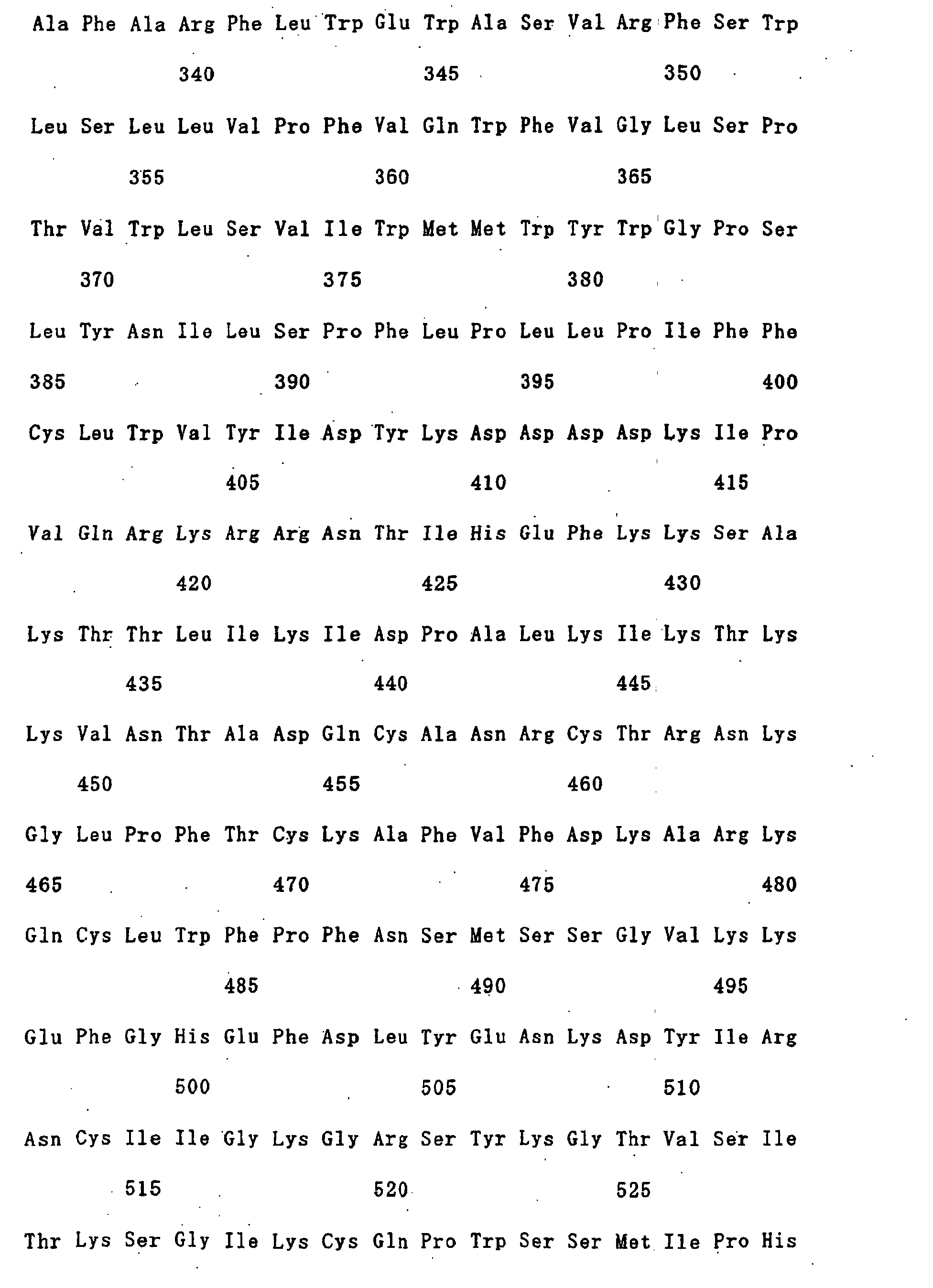

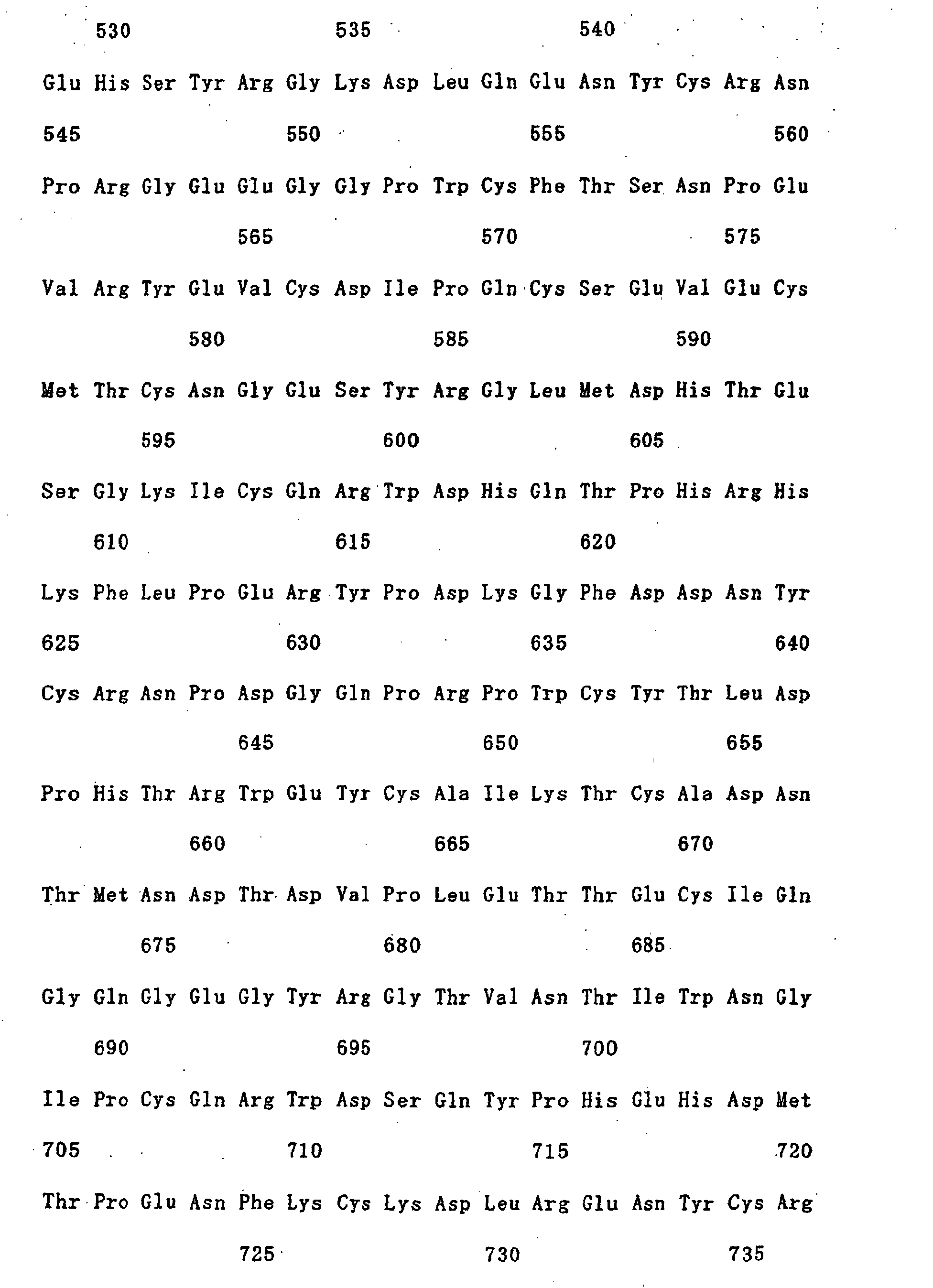

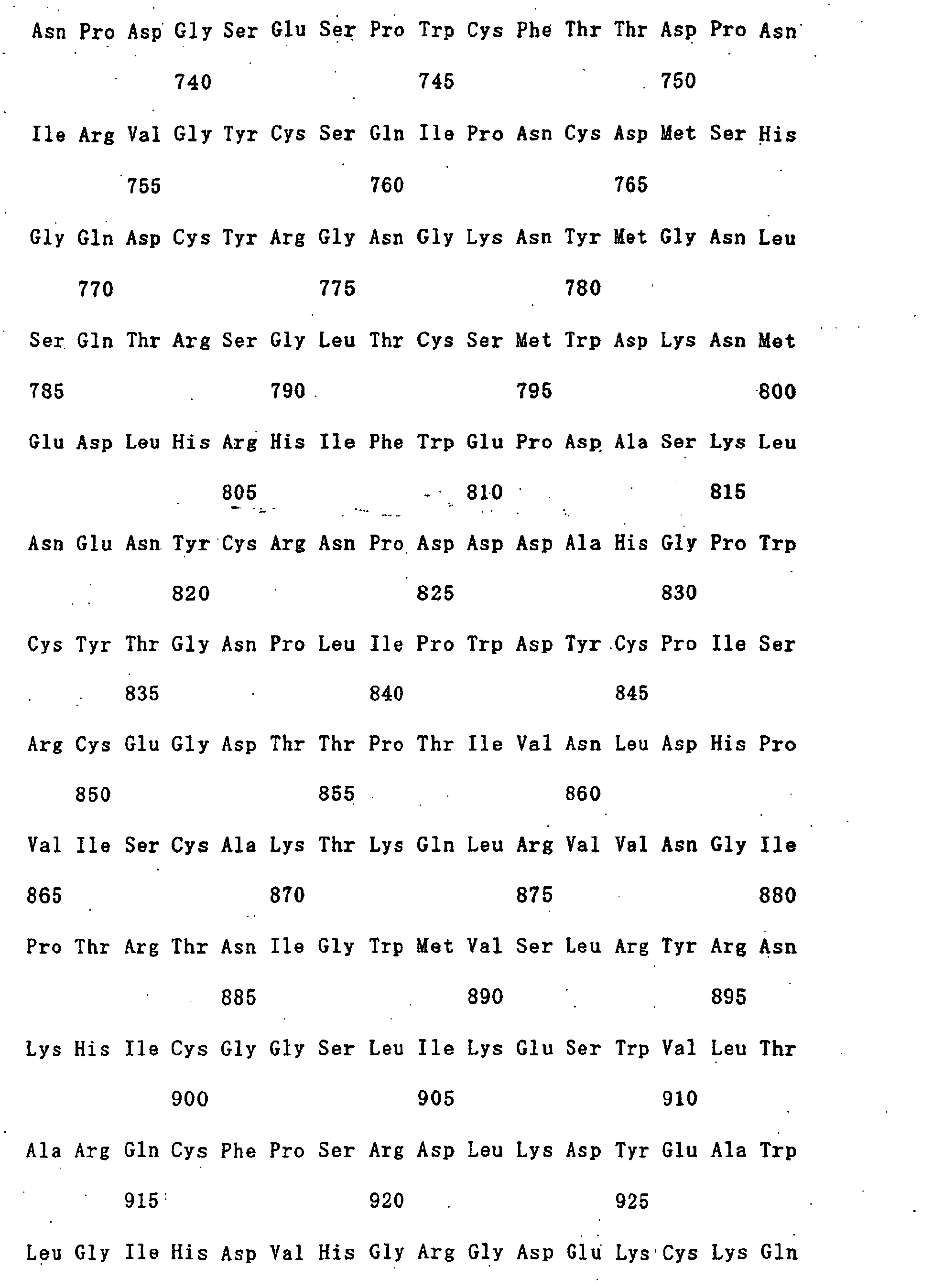

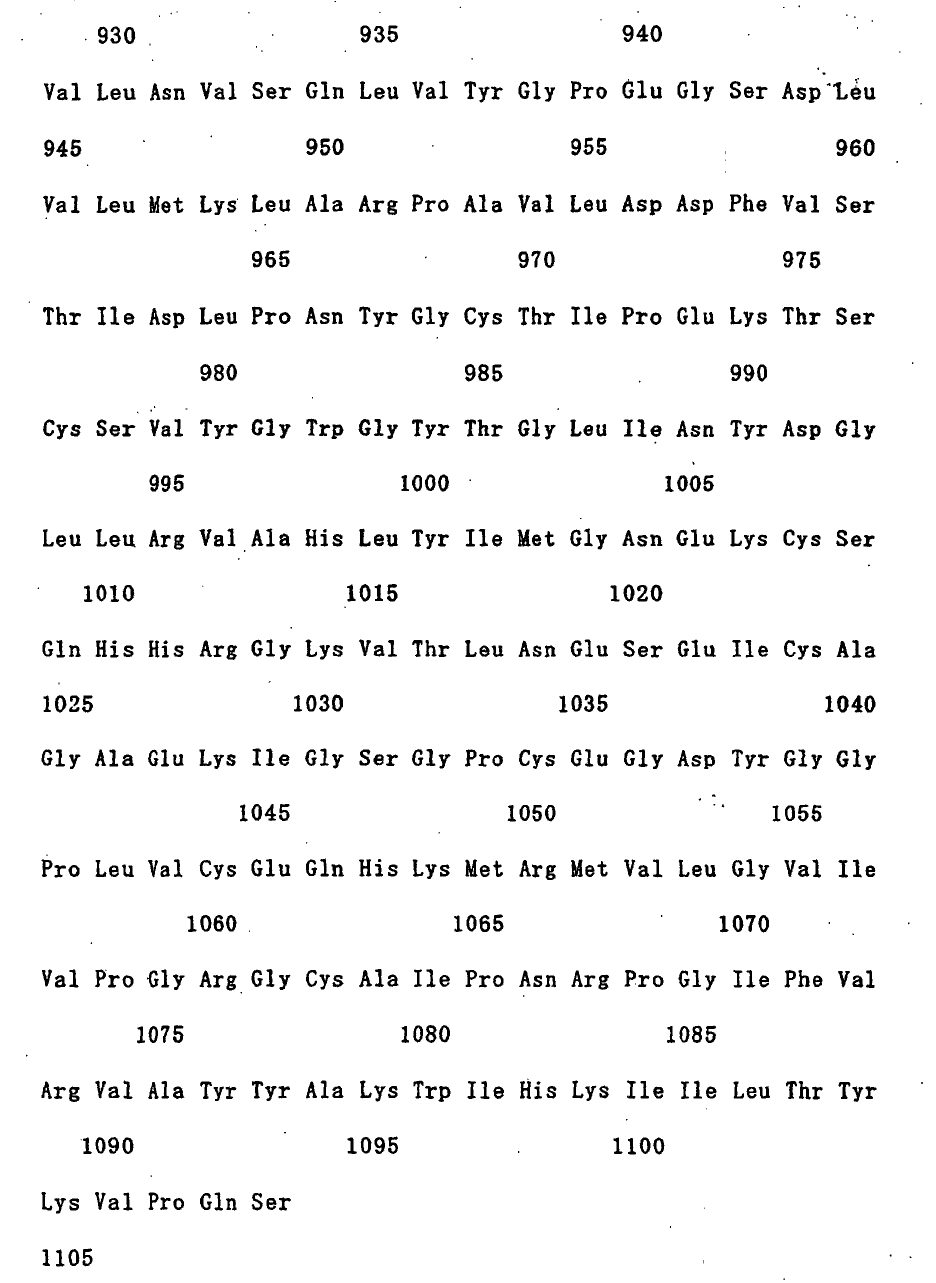

## Claims

1. A drug that comprises hollow nanoparticles of a particle-forming protein, that is capable of recognizing a specific cell or tissue, and is fused with a disease-treating target-cell-substance.

2. The drug as set forth in claim 1, wherein the particle-forming protein comprises a hepatitis B virus surface-antigen protein.

3. The drug as set forth in claim 1 or 2, wherein the drug is obtained by transforming an eukaryotic cell with a vector that contains a first gene encoding the particle-forming protein and a second gene, downstream of the first gene, encoding the target-cell-substance, and by expressing the first and second genes in the eukaryotic cell that has been transformed.

4. The drug as set forth in claim 3, wherein the eukaryotic cell is selected from a group consisting of a yeast cell, insect cell, and animal cell.

5. The drug as set forth in any one of claims 1 through 4, wherein the drug is used for treatment of hepatic diseases.

6. The drug as set forth in any one of claims 1 through 5, wherein the target-cell substance is an interferon or a hepatocyte growth factor.

7. The drug as set forth in any one of claims 1 through 6, wherein the drug is administered to the human body through intravenous injection.

8. A disease treating method comprising administering the drug of any one of claims 1 through 7.
